# EUROPÄISCHE PATENTSCHRIFT

(11) **EP 2 097 404 B1**
(45) Veröffentlichungstag und Bekanntmachung des Hinweises auf die Patenterteilung: **27.07.2016**
(21) Anmeldenummer: 07819021.2
(22) Anmeldetag: 16.10.2007
(51) Int. Cl.: C07D 401/14, A61K 31/444, A61P 9/00

(54) **SUBSTITUIERTE DIPYRIDYL-DIHYDROPYRAZOLONE UND IHRE VERWENDUNG**
SUBSTITUTED DIPYRIDYL-DIHYDROPYRAZOLONES AND USE THEREOF
DIPYRIDYL-DIHYDROPYRAZOLONES SUBSTITUÉES ET LEUR UTILISATION

(30) Priorität: 26.10.2006 DE 102006050515
(43) Veröffentlichungstag der Anmeldung: 09.09.2009
(73) Patentinhaber: Bayer Intellectual Property GmbH, 40789 Monheim (DE)
(72) Erfinder: THEDE, Kai, 10405 Berlin (DE); FLAMME, Ingo, 51580 Reichshof (DE); OEHME, Felix, 42113 Wuppertal (DE); ERGÜDEN, Jens-Kerim, 42489 Wülfrath (DE); STOLL, Friederike, 40215 Düsseldorf (DE); WILD, Hanno, 42113 Wuppertal (DE); KOLKHOF, Peter, 42113 Wuppertal (DE); BECK, Hartmut, 50733 Köln (DE); JESKE, Mario, 42699 Solingen (DE)
(74) Vertreter: BIP Patents
(86) Internationale Anmeldenummer: PCT/EP2007/008951
(87) Internationale Veröffentlichungsnummer: WO 2008/067874

(56) Entgegenhaltungen:
- WO-A-2006/114213
- US-A1- 2003 083 351

## Beschreibung

Die vorliegende Anmeldung betrifft neue, substituierte Dipyridyldihydropyrazolon-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären und hämatologischen Erkrankungen, von Nierenerkrankungen sowie zur Förderung der Wundheilung.

Eine mangelhafte Versorgung des menschlichen Organismus oder seiner Teile mit Sauerstoff, die entweder durch ihre Dauer und/oder ihr Ausmaß eine regelrechte Funktion des Organismus oder seiner Teile beeinträchtigt oder ihre Funktion völlig zum Erliegen bringt, wird als Hypoxie bezeichnet. Eine Hypoxie kann verursacht werden durch eine Verminderung des verfügbaren Sauerstoffs in der Atemluft (z.B. bei Aufenthalten in großer Höhe), durch Störungen der äußeren Atmung (z.B. infolge gestörter Funktion der Lungen oder Verlegung der Atemwege), durch eine Verminderung des Herzzeitvolumens (z.B. bei einer Herzinsuffizienz, einer akuten Rechtsherzüberlastung bei Lungenembolie), durch eine zu geringe Sauerstoff-Transportkapazität des Blutes (z.B. infolge einer Blutarmut (Anämie) oder Intoxikation z.B. mit Kohlenmonoxid), örtlich begrenzt durch eine Minderdurchblutung infolge von Gefäßverschlüssen (Ischämie-Zustände typischerweise z.B. des Herzens, der unteren Extremitäten oder des Gehirns, diabetische Makro- und Mikroangiopathie) oder auch durch erhöhten Sauerstoffbedarf des Gewebes (z.B. infolge erhöhter Muskelarbeit oder lokaler Entzündungen) [Eder, Gedigk (Hrsg.), Allgemeine Pathologie und pathologische Anatomie, 33. Aufl., Springer Verlag, Berlin, 1990].

Der menschliche Organismus ist bedingt fähig, sich an Situationen verminderter Sauerstoffversorgung akut und chronisch anzupassen. Neben einer Sofortantwort, welche u.a. durch vegetativnervöse Kontrollmechanismen eine Erhöhung des Herzzeitvolumens und des Atemzeitvolumens sowie eine lokale Erweiterung von Blutgefäßen einschließt, zieht die Hypoxie eine Veränderung der Transkription zahlreicher Gene nach sich. Die Funktion der Genprodukte dient dabei der Kompensation des Sauerstoffmangels. So werden mehrere Enzyme der Glykolyse und der Glukosetransporter 1 verstärkt exprimiert, wodurch die anaerobe ATP-Gewinnung gesteigert und ein Überleben des Sauerstoffmangels ermöglicht wird [Schmidt, Thews (Hrsg.), Physiologie des Menschen, 27. Aufl., Springer Verlag, Berlin, 1997; Löffler, Petrides (Hrsg.), Biochemie und Pathobiochemie, 7. Aufl., Springer Verlag, Berlin, 2003].

Ferner führt Hypoxie zur verstärkten Expression des vaskulären Endothelzellwachstumsfaktors, VEGF, wodurch in hypoxischen Geweben die Neubildung von Blutgefäßen (Angiogenese) angeregt wird. Hierdurch wird langfristig die Durchblutung ischämischer Gewebe verbessert. Bei verschiedenen Herzkreislauferkrankungen und Gefäßverschluß-Erkrankungen erfolgt diese Gegen-regulation offenbar nur sehr unzureichend [Übersicht bei: Simons und Ware, Therapeutic angiogenesis in cardiovasculardisease, Nat. Rev. Drug. Discov. 2 (11), 863-71 (2003)].

Ferner wird bei systemischer Hypoxie das vorwiegend in den interstitiellen Fibroblasten der Nieren gebildete Peptidhormon Erythropoietin verstärkt exprimiert. Hierdurch wird die Bildung roter Blutzellen im Knochenmark angeregt und damit die Sauerstoff-Transportkapazität des Blutes gesteigert. Dieser Effekt wurde und wird von Leistungssportlern beim sogenannten Höhentraining genutzt. Eine Abnahme der Sauerstoff-Transportkapazität des Blutes z.B. infolge einer Blutungsanämie verursacht normalerweise eine Zunahme der Erythropoietin-Produktion in der Niere. Bei bestimmten Formen der Anämie kann dieser Regelmechanismus gestört oder sein Sollwert niedriger eingestellt sein. So wird z.B. bei Patienten, die unter einer Niereninsuffizienz leiden, Erythropoietin im Nierenparenchym zwar produziert, jedoch bezogen auf die Sauerstoff-Transportkapazität des Blutes in deutlich verminderten Mengen, was eine sogenannte renale Anämie zur Folge hat. Insbesondere die renale Anämie, aber auch Tumor- und HIV-Infektion-bedingte Anämien werden üblicherweise durch parenterale Verabfolgung von rekombinantem humanen Erythropoietin (rhEPO) behandelt. Derzeit existiert zu dieser kostspieligen Therapie keine alternative Therapie mit einem oral verfügbaren Arzneistoff [Übersichten bei: Eckardt, The potential of erythropoietin and related strategies to stimulate erythropoiesis, Curr. Opin. Investig. Drugs 2(8), 1081-5 (2001); Berns, Should the target hemoglobin for patients with chronic kidney disease treated with erythropoietic replacement therapy be changed?, Semin. Dial. 18 (1), 22-9 (2005)]. Jüngere Untersuchungen belegen, dass Erythropoetin neben seiner die Erythropoese steigernden Wirkung auch eine hiervon unabhängige, schützende (anti-apoptotische) Wirkung auf hypoxische Gewebe, insbesondere das Herz und das Gehirn ausübt. Ferner mindert eine Therapie mit Erythropoetin neueren Studien zufolge an herzinsuffizienten Patienten den durchschnittlichen Morbiditäts-Schweregrad [Übersichten bei: Caiola und Cheng, Use of erythropoietin in heart failure management, Ann. Pharmacother. 38 (12), 2145-9 (2004); Katz, Mechanisms and treatment of anemia in chronic heart failure, Congest. Heart. Fail. 10 (5), 243-7 (2004)].

Den oben beschriebenen durch Hypoxie induzierten Genen ist gemeinsam, dass die Steigerung ihrer Expression unter Hypoxie durch den sogenannten Hypoxie-induzierbaren Transkriptionsfaktor (HIF) hervorgerufen wird. Bei HIF handelt es sich um einen heterodimeren Transkriptionsfaktor, der aus einer alpha- und einer beta-Untereinheit besteht. Es wurden drei HIF-alpha-Isoformen beschrieben, von denen HIF-1 alpha und HIF-2 alpha hoch homolog und von Bedeutung für die Hypoxie-induzierte Genexpression sind. Während die auch als ARNT (aryl hydrocarbon receptor nuclear translocator) bezeichnete beta-Untereinheit (von der 2 Isoformen beschrieben wurden) konstitutiv exprimiert ist, hängt die Expression der alpha-Untereinheit vom Sauerstoffgehalt in der Zelle ab. Unter Normoxie wird das HIF-alpha-Protein poly-ubiquitiniert und anschließend proteasomal degradiert. Unter Hypoxie ist diese Degradierung gehemmt, so dass HIF-alpha mit ARNT dimerisieren und seine Zielgene aktivieren kann. Das HIF-Dimer bindet hierbei an sogenannte Hypoxie-responsible Elemente (HRE) in den regulatorischen Sequenzen seiner Zielgene. Die HRE sind durch eine Konsensus-Sequenz definiert. Funktionelle HRE wurden in den regulatorischen Elementen zahlreicher Hypoxie-induzierter Gene nachgewiesen [Übersichten bei: Semenza, Hypoxia-inducible factor 1: oxygen homeostasis and disease pathophysiology, Trends Mol. Med. 7 (8), 345-50 (2001); Wenger und Gassmann, Oxygen(es) and the hypoxia-inducible factor-1, Biol. Chem. 378 (7), 609-16 (1997)].

Der molekulare Mechanismus, der dieser Regulation von HIF-alpha zugrunde liegt, wurde durch die Arbeiten mehrerer voneinander unabhängiger Forschergruppen aufgeklärt. Der Mechanismus ist Spezies-übergreifend konserviert: HIF-alpha wird durch eine als PHD oder EGLN bezeichnete Subklasse von Sauerstoff-abhängigen Prolyl-4-Hydroxylasen an zwei spezifischen Prolyl-Resten hydroxyliert (P402 und P564 der humanen HIF-1-alpha-Untereinheit). Bei den HIF-Prolyl-4-Hydroxylasen handelt es sich um Eisen-abhängige, 2-Oxoglutarat-umsetzende Dioxygenasen [Epstein et al., C. elegans EGL-9 and mammalian homologs define a family of dioxygenases that regulate HIF by prolyl hydroxylation, Cell 107 (1), 43-54 (2001); Bruick und McKnight, A conserved family of prolyl-4-hydroxylases that modify HIF, Science 294 (5545), 1337-40 (2001); Ivan et al., Biochemical purification and pharmacological inhibition of a mammalian prolyl hydroxylase acting on hypoxia-inducible factor, Proc. Natl. Acad. Sci. U.S.A. 99 (21), 13459-64 (2002)]. Die Enzyme wurden 2001 erstmals als Prolyl-Hydroxylasen annotiert [Aravind und Koonin, The DNA-repair protein AlkB, EGL-9, and leprecan define new families of 2-oxoglutarate- and irondependent dioxygenases, Genome Biol. 2 (3), research0007.1-0007.8, Epub 2001 Feb 19].

An die prolylhydroxylierte HIF-alpha-Untereinheit bindet das pVHL Tumor Suppressor Protein, welches zusammen mit Elongin B und C den sogenannten VBC-Komplex bildet, der die HIF-alpha-Untereinheit an eine E3 Ubiquitin-Ligase adaptiert. Da die Prolyl-4-Hydroxylierung der HIF-alpha-Untereinheit und ihre nachfolgende Degradierung in Abhängigkeit von der intrazellulären Konzentration des Sauerstoffs erfolgt, wurden die HIF-Prolyl-4-Hydroxylasen auch als zellulärer Sauerstoffsensor bezeichnet. Es wurden drei Isoformen dieser Enzyme identifiziert: EGLN1/PHD2, EGLN2/PHD1 und EGLN3/PHD3. Zwei dieser Enzyme (EGLN2/PHD1 und EGLN3/PHD3) werden selbst unter Hypoxie transkriptionell induziert und sind möglicherweise für die unter chronischer Hypoxie zu beobachtende Absenkung der HIF-alpha-Spiegel verantwortlich [Übersicht bei: Schofield und Ratcliffe, Oxygen sensing by HIF hydroxylases, Nat. Rev. Mol. Cell. Biol. 5 (5), 343-54 (2004)].

Eine selektive pharmakologische Hemmung der HIF-Prolyl-4-Hydroxylasen zieht die Steigerung der Genexpression HIF-abhängiger Zielgene nach sich und ist damit für die Therapie zahlreicher Krankheitsbilder von Nutzen. Insbesondere bei Erkrankungen des Herz-Kreislaufsystems ist von der Induktion neuer Blutgefäße sowie der Änderung der Stoffwechselsituation ischämischer Organe von aerober hin zu anaerober ATP-Gewinnung eine Besserung des Krankheitsverlaufs zu erwarten. Eine Verbesserung der Vaskularisierung chronischer Wunden fördert den Heilungsprozess, insbesondere bei schwer heilenden Ulcera cruris und anderen chronischen Hautwunden. Die Induktion von körpereigenem Erythropoietin bei bestimmten Krankheitsformen, insbesondere bei Patienten mit renaler Anämie, ist ebenfalls ein anzustrebendes therapeutisches Ziel.

Die bislang in der wissenschaftlichen Literatur beschriebenen HIF-Prolyl-4-Hydroxylase-Inhibitoren erfüllen nicht die an ein Arzneimittel zu stellenden Anforderungen. Es handelt sich hierbei entweder um kompetitive Oxoglutarat-Analoga (wie z.B. N-Oxalylglycin), die durch ihre sehr geringe Wirkstärke charakterisiert sind und daher in *in vivo*-Modellen bislang keine Wirkung im Sinne einer Induktion von HIF-Zielgenen gezeigt haben. Oder es handelt sich um Eisen-Komplexbildner (Chelatoren) wie Desferroxamin, die als unspezifische Hemmstoffe Eisen-haltiger Dioxygenasen wirken und, obwohl sie eine Induktion der Zielgene wie z.B. Erythropoetin *in vivo* herbeiführen, durch Komplexierung des verfügbaren Eisens offenbar der Erythropoese entgegenwirken.

Aufgabe der vorliegenden Erfindung ist die Bereitstellung neuer Verbindungen, die für die Behandlung von Erkrankungen, insbesondere kardiovaskulärer und hämatologischer Erkrankungen, eingesetzt werden können.

Im Rahmen der vorliegenden Erfindung werden nunmehr Verbindungen beschrieben, die als spezifische Hemmstoffe der HIF-Prolyl-4-Hydroxylasen wirken und aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbeiführen.

2-Heteroaryl-4-aryl-1,2-dihydropyrazolone mit bakterizider und/oder fungizider Wirkung werden in EP 165 448 und EP 212 281 offenbart. Die Verwendung von 2-Heteroaryl-4-aryl-1,2-dihydropyrazolonen als Lipoxygenase-Inhibitoren zur Behandlung von Atemwegs-, Herz-Kreislauf- und Entzündungserkrankungen wird in EP 183 159 beansprucht. 2,4-Diphenyl-1,2-dihydropyrazolone mit herbizider Aktivität werden in DE 2 651 008 beschrieben. Über die Herstellung und pharmakologischen Eigenschaften bestimmter 2-Pyridyl-1,2-dihydropyrazolone wird in Helv. Chim. Acta 49 (1), 272-280 (1966) berichtet. Weitere Verbindungen mit Dihydropyrazolon-Partialstruktur sowie ihre Verwendung zur Behandlung von Erkrankungen werden in WO 96/12706, WO 00/51989 und WO 03/074550 beansprucht, und in WO 02/092573 werden Pyrazol-4-ylpyridin- und Pyrazol-4-ylpyrimidin-Derivate als Kinase-Inhibitoren beschrieben. Weiterhin werden in WO 03/051833 und WO 2004/089303 Heteroaryl-substituierte Pyrazol-Derivate zur Behandlung von Schmerz und verschiedenen ZNS-Erkrankungen beschrieben. Zwischenzeitlich sind in WO 2006/114213 2,4-Dipyridyl-1,2-dihydropyrazolone als Inhibitoren von HIF-Prolyl-4-Hydroxylasen offenbart worden.

Gegenstand der vorliegenden Erfindung sind Verbindungen der allgemeinen Formel (I) 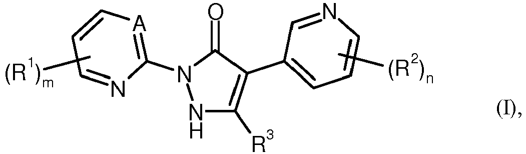 in welcher
- A: für CH steht,
- R¹: für einen Substituenten ausgewählt aus der Reihe (C₃-C₆)-Cycloalkyl, 4- bis 6-gliedriges Heterocycloalkyl, Phenyl, 5- oder 6-gliedriges Heteroaryl, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹ und -NR²⁵R²⁶ steht, worin
(*i*) (C₃-C₆)-Cycloalkyl, 4- bis 6-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, Halogen, Cyano, Oxo, Hydroxy, Amino, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, NR¹⁸-SO₂-R¹⁹, -OR²³ und -NR²⁵R²⁶ substituiert sein können,
   wobei der letztgenannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl, 4- bis 6-gliedrigem Heterocycloalkyl, Phenyl und/oder 5-oder 6-gliedrigem Heteroaryl substituiert sein kann,
(*ii*) R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
   (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino,
   Di-(C₁-C₄)-alkylamino, Hydroxycarbonyl und/oder (C₁-C₄)-Alkoxycarbonyl substituiert sein können
   und
   (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
(*iii*) R⁷*,* R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
   wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   und/oder worin
(*iv*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein-bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbo-nyl substituiert sein kann,
- R²: für einen Substituenten ausgewählt aus der Reihe -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} und -NR^{25A}R^{26A} steht, worin
(*i*) R^{9A}, R^{12A}, R^{14A}, R^{16A}, R^{19A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*ii*) R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
   und/oder worin
(*iii*) R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- m: für die Zahl 1 steht,
- n: für die Zahl 0 oder 1 steht
und
- R³: für Wasserstoff steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Trimethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-butyl, *iso*-Butyl, *sec*.-Butyl, *tert*.-Butyl, 1-Ethyl-propyl, *n*-Pentyl und *n*-Hexyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, *tert*.-Butoxy, *n*-Pentoxy und *n-*Hexoxy.

Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.

Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N*-Dimethylamino, *N,N*-Diethylamino, *N*-Ethyl-*N*-methylamino, N-Methyl-*N*-*n*-propylamino, *N*-Isopropyl-*N*-*n*-propylamino, *N,N*-Diisopropylamino, *N*-*n*-Butyl-*N-*methylamino, *N*-*tert*.-Butyl-*N*-methylamino, *N*-Methyl-*N*-*n*-pentylamino und *N*-*n*-Hexyl-*N*-methylamino.

(C₁-C₄)-Acyl [(C₁-C₄)-Alkanoyl] steht im Rahmen der Erfindung für einen geradkettigen oder verzweigten Alkylrest mit 1 bis 4 Kohlenstoffatomen, der in der 1-Position ein doppelt gebundenes Sauerstoffatom trägt und über die 1-Position verknüpft ist. Bevorzugt ist ein Acylrest mit 1 bis 4 Kohlenstoffatomen. Beispielhaft und vorzugsweise seien genannt: Formyl, Acetyl, Propionyl, *n-*Butyryl und *iso*-Butyryl.

(C₁-C₄)-Acylamino steht im Rahmen der Erfindung für eine Amino-Gruppe mit einem geradkettigen oder verzweigten Acyl-Substituenten, der 1 bis 4 Kohlenstoffatome aufweist und über die Carbonylgruppe mit dem N-Atom verknüpft ist. Beispielhaft und vorzugsweise seien genannt: Formylamino, Acetylamino, Propionylamino, *n*-Butyrylamino und *iso*-Butyrylamino.

(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonyl-Rest mit 1 bis 4 Kohlenstoffatomen in der Alkoxygruppe. Beispielhaft und vorzugsweise seien genannt: Methoxycarbonyl, Ethoxycarbonyl, *n*-Propoxycarbonyl, Isopropoxycarbonyl, *n*-Butoxy-carbonyl und *tert*.-Butoxycarbonyl.

(C₃-C₇)-Cycloalkyl und (C₃-C₆)-Cycloalkyl stehen im Rahmen der Erfindung für einen monocyclischen, gesättigten Carbocyclus mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

4- bis 7-gliedriges Heterocycloalkyl steht im Rahmen der Erfindung für einen monocyclischen, gesättigten Heterocyclus mit insgesamt 4 bis 7 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Oxetanyl, Thietanyl, Pyrrolidinyl, Pyrazolidinyl, Tetrahydrofuranyl, Thiolanyl, 1,3-Oxazolidinyl, Piperidinyl, Piperazinyl, Tetrahydropyranyl, Tetrahydrothiopyranyl, 1,3-Dioxanyl, 1,4-Dioxanyl, Morpholinyl, Thiomorpholinyl, Hexahydroazepinyl, Hexahydro-1,4-diazepinyl. Bevorzugt ist ein 4- bis 6- gliedriger Heterocycloalkyl-Rest mit insgesamt 4 bis 6 Ringatomen, der ein oder zwei Ring-Heteroatome aus der Reihe N und/oder O enthält, wie beispielsweise Pyrrolidinyl, Tetrahydrofuranyl, Piperidinyl, Piperazinyl, Tetrahydropyranyl und Morpholinyl.

5- oder 6-gliedriges Heteroaryl steht im Rahmen der Erfindung für einen aromatischen Heterocyclus (Heteroaromaten) mit insgesamt 5 bzw. 6 Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl, Triazinyl. Bevorzugt sind 5- oder 6-gliedrige Heteroaryl-Reste mit bis zu drei Ring-Heteroatomen aus der Reihe N, O und/oder S, wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl.

Halogen schließt im Rahmen der Erfindung Fluor, Chlor, Brom und Iod ein. Bevorzugt sind Fluor, Chlor und Brom, besonders bevorzugt Fluor und Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Besonders bevorzugt ist die Substitution mit einem oder zwei gleichen oder verschiedenen Substituenten.

Bevorzugt im Rahmen der vorliegenden Erfindung sind Verbindungen der Formel (I), in welcher
- A: für CH steht,
- R¹: für einen Substituenten ausgewählt aus der Reihe (C₃-C₆)-Cycloalkyl, 4- bis 6-gliedriges Heterocycloalkyl, Phenyl, 5- oder 6-gliedriges Heteroaryl, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹ und -NR²⁵R²⁶ steht, worin
(i) (C₃-C₆)-Cycloalkyl, 4- bis 6-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, Halogen, Cyano, Oxo, Hydroxy, Amino, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert sein können,
   wobei der letztgenannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl, 4- bis 6-gliedrigem Heterocycloalkyl, Phenyl und/oder 5-oder 6-gliedrigem Heteroaryl substituiert sein kann,
(*ii*) R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
   (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino,
   Di-(C₁-C₄)-alkylamino, Hydroxycarbonyl und/oder (C₁-C₄)-Alkoxycarbonyl substituiert sein können
   und
   (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
(*iii*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
   wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   und/oder worin
(*iv*) R⁶ und R⁷, R⁹ und R¹⁰, R¹¹ und R¹², R¹³ und R¹⁴ , R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein-bis dreifach, gleich oder verschieden, mit Halogen, Cyano, (C₁-C₄)-Alkyl, Trifluor-methyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- R²: für einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino, Hydroxycarbonyl und -C(=O)-NH-R^{7A} steht, worin
(C₁-C₆)-Alkyl und (C₁-C₆)-Alkoxy ihrerseits mit Hydroxy substituiert sein können
und
R^{7A} Wasserstoff oder (C₁-C₄)-Alkyl bedeutet,
- m: für die Zahl 1 steht,
- n: für die Zahl 0 oder 1 steht
und
- R³: für Wasserstoff steht,
sowie ihre Salze, Solvate und Solvate der Salze.

Bevorzugt im Rahmen der vorliegenden Erfindung sind auch Verbindungen der Formel (I), in welcher
- A: für CH steht,
- R¹: für einen Substituenten ausgewählt aus der Reihe (C₁-C₆)-Alkyl, Trifluormethyl, Halogen, Cyano, Nitro, Hydroxy, (C₁-C₆)-Alkoxy, Amino, Hydroxycarbonyl, (C₁-C₆)-Alkoxycarbonyl und -C(=O)-NH-R⁷ steht, worin
(C₁-C₆)-Alkyl seinerseits mit Hydroxy, (C₁-C₄)-Alkoxy, Amino, Mono-(C₁-C₄)-alkylamino, Di-(C₁-C₄)-alkylamino oder einer Gruppe der Formel -NH-C(=O)-R¹² -NH-C(=O)-NH-R¹⁶ oder -NH-SO₂-R¹⁹ substituiert sein kann, worin
R¹², R¹⁶ und R¹⁹ jeweils (C₁-C₆)-Alkyl, welches mit Hydroxy oder (C₁-C₄)-Alkoxy substituiert sein kann, bedeuten,
und
R⁷ Wasserstoff oder (C₁-C₆)-Alkyl, das mit Hydroxy oder (C₁-C₄)-Alkoxy substituiert sein kann, bedeutet,
- R²: für einen Substituenten ausgewählt aus der Reihe -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} und -NR^{25A}R^{26A} steht, worin
(i) R^{9A}, R^{12A}, R^{14A}, R^{16A}, R^{19A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*ii*) R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
   und/oder worin
(*iii*) R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁₋C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxy-carbonyl substituiert sein kann,
- m: für die Zahl 1 steht,
- n: für die Zahl 0 oder 1 steht
und
- R³: für Wasserstoff steht,
sowie ihre Salze, Solvate und Solvate der Salze.

Bevorzugt im Rahmen der vorliegenden Erfindung sind auch Verbindungen der Formel (I), in welcher
- A: für CH steht,
- R¹: für (C₁-C₆)-Alkyl steht, welches
(i) ein- oder zweifach, gleich oder verschieden, mit einem Rest ausgewählt aus der Reihe Halogen, Cyano, Trifluormethyl, (C₃-C₆)-Cycloalkyl, 4- bis 6-gliedriges Heterocycloalkyl, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist
   und darüber hinaus mit Hydroxy, (C₁-C₄)-Alkoxy, Amino, Mono-(C₁-C₄)-alkylamino, Di-(C₁-C₄)-alkylamino oder (C₁-C₄)-Acylamino substituiert sein kann,
   oder
(*ii*) zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkylamino und (C₁-C₄)-Acylamino substituiert ist,
   worin
   (*a*) die oben genannten Cycloalkyl- und Heterocycloalkyl-Reste ihrerseits jeweils bis zu dreifach, gleich oder verschieden, mit Halogen, Cyano, (C₁-C₄)-Alkyl, Tri-fluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
   (*b*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
      wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   (c) R⁶, R⁹ und R¹⁴ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
      (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
      und
      (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
   (*d*) R¹², R¹⁶ und R¹⁹ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
      (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Tri-fluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
      und
      (C₁-C₆)-Alkyl ein- bis dreifach, gleich oder verschieden, mit einem Rest ausgewählt aus der Reihe Halogen, Cyano, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist,
   (e) R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
      (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Tri-fluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
      und
      (C₁-C₆)-Alkyl ein- bis dreifach, gleich oder verschieden, mit einem Rest ausgewählt aus der Reihe Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist,
      und/oder worin
   (*f*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- R²: für einen Substituenten ausgewählt aus der Reihe Halogen, Cyano, (C₁-C₆)-Alkyl, Trifluormethyl, Hydroxy, Trifluormethoxy, Amino, Hydroxycarbonyl, Aminocarbonyl, -C(=O)-NR^{6A}R^{7A}, -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A}, -OR^{23A} und -NR^{25A}R^{26A} steht, worin
(*i*) (C₁-C₆)-Alkyl mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann,
(*ii*) R^{6A}, R^{9A}, R^{12A}, R^{14A}, R^{16A}, R^{19A}, R^{23A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*iii*) R^{7A}, R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
   und/oder worin
(*iv*) R^{6A} und R^{7A}, R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- m: für die Zahl 1 steht,
- n: für die Zahl 0 oder 1 steht
und
- R³: für Wasserstoff steht,
sowie ihre Salze, Solvate und Solvate der Salze.

Bevorzugt im Rahmen der vorliegenden Erfindung sind auch Verbindungen der Formel (I), in welcher
- A: für CH steht,
- R¹: für (C₁-C₆)-Alkoxy steht, welches ein- oder zweifach, gleich oder verschieden, mit einem Rest ausgewählt aus der Reihe Halogen, Cyano, Trifluormethyl, (C₃-C₆)-Cycloalkyl, 4- bis 6-gliedriges Heterocycloalkyl, Hydroxy, Amino, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist, worin
(i) (C₃-C₆)-Cycloalkyl und 4- bis 6-gliedriges Heterocycloalkyl ihrerseits jeweils ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*ii*) R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
   (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
   und
   (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
(*iii*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
   wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   und/oder worin
(*iv*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher einbis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- R²: für einen Substituenten ausgewählt aus der Reihe Halogen, Cyano, (C₁-C₆)-Alkyl, Trifluormethyl, Hydroxy, Trifluormethoxy, Amino, Hydroxycarbonyl, Aminocarbonyl, -C(=O)-NR^{6A}R^{7A}, -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A}, -OR^{23A} und -NR^{25A}R^{26A} steht, worin
(i) (C₁-C₆)-Alkyl mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann,
(*ii*) R^{6A}, R^{9A}, R^{12A}, R^{14A}, R^{16A}, R^{19A}, R^{23A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*iii*) R^{7A}, R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
   und/oder worin
(*iv*) R^{6A} und R^{7A}, R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- m: für die Zahl 1 steht,
- n: für die Zahl 0 oder 1 steht
und
- R³: für Wasserstoff steht,
sowie ihre Salze, Solvate und Solvate der Salze.

Bevorzugt im Rahmen der vorliegenden Erfindung sind auch Verbindungen der Formel (I), in welcher
- A: für CH steht,
- R¹: für die Gruppe -C(=O)-NR⁶R⁷ steht,
worin (i)
R⁶ (C₃-C₆)-Cycloalkyl oder 4- bis 6-gliedriges Heterocycloalkyl bedeutet, welche jeweils ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
oder
(C₁-C₆)-Alkyl bedeutet, welches
(*a*) ein- oder zweifach, gleich oder verschieden, mit einem Rest ausgewählt aus der Reihe Halogen, Cyano, Trifluormethyl, Amino, Mono-(C₁-C₄)-alkyl-amino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist
   und darüber hinaus mit Hydroxy oder (C₁-C₄)-Alkoxy substituiert sein kann,
   oder
(b) zweifach mit Hydroxy und/oder (C₁-C₄)-Alkoxy substituiert ist,
   und

R⁷ Wasserstoff bedeutet,
oder worin (*ii*)
R⁶ (C₁-C₆)-Alkyl, (C₃-C₆)-Cycloalkyl oder 4- bis 6-gliedriges Heterocycloalkyl bedeutet, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxy-carbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
und
R⁷ (C₁-C₆)-Alkyl bedeutet, welches ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
oder worin (*iii*)
R⁶ und R⁷ gemeinsam mit dem Stickstoffatom, an das sie gebunden sind, einen 5- oder 6-gliedrigen Heterocycloalkyl-Ring bilden, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- R²: für einen Substituenten ausgewählt aus der Reihe Halogen, Cyano, (C₁-C₆)-Alkyl, Trifluormethyl, Hydroxy, Trifluormethoxy, Amino, Hydroxycarbonyl, Aminocarbonyl, -C(=O)-NR^{6A}R^{7A}, -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}; -NR^{18A}-SO₂-R^{19A}; -OR^{23A} und -NR^{25A}R^{26A} steht, worin
(*i*) (C₁-C₆)-Alkyl mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann,
(*ii*) R^{6A}, R^{9A}, R^{12A}, R^{14A}, R^{16A}, R^{19A}, R^{23A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*iii*) R^{7A}, R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
   und/oder worin
(*iv*) R^{6A} und R^{7A}, R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- m: für die Zahl 1 steht,
- n: für die Zahl 0 oder 1 steht
und
- R³: für Wasserstoff steht,
sowie ihre Salze, Solvate und Solvate der Salze.

Besonders bevorzugt im Rahmen der vorliegenden Erfindung sind Verbindungen der Formel (I-A) oder (I-B) 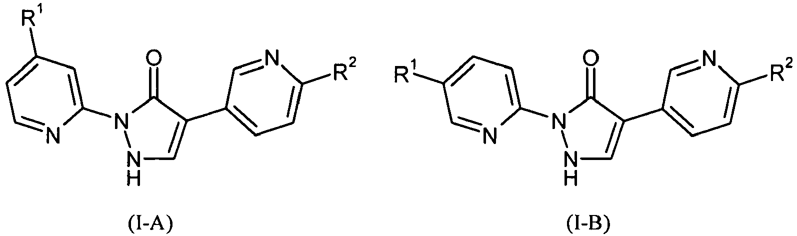 in welchen
- R¹: für 4- bis 6-gliedriges Heterocycloalkyl, Phenyl oder 5- oder 6-gliedriges Heteroaryl steht, welche jeweils ein- oder zweifach, gleich oder verschieden, mit einem Rest ausgewählt aus der Reihe Fluor, Chlor, Brom, Cyano, (C₁-C₄)-Alkyl, Hydroxy, Amino, Hydroxycarbonyl, -OR²³ und -NR²⁵R²⁶ substituiert sein können, worin
(C₁-C₄)-Alkyl seinerseits ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl, 4- bis 6-gliedrigem Heterocycloalkyl, Phenyl und/oder 5- oder 6-gliedrigem Heteroaryl substituiert sein kann,
R²³ und R²⁵ unabhängig voneinander bei jedem einzelnen Auftreten (C₁-C₄)-Alkyl bedeuten, welches ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkyl-amino, Hydroxycarbonyl, (C₃-C₆)-Cycloalkyl und/oder 4- bis 6-gliedrigem Heterocycloalkyl substituiert sein kann,
und
R²⁶ bei jedem einzelnen Auftreten Wasserstoff oder (C₁-C₄)-Alkyl bedeutet, welches ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann, und
- R²: für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

Besonders bevorzugt im Rahmen der vorliegenden Erfindung sind auch Verbindungen der Formel (I-A) oder (I-B) 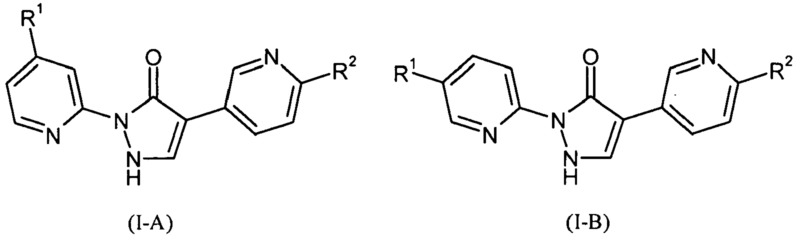 in welchen
- R¹: für (C₁-C₆)-Alkyl steht, welches
(i) ein- oder zweifach, gleich oder verschieden, mit einem Rest ausgewählt aus der Reihe Fluor, Chlor, Cyano, Trifluormethyl, (C₃-C₆)-Cycloalkyl, 4- bis 6-gliedriges Heterocycloalkyl, Hydroxycarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist
   und darüber hinaus mit Hydroxy, (C₁-C₄)-Alkoxy, Amino, Mono-(C₁-C₄)-alkylamino, Di-(C₁-C₄)-alkylamino oder (C₁-C₄)-Acylamino substituiert sein kann,
   oder
(*ii*) zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkylamino und (C₁-C₄)-Acylamino substituiert ist,
   worin
   (*a*) die oben genannten Cycloalkyl- und Heterocycloalkyl-Reste ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können,
   (*b*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₄)-Alkyl stehen,
      wobei (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
   (*c*) R⁶, R⁹ und R¹⁴ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
      (C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Tri-fluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
      und
      (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkyl-amino, Hydroxycarbonyl, (C₃-C₆)-Cycloalkyl und/oder 4- bis 6-gliedrigem Heterocycloalkyl substituiert sein kann,
   (*d*) R¹², R¹⁶ und R¹⁹ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
      (C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
      und
      (C₁-C₄)-Alkyl ein- bis dreifach, gleich oder verschieden, mit einem Rest ausgewählt aus der Reihe Fluor, Chlor, Cyano, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist,
   *(e)* R²³ und R²⁵ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
      (C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
      und
      (C₁-C₄)-Alkyl ein- bis dreifach, gleich oder verschieden, mit einem Rest ausgewählt aus der Reihe Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und 4- bis 6-gliedriges Heterocycloalkyl substituiert ist,
      und/oder worin
   (*f*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Fluor, Chlor, Cyano, (C₁-C₄)-Alkyl, Trifluormethyl, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
   und
- R²: für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

Besonders bevorzugt im Rahmen der vorliegenden Erfindung sind auch Verbindungen der Formel (I-A) oder (I-B) 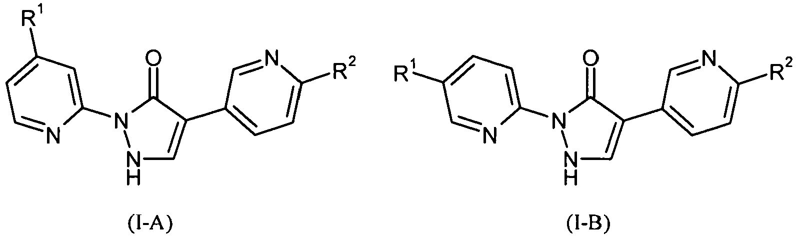 in welchen
- R¹: für (C₁-C₆)-Alkoxy steht, welches ein- oder zweifach, gleich oder verschieden, mit einem Rest ausgewählt aus der Reihe Fluor, Chlor, Cyano, Trifluormethyl, (C₃-C₆)-Cycloalkyl, 4-bis 6-gliedriges Heterocycloalkyl, Hydroxy, Amino, Hydroxycarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist, worin
(i) (C₃-C₆)-Cycloalkyl und 4- bis 6-gliedriges Heterocycloalkyl ihrerseits jeweils ein- bis dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können,
(*ii*) R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
   (C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
   und
   (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und/oder 4- bis 6-gliedrigem Heterocycloalkyl substituiert sein kann,
(*iii*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₄)-Alkyl stehen, wobei (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
   und/oder worin
(*iv*) R⁶ und R⁷, R⁹ und R¹⁰, R¹¹ und R¹², R¹³ und R¹⁴, R¹⁵ und R¹⁶, R¹⁶ und R¹⁷ , R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher einbis dreifach, gleich oder verschieden, mit Fluor, Chlor, Cyano, (C₁-C₄)-Alkyl, Trifluormethyl, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
   und
- R²: für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

Besonders bevorzugt im Rahmen der vorliegenden Erfindung sind auch Verbindungen der Formel (I-A) oder (I-B) 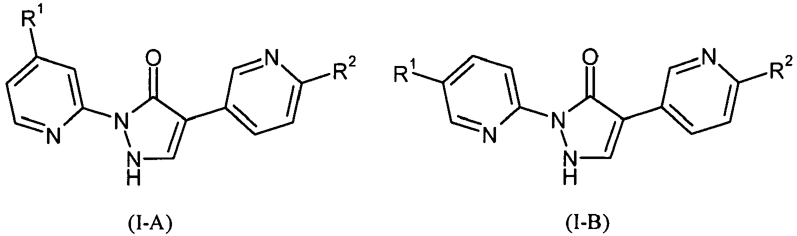 in welchen
- R¹: für die Gruppe -C(=O)-NR⁶R⁷ steht,
worin (i)
R⁶ (C₃-C₆)-Cycloalkyl oder 4- bis 6-gliedriges Heterocycloalkyl bedeutet, welche jeweils ein- bis dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können,
oder
(C₁-C₆)-Alkyl bedeutet, welches
(*a*) ein- oder zweifach, gleich oder verschieden, mit einem Rest ausgewählt aus der Reihe Fluor, Chlor, Cyano, Trifluormethyl, Amino, Mono-(C₁-C₄)-alkyl-amino, Di-(C₁-C₄)-alkylamino, Hydroxycarbonyl, (C₃-C₆)-Cycloalkyl und 4-bis 6-gliedriges Heterocycloalkyl substituiert ist
   und darüber hinaus mit Hydroxy oder (C₁-C₄)-Alkoxy substituiert sein kann,
   oder
(*b*) zweifach mit Hydroxy und/oder (C₁-C₄)-Alkoxy substituiert ist,
   und

R⁷ Wasserstoff bedeutet,
oder worin (*ii*)
R⁶ (C₁-C₆)-Alkyl, (C₃-C₆)-Cycloalkyl oder 4- bis 6-gliedriges Heterocycloalkyl bedeutet, wobei
(C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können und
(C₁-C₆)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und/oder 4- bis 6-gliedrigem Heterocycloalkyl substituiert sein kann,
und
R⁷ (C₁-C₄)-Alkyl bedeutet, welches ein- bis dreifach, gleich oder verschieden, mit Fluor, Chlor, Cyano, Hydroxy, (C₁-C₄)-Alkoxy, Amino, Mono-(C₁-C₄)-alkyl-amino, Di-(C₁-C₄)-alkylamino und/oder Hydroxycarbonyl substituiert sein kann,
oder worin (*iii*)
R⁶ und R⁷ gemeinsam mit dem Stickstoffatom, an das sie gebunden sind, einen 5- oder 6-gliedrigen Heterocycloalkyl-Ring bilden, welcher ein- bis dreifach, gleich oder verschieden, mit Fluor, Chlor, Cyano, (C₁-C₄)-Alkyl, Trifluormethyl, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
und
R² für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen 1,2-Dihydropyrazol-3-on-Derivate der Formel (I) können auch in der tautomeren 1*H*-Pyrazol-5-ol-Form (I') vorliegen (siehe nachfolgendes Schema 1); beide tautomeren Formen werden von der vorliegenden Erfindung ausdrücklich umfasst.

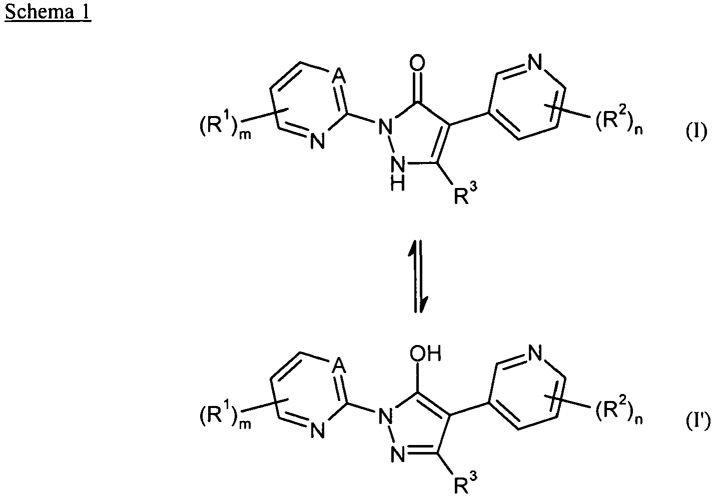

Weiterer Gegenstand der Erfindung ist ein Verfahren zur Herstellung der erfindungsgemäßen Verbindungen der Formel (I), dadurch gekennzeichnet, dass man eine Verbindung der Formel (II) 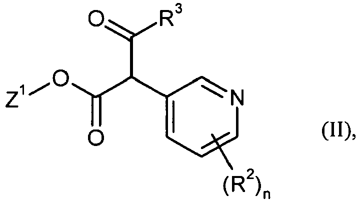 in welcher R², R³ und n die oben angegebenen Bedeutungen aufweisen und
- Z¹: für Methyl oder Ethyl steht,
in einem inerten Lösungsmittel gegebenenfalls in Gegenwart einer Säure mit einer Verbindung der Formel (III) 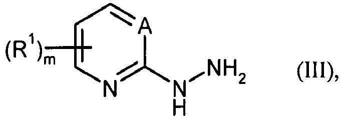 in welcher A, R¹ und m die oben angegebenen Bedeutungen aufweisen,
zu Verbindungen der Formel (IV) 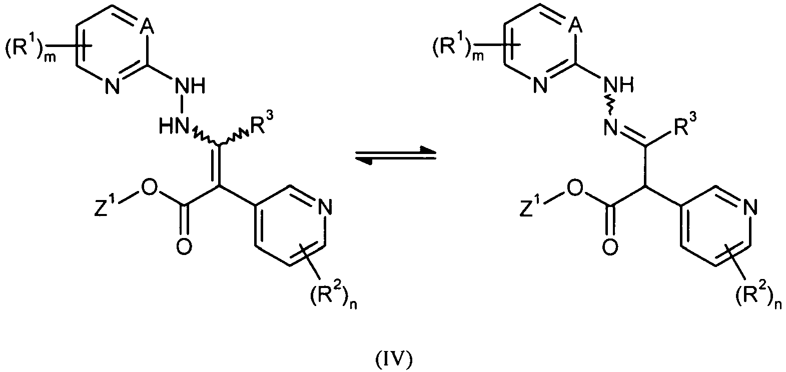 in welcher Z¹, A, R¹, R², R³, m und n die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Solvate, Salze und/oder Solvate der Salze überführt.

Die erfindungsgemäßen Verbindungen der Formel (I), in welcher R³ Wasserstoff bedeutet, können auch dadurch hergestellt werden, dass man zunächst eine Verbindung der Formel (V) 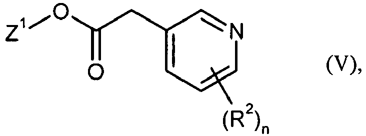 in welcher Z¹, R² und n die oben angegebenen Bedeutungen aufweisen, mit einer Verbindung der Formel (VI) 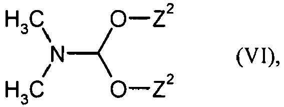 in welcher
- Z²: für Methyl oder Ethyl steht,
zu Verbindungen der Formel (VII) 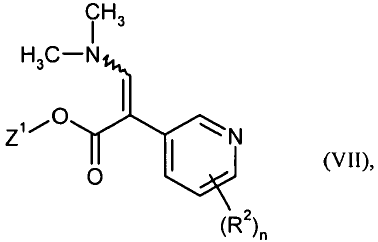 in welcher Z¹, R² und n die oben angegebenen Bedeutungen aufweisen,
kondensiert und anschließend in Gegenwart einer Säure mit einer Verbindung der Formel (III) zu Verbindungen der Formel (IV-A) 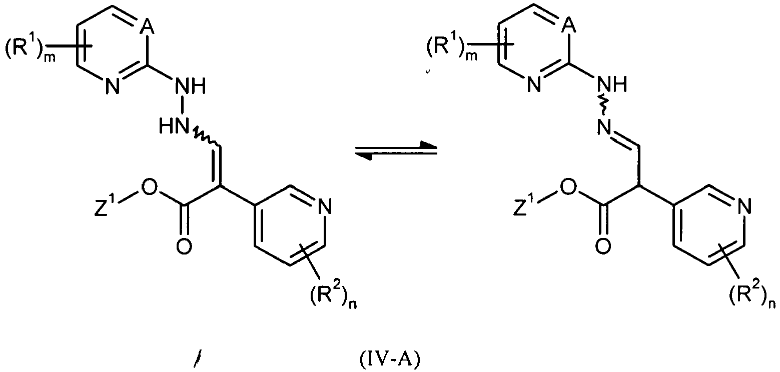 in welcher Z¹, A, R¹, R², m und n die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I), worin R³ für Wasserstoff steht, cyclisieren.

Weitere erfindungsgemäße Verbindungen können gegebenenfalls auch hergestellt werden durch Umwandlungen von funktionellen Gruppen einzelner Substituenten, insbesondere den unter R¹ und R² aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile oder elektrophile Substitution, Oxidation, Reduktion, Hydrierung, Übergangsmetall-katalysierte Kupplungsreaktionen, Alkylierung, Acylierung, Aminierung, Veresterung, Esterspaltung, Veretherung, Etherspaltung, Bildung von Carbonamiden, Sulfonamiden, Carbamaten und Harnstoffen, sowie die Einführung und Entfernung temporärer Schutzgruppen.

Als inerte Lösungsmittel für die Verfahrensschritte (II) + (III) → (IV), (IV) → (I), (VII) + (III) → (IV-A) und (IV-A) → (I) eignen sich insbesondere Ether wie Diethylether, Methyl-tert.-butylether, 1,2-Dimethoxyethan, Tetrahydrofuran und Dioxan, oder Alkohole wie Methanol, Ethanol, n-Propanol, iso-Propanol, n-Butanol und tert.-Butanol. Bevorzugt werden Methanol, Ethanol, Tetrahydrofuran oder Gemische dieser Lösungsmittel eingesetzt.

Der Verfahrensschritt (V) + (VI) → (VII) wird bevorzugt in Dimethylformamid als Lösungsmittel oder auch in Gegenwart eines Überschusses an (VI) ohne weiteres Lösungsmittel durchgeführt. Gegebenenfalls kann die Reaktion auch vorteilhaft unter Mikrowellen-Bestrahlung erfolgen. Die Umsetzung geschieht im Allgemeinen in einem Temperaturbereich von +20°C bis +150°C, bevorzugt bei +80°C bis +120°C [vgl. auch J.P. Bazureau et al., Synthesis 1998, 967; *ibid.* 2001 (4), 581].

Die Verfahrensschritte (II) + (III) → (IV) und (VII) + (III) → (IV-A) können gegebenenfalls vorteilhaft unter Zusatz einer Säure durchgeführt werden. Hierfür eignen sich übliche anorganische oder organische Säuren wie beispielsweise Chlorwasserstoff, Essigsäure, Trifluoressigsäure, Methansulfonsäure, p-Toluolsulfonsäure oder Campher-10-sulfonsäure. Bevorzugt werden Essigsäure oder insbesondere Campher-10-sulfonsäure oder p-Toluolsulfonsäure verwendet.

Die Umsetzung (II) + (III) → (IV) erfolgt im Allgemeinen in einem Temperaturbereich von 0°C bis +100°C, bevorzugt bei +10°C bis +50°C. Die Reaktion (VII) + (III) → (IV-A) wird im Allgemeinen in einem Temperaturbereich von +20°C bis +120°C, bevorzugt bei +50°C bis +100°C durchgeführt.

Die Verfahrenssequenzen (II) + (III) → (IV) → (I) und (VII) + (III) → (IV-A) → (I) können unter zweistufiger Reaktionsführung oder auch als Eintopf-Reaktion, ohne Isolierung der Zwischenstufe (IV) bzw. (IV-A), durchgeführt werden. Für letztere Variante ist insbesondere die Umsetzung der Komponenten unter Mikrowellen-Bestrahlung geeignet; die Reaktion erfolgt hierbei im Allgemeinen in einem Temperaturbereich von +50°C bis +200°C, bevorzugt bei +100°C bis +180°C.

Teilweise tritt ein Ringschluss zu (I) auch bereits schon bei der Herstellung von (IV) bzw. (IV-A) ein; die Cyclisierung kann dann gegebenenfalls durch *in situ*-Behandlung des Reaktionsgemisches mit einer Base vervollständigt werden.

Als Base für einen solchen separaten Cyclisierungsschritt (IV) → (I) bzw. (IV-A) → (I) eignen sich übliche anorganische oder organische Basen. Hierzu gehören insbesondere Alkalihydroxide wie beispielsweise Natrium- oder Kaliumhydroxid, Alkali- oder Erdalkalicarbonate wie Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, oder Alkalihydride wie Natriumhydrid. Bevorzugt werden Natriummethanolat oder -ethanolat verwendet.

Die Basen-induzierte Umsetzung (IV) → (I) bzw. (IV-A) → (I) erfolgt im Allgemeinen in einem Temperaturbereich von 0°C bis +60°C, bevorzugt bei 0°C bis +30°C.

Alle Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formel (II) können nach literaturüblichen Methoden zur C-Acylierung von Carbonsäureestern aus Verbindungen der Formel (V) hergestellt werden. Die Verbindungen der Formeln (III), (V) und (VI) sind kommerziell erhältlich, literaturbekannt oder können in Analogie zu in der Literatur beschriebenen Verfahren hergestellt werden.

Die Herstellung der erfindungsgemäßen Verbindungen kann durch das folgende Reaktionsschema 2 veranschaulicht werden: 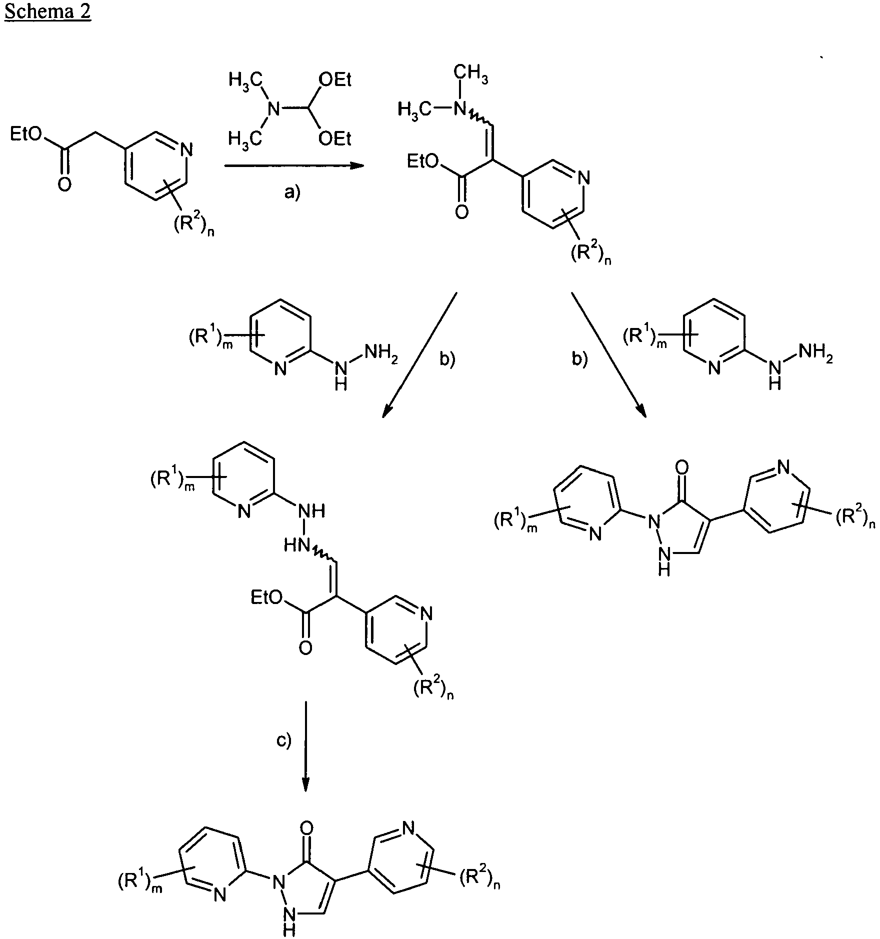 [a): DMF, 16 h, +100°C; b): Ethanol, cat. Campher-10-sulfonsäure, +78°C; c): NaOEt, Ethanol, 1 h, RT].

Die erfindungsgemäßen Verbindungen zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen zeichnen sich als spezifische Hemmstoffe der HIF-Prolyl-4-Hydroxylasen aus.

Die erfindungsgemäßen Verbindungen können aufgrund ihrer pharmakologischen Eigenschaften zur Behandlung und/oder Prophylaxe von kardiovaskulären Krankheiten, insbesondere von Herzinsuffizienz, koronarer Herzkrankheit, Angina pectoris, Myokardinfarkt, Schlaganfall, Arteriosklerose, essentieller, pulmonaler und maligner Hypertonie sowie peripherer arterieller Verschlusskrankheit eingesetzt werden.

Die erfindungsgemäßen Verbindungen eignen sich weiterhin zur Behandlung und/oder Prophylaxe von Störungen der Blutbildung, wie z.B. idiopathischen Anämien, renaler Anämie und Anämien in Begleitung einer Tumor-Erkrankung (insbesondere einer Chemotherapie-induzierten Anämie), einer Infektion (insbesondere HIV-Infektion) oder einer anderen entzündlichen Erkrankung wie z.B. rheumatoider Arthritis. Die erfindungsgemäßen Verbindungen sind darüber hinaus geeignet zur unterstützenden Behandlung von Anämien infolge Blutverlust, Eisenmangel-Anämie, Vitaminmangel-Anämie (z.B. infolge Vitamin B12-Mangel oder infolge Folsäure-Mangel), hypoplastischer und aplastischer Anämie, hämolytischer Anämie oder zur unterstützenden Behandlung von Anämien in Folge von Eisenverwertungsstörungen (sideroachrestische Anämie) oder Anämien infolge anderer endokriner Störungen (z.B. Hypothyreose).

Die Verbindungen sind ferner geeignet zur Steigerung des Hämatokrits mit dem Ziel der Gewinnung von Blut zur Eigenblutspende vor Operationen.

Die erfindungsgemäßen Verbindungen können außerdem zur Behandlung und/oder Prophylaxe von operationsbedingten Ischämiezuständen und deren Folgeerscheinungen nach chirurgischen Eingriffen, insbesondere Eingriffen am Herzen unter Verwendung einer Herz-Lungenmaschine (z.B. Bypass-Operationen, Herzklappen-Implantationen), Eingriffen an den Halsschlagadern, Eingriffen an der Körperschlagader (Aorta) und Eingriffen mit instrumenteller Öffnung oder Durchdringung der Schädelkalotte verwendet werden. Die Verbindungen eignen sich ferner zur allgemeinen Behandlung und/oder Prophylaxe bei chirurgischen Eingriffen mit dem Ziel einer Beschleunigung der Wundheilung und Verkürzung der Rekonvaleszenzzeit.

Die Verbindungen sind darüber hinaus zur Behandlung und Prophylaxe von Folgeerscheinungen akuter und protrahierter Ischämiezustände des Gehirns geeignet (z.B. Schlaganfall, Geburtsasphyxie).

Die Verbindungen können ferner zur Behandlung und/oder Prophylaxe von Krebs und zur Behandlung und/oder Prophylaxe einer im Zuge der Behandlung von Krebs auftretenden Beeinträchtigung des Gesundheitszustandes insbesondere nach Therapie mit Zytostatika, Antibiotika und Bestrahlungen eingesetzt werden.

Die Verbindungen eignen sich ferner zur Behandlung und/oder Prophylaxe von Erkrankungen des rheumatischen Formenkreises und anderen den Autoimmunerkrankungen zuzurechnenden Krankheitsformen und insbesondere zur Behandlung und/oder Prophylaxe einer im Zuge der medikamentösen Behandlung derartiger Erkrankungen auftretenden Beeinträchtigung des Gesundheitszustandes.

Weiterhin können die erfindungsgemäßen Verbindungen eingesetzt werden zur Behandlung und/ oder Prophylaxe von Erkrankungen des Auges (z.B. Glaukom), des Gehirns (z.B. Morbus Parkinson, Morbus Alzheimer, Demenz, chronisches Schmerzempfinden), von chronischen Nierenerkrankungen, Niereninsuffizienz und akutem Nierenversagen sowie zur Förderung der Wundheilung.

Weiterhin eignen sich die Verbindungen zur Behandlung und/oder Prophylaxe einer insbesondere im höheren Lebensalter gehäuft auftretenden allgemeinen körperlichen Schwäche bis hin zur Kachexie.

Ferner eignen sich die Verbindungen zur Behandlung und/oder Prophylaxe sexueller Dysfunktion. Außerdem sind die Verbindungen zur Behandlung und/oder Prophylaxe von Diabetes mellitus und seinen Folgeerkrankungen, wie z.B. diabetischer Makro- und Mikroangiopathie, diabetischer Nephropathie und Neuropathie, geeignet.

Weiterhin eignen sich die erfindungsgemäßen Verbindungen zur Behandlung und/oder Prophylaxe von fibrotischen Erkrankungen z.B. des Herzens, der Lunge und der Leber.

Insbesondere sind die erfindungsgemäßen Verbindungen auch zur Prophylaxe und Behandlung der Frühgeborenenretinopathie (Retinopathia praematurorum) geeignet.

Weiterhin offenbart ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterhin offenbart ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika sowie Antibiotika.

Die vorliegenden Verbindungen können in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht werden.

Die vorliegenden Verbindungen können in Kombination mit einem Angiotensin AII-Antagonisten, wie beispielhaft und vorzugsweise Losartan, Candesartan, Valsartan, Telmisartan oder Embusartan, verabreicht werden.

Die vorliegenden Verbindungen können in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht werden.

Die vorliegenden Verbindungen können in Kombination mit einem Calcium-Antagonisten, wie beispielhaft und vorzugsweise Nifedipin, Amlodipin, Verapamil oder Diltiazem, verabreicht werden.

Die vorliegenden Verbindungen können in Kombination mit einem Phosphodiesterase (PDE)-Inhibitor, wie beispielhaft und vorzugsweise Milrinone, Amrinone, Pimobendan, Cilostazol, Sildenafil, Vardenafil oder Tadalafil, verabreicht werden.

Die vorliegenden Verbindungen können in Kombination mit einem Mineralocorticoid-Rezeptor-Antagonisten, wie beispielhaft und vorzugsweise Spironolacton, Eplerenon, Canrenon oder Kalium-Canrenoat, verabreicht werden.

Die vorliegenden Verbindungen können in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht werden.

Die vorliegenden Verbindungen können in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht werden.

Die vorliegenden Verbindungen können in Kombination mit einem Tumor-Chemotherapeutikum verabreicht werden, beispielhaft und vorzugsweise aus der Gruppe der Platin-Komplexe, wie z.B. Cisplatin und Carboplatin, der Alkylantien, wie z.B. Cyclophosphamid und Chlorambucil, der Antimetabolite, wie z.B. 5-Fluoruracil und Methotrexat, der Topoisomerase-Hemmer, wie z.B. Etoposid und Camptothecin, der Antibiotika, wie z.B. Doxorubicin und Daunorubicin, oder der Kinase-Inhibitoren, wie z.B. Sorafenib und Sunitinib.

Die vorliegenden Verbindungen können in Kombination mit einem Antibiotikum verabreicht werden, beispielhaft und vorzugsweise aus der Gruppe der Penicilline, Cephalosporine oder Chinolone, wie z.B. Ciprofloxacin und Moxifloxacin. Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- cat.: katalytisch
- d: Tag(e)
- DCI: direkte chemische Ionisation (bei MS)
- DMF: Dimethylformamid
- DMSO: Dimethylsulfoxid
- d. Th.: der Theorie (bei Ausbeute)
- EI: Elektronenstoß-Ionisation (bei MS)
- ESI: Elektrospray-Ionisation (bei MS)
- Et: Ethyl
- GC-MS: Gaschromatographie-gekoppelte Massenspektroskopie
- h: Stunde(n)
- HPLC: Hochdruck-, Hochleistungsflüssigchromatographie
- konz.: konzentriert
- LC-MS: Flüssigchromatographie-gekoppelte Massenspektroskopie
- Meth.: Methode
- min: Minute(n)
- MS: Massenspektroskopie
- NMR: Kernresonanzspektroskopie
- Rₜ: Retentionszeit (bei HPLC)
- RT: Raumtemperatur
- TFA: Trifluoressigsäure
- THF: Tetrahydrofuran

### LC-MS-, GC-MS- und HPLC-Methoden:

### Methode 1:

Instrument: Micromass Platform LCZ mit HPLC Agilent Serie 1100; Säule: Thermo Hypersil GOLD 3 µ, 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 2:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3:

Instrument MS: Micromass TOF (LCT); Instrument HPLC: Waters 2690; Autosampler: Waters 2700; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode 4:

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode 5:

Instrument: Micromass QuattroPremier mit Waters UPLC Acquity; Säule: Thermo Hypersil GOLD 1.9µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 6:

Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 7:

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.1 min 100% A; Fluss: 2.5 ml/min; Ofen: 55°C; UV-Detektion: 210 nm.

### Methode 8:

Instrument MS: Micromass TOF (LCT); Instrument HPLC: Waters 2690; Autosampler: Waters 2700; Säule: YMC-ODS-AQ, 3µ, 50 mm x 4.6 mm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2 min 5% A → 3.2 min 5% A → 3.21 min 100% A → 3.35 min 100% A; Ofen: 40°C; Fluss: 3 ml/min; UV-Detektion: 210 nm.

### Methode 9:

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode 10:

Instrument: HP 1100 mit DAD-Detektion; Säule: Kromasil 100 RP-18, 60 mm x 2.1 mm, 3.5 µm; Eluent A: 5 ml Perchlorsäure (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 11:

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Ausgangsverbindungen und Intermediate:

### Beispiel 1A

### 2-Hydrazino-isonicotinsäurenitril

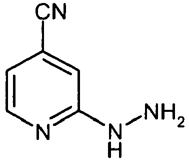

20.0 g (144 mmol) 2-Chlorisonicotinsäurenitril werden in 150 ml 1-Butanol vorgelegt, mit 303 ml (303 mmol) einer 1 M Lösung von Hydrazinhydrat in THF versetzt und 16 h lang erhitzt (110°C Badtemperatur). Es wird eingeengt und der Rückstand mittels Flash-Chromatographie an Kieselgel
(Laufmittel: Dichlormethan/Methanol 10:1) gereinigt.
Ausbeute: 9.48 g (49% d. Th.)
¹H-NMR (400 MHz, DMSO-d₆): δ = 8.15 (d, 1H), 8.05 (s, 1H), 7.01 (s, 1H), 6.83 (dd, 1H), 4.30 (s, 2H).
LC-MS (Methode 1): Rₜ = 0.52 min; MS (ESIpos): m/z = 135 [M+H]⁺.

### Beispiel 2A

### 3-(Dimethylamino)-2-pyridin-3-yl-acrylsäureethylester

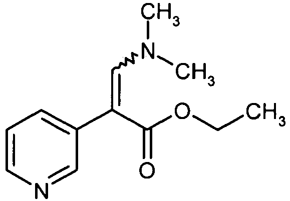

37.4 g (226 mmol) Pyridin-3-ylessigsäureethylester werden in 100 g (679 mmol) Dimethylformamid-diethylacetal über Nacht bei 100°C erhitzt. Nach dem Abkühlen engt man ein und reinigt den Rückstand mittels Flash-Chromatographie an Kieselgel vor (Laufmittel: Gradient Cyclohexan/ Essigsäureethylester 1:1 → Essigsäureethylester/Ethanol 9:1). Das erhaltene Produkt wird durch Vakuumdestillation (1 mbar, 200°C Badtemperatur) feingereinigt.
Ausbeute: 35.0 g (70% d. Th.)
¹H-NMR (400 MHz, DMSO-d₆): δ = 8.37 (dd, 1H), 8.31 (dd, 1H), 7.59 (s, 1H), 7.51 (dt, 1H), 7.29 (ddd, 1H), 4.00 (q, 2H), 2.67 (s, 6H), 1.11 (t, 3H).
LC-MS (Methode 1): Rₜ = 2.38 min; MS (ESIpos): m/z = 221 [M+H]⁺.

### Beispiel 3A

### 2-(4-Cyanopyridin-2-yl)-4-pyridin-3-yl-1,2-dihydro-3H-pyrazol-3-on

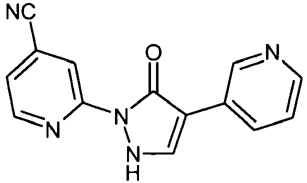

545 mg (4.06 mmol) der Verbindung aus Beispiel 1A und 1.07 g (4.88 mmol) der Verbindung aus Beispiel 2A werden in 15 ml Eisessig 2 h lang bei RT gerührt. Der Ansatz wird eingeengt, der Rückstand in 300 ml Essigsäureethylester aufgenommen und mehrfach mit gesättigter Natriumhydrogencarbonat-Lösung gewaschen. Die organische Phase wird über Natriumsulfat getrocknet, filtriert und eingeengt. Der Rückstand wird in 30 ml Ethanol aufgenommen, bei RT mit 1.33 g (4.88 mmol) einer 25%-igen Lösung von Natriumethanolat in Ethanol versetzt und 30 min gerührt. Durch Zugabe von 1 M Salzsäure stellt man einen pH-Wert von 5 ein, saugt den entstandenen Feststoff ab, wäscht ihn mit Diethylether und trocknet im Hochvakuum.
Ausbeute: 890 mg (83% d. Th.)
¹H-NMR (400 MHz, DMSO-d₆): δ = 9.01-8.98 (m, 2H), 8.54 (dd, 1H), 8.18 (dt, 1H), 8.01 (dd, 1H), 7.85 (s, 1H), 7.39 (dd, 1H), 7.14 (dd, 1H).
LC-MS (Methode 2): Rₜ = 1.13 min; MS (ESIpos): m/z = 264 [M+H]⁺.

### Beispiel 4A

### 2-[4-(Aminomethyl)pyridin-2-yl]-4-pyridin-3-yl-1,2-dihydro-3H-pyrazol-3-on-Dihydrochlorid

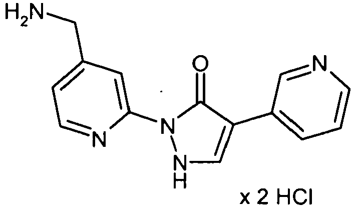

100 mg (380 µmol) der Verbindung aus Beispiel 3A werden in 10 ml Eisessig gelöst, mit 50.0 mg Katalysator (10% Palladium auf Kohle) versetzt und über Nacht unter Wasserstoffatmosphäre bei Normaldruck und RT gerührt. Anschließend wird die Reaktionsmischung filtriert, eingeengt und der Rückstand mittels präparativer HPLC (RP18-Säule; Laufmittel: Acetonitril/Wasser-Gradient unter Zusatz von 0.1% konz. Salzsäure) gereinigt.
Ausbeute: 64 mg (49% d. Th.)
¹H-NMR (400 MHz, DMSO-d₆): δ = 9.38 (s, 1H), 8.93 (d, 1H), 8.79 (s, 1H), 8.75 (s, 3H), 8.64 (d, 1H), 8.56 (d, 1H), 8.48 (s, 1H), 7.99 (dd, 1H), 7.54 (d, 1H), 4.21 (q, 2H).
LC-MS (Methode 1): Rₜ = 1.39 min; MS (ESIpos): m/z = 268 [M+H]⁺.

### Beispiel 5A

### 1-(6-Hydrazinopyridin-3-yl)-N-methylmethanamin

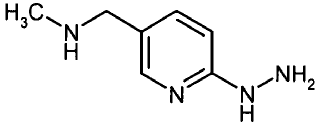

1.0 g (6.4 mmol) 1-(6-Chlorpyridin-3-yl)-*N*-methylmethanamin [Herstellung siehe EP 0 556 684-A1] werden in 1.5 ml (1.6 g, 31.9 mmol) Hydrazinhydrat vorgelegt und 12 h in der Siedehitze bei einer Badtemperatur von 150°C gerührt. Die abgekühlte Reaktionslösung wird eingeengt und der Rückstand im Vakuum getrocknet. Man erhält 1.1 g der Titelverbindung, welche ohne weitere Reinigung eingesetzt wird.
LC-MS (Methode 1): Rₜ = 0.52 min; MS (ESIpos): m/z = 153 [M+H]⁺.

### Beispiel 6A

### N-[(6-Hydrazinopyridin-3-yl)methyl]-2-methoxyethanamin

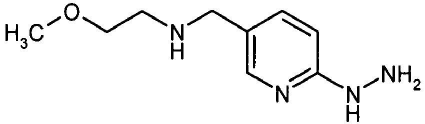

3.6 g (17.7 mmol) *N*-[(6-Chlorpyridin-3-yl)methyl]-2-methoxyethanamin [hergestellt analog WO 2004/081007] werden in 4.3 ml (4.4 g, 88.5 mmol) Hydrazinhydrat vorgelegt und 16 h in der Siedehitze bei einer Badtemperatur von 150°C gerührt. Die abgekühlte Reaktionslösung wird eingeengt und der Rückstand säulenchromatographisch an Kieselgel aufgereinigt (Laufmittel: Acetonitril/Wasser 9:1).
Ausbeute: 1.6 g (47% d. Th.)
LC-MS (Methode 1): Rₜ = 0.44 min; MS (ESIpos): m/z = 197 [M+H]⁺.

### Beispiel 7A

### 2-Hydrazino-5-(methylsulfonyl)pyridin

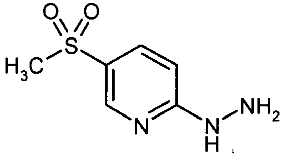

2.0 g (8.5 mmol) 2,5-Bis(methylsulfonyl)pyridin [Woods et al., J. Heterocycl. Chem. 1984, 21, 97-101] werden in 15 ml Ethanol mit 1.7 ml (1.7 g, 34.0 mmol) Hydrazinhydrat versetzt und 4 h unter Rückfluss gerührt. Zur Aufarbeitung wird die Reaktionslösung auf 15°C abgekühlt, der ausgefallene Feststoff abfiltriert, der Filterrückstand mit Ethanol und Diethylether gewaschen und das Produkt im Vakuum getrocknet.
Ausbeute: 1.4 g (89% d. Th.)
LC-MS (Methode 1): Rₜ = 0.51 min; MS (ESIpos): m/z = 188 [M+H]⁺;
¹H-NMR (400 MHz, DMSO-d₆): δ = 8.56 (s, 1H), 8.38 (d, 1H), 7.81 (dd, 1H), 6.79 (d, 1H), 4.42 (s, 2H), 3.11 (s, 3H).

### Beispiel 8A

### (5-Brompyridin-3-yl)essigsäureethylester

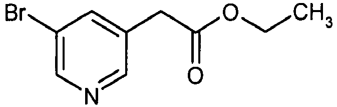

5.0 g (23.1 mmol) (5-Brompyridin-3-yl)essigsäure werden in 30 ml Ethanol mit 15 Tropfen konzentrierter Schwefelsäure versetzt und 16 h unter Rückfluss erhitzt. Nach Abkühlen auf RT wird im Vakuum eingeengt, der Rückstand in Essigsäureethylester aufgenommen und mit halbkonzentrierter wässriger Natriumhydrogencarbonat-Lösung gewaschen. Die organische Phase wird über Natriumsulfat getrocknet, filtriert und im Vakuum eingeengt.
Ausbeute: 5.2 g (91% d. Th.)
LC-MS (Methode 2): Rₜ = 1.48 min; MS (ESIpos): m/z = 244 [M+H]⁺;
¹H-NMR (400 MHz, DMSO-d₆): δ = 8.61 (d, 1H), 8.48 (d, 1H), 8.00 (dd, 1H), 4.10 (q, 2H), 3.33 (s, 2H), 1.20 (t, 3H).

### Beispiel 9A

### (2Z)-2-(5-Brompyridin-3-yl)-3-(dimethylamino)prop-2-ensäureethylester

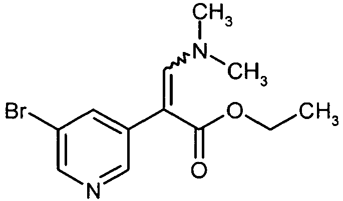

5.1 g (20.8 mmol) der Verbindung aus Beispiel 8A werden in 7.1 ml (6.2 g, 41.8 mmol) Dimethyl-formamid-diethylacetal 16 h lang auf 100°C erhitzt. Nach dem Abkühlen engt man im Vakuum ein.
Ausbeute: 6.1 g (73% d. Th.)
LC-MS (Methode 6): Rₜ = 1.86 min; MS (ESIpos): m/z = 299 [M+H]⁺.

### Beispiel 10A

### 2-Hydrazino-4-methylpyridin

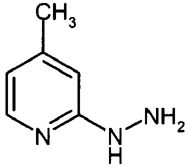

3.3 g (30.0 mmol) 2-Fluor-4-methylpyridin werden in 40 ml Ethylenglykolmonoethylether vorgelegt, die Lösung mit 14.6 ml (15.0 g, 300 mmol) Hydrazinhydrat versetzt und der Ansatz in der Siedehitze (150°C Badtemperatur) 16 h lang gerührt. Die Reaktionslösung wird danach am Rotationsverdampfer eingeengt, der Rückstand auf 100 ml Wasser gegeben und mit Essigsäureethylester extrahiert (dreimal je 100 ml). Die vereinigten organischen Phasen werden über Natriumsulfat getrocknet, filtriert und eingeengt. Der erhaltene Rückstand wird im Vakuum getrocknet.
Ausbeute: 1.90 g (51% d. Th.)
LC-MS (Methode 1): Rₜ = 0.80 min; MS (ESIpos): m/z = 124 [M+H]⁺;
¹H-NMR (400 MHz, DMSO-d₆): δ = 7.83 (d, 1H), 7.22 (s, 1H), 6.51 (s, 1H), 6.38 (d, 1H), 4.04 (s, 2H), 2.17 (s, 3H).

### Beispiel 11A

### 2-Hydrazino-5-methylpyridin

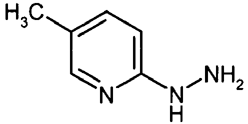

1.0 g (7.8 mmol) 2-Chlor-5-methylpyridin werden mit 5.7 ml (5.9 g, 117.6 mmol) Hydrazinhydrat versetzt und 16 h in der Siedehitze (150°C Badtemperatur) gerührt. Die abgekühlte Reaktionsmischung wird danach am Rotationsverdampfer eingeengt und der Rückstand dreimal mit jeweils 10 ml Ethylenglykolmonoethylether co-evaporiert. Der Rückstand wird dann in Dichlormethan aufgenommen, der Niederschlag abgetrennt und das Filtrat im Vakuum eingeengt.
Ausbeute: 644 mg (67% d. Th.)
LC-MS (Methode 6): Rₜ = 0.35 min; MS (ESIpos): m/z = 124 [M+H]⁺.

### Beispiel 12A

### 2-Chlor-5-(methoxymethyl)pyridin

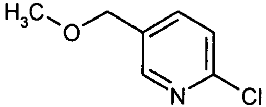

2.6 g (23.0 mmol) Kalium-*tert*.-butylat werden in 50 ml THF gelöst. Man gibt 3.0 g (20.9 mmol) (6-Chlorpyridin-3-yl)methanol hinzu und rührt 15 min bei RT. Man gibt dann langsam 4.4 g (31.3 mmol) Iodmethan hinzu und rührt ca. 30 min, bis die leicht exotherme Reaktion abgeklungen ist. Man entfernt das Lösungsmittel, nimmt den Rückstand in Dichlormethan auf und wäscht zweimal mit Wasser. Man trocknet die organische Phase über Magnesiumsulfat, engt ein und reinigt den Rückstand säulenchromatographisch an Kieselgel (Biotage-Chromatographie, Laufmittel: Cyclohexan/Essigsäureethylester 85:15).
Ausbeute: 2.2 g (68% d. Th.)
LC-MS (Methode 1): Rₜ = 2.62 min; MS (ESIpos): m/z = 158 [M+H]⁺;
¹H-NMR (400 MHz, CDCl₃): δ = 8.34 (d, 1H), 7.65 (dd, 1H), 7.32 (d, 1H), 4.45 (s, 2H), 3.41 (s, 3H).

### Beispiel 13A

### 5-(tert.-Butoxymethyl)-2-chlorpyridin

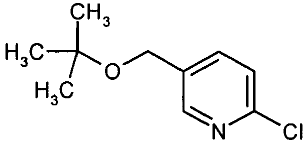

7.2 g (50.0 mmol) (6-Chlorpyridin-3-yl)methanol werden in 50 ml Dichlormethan vorgelegt. Man gibt 25.1 g (115.0 mmol) Di-*tert*.-butyldicarbonat und 1.1 g (5.0 mmol) Magnesiumperchlorat hinzu und rührt 24 h bei 40°C. Man kühlt dann auf RT ab, gibt weitere 12.5 g (57.3 mmol) Di*-tert.-*butyldicarbonat sowie 600 mg (2.7 mmol) Magnesiumperchlorat hinzu und rührt erneut für 2.5 h unter Rückfluss. Man gibt nochmals 12.5 g (57.3 mmol) Di-*tert*.-butyldicarbonat hinzu und rührt weitere 3 h unter Rückfluss. Danach verdünnt man mit Dichlormethan und wäscht einmal mit Wasser und einmal mit gesättigter Natriumchlorid-Lösung. Man trocknet über Magnesiumsulfat, engt ein und reinigt den Rückstand säulenchromatographisch an Kieselgel (Laufmittel: Cyclohexan/Essigsäureethylester 85:15).
Ausbeute: 7.9 g (79% d. Th.)
LC-MS (Methode 5): Rₜ = 1.12 min; MS (ESIpos): m/z = 200 [M+H]⁺;
¹H-NMR (400 MHz, DMSO-d₆): δ = 8.35 (d, 1H), 7.78 (dd, 1H), 7.48 (d, 1H), 4.45 (s, 2H), 1.22 (s, 9H).

### Beispiel 14A

### 6-Hydrazinopyridin-3-carbonitril

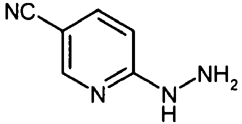

25.0 g (180.4 mmol) 6-Chlornicotinsäurenitril werden mit 90.3 g (1.8 mol) Hydrazinhydrat versetzt und 15 min bei 100°C Badtemperatur gerührt. Die auf RT abgekühlte Reaktionsmischung wird mit Wasser verdünnt und 30 min bei RT gerührt. Man filtriert den gebildeten Niederschlag ab, wäscht den Filterrückstand mit Wasser, trocknet die Kristalle 24 h lang an der Luft und kristallisiert einmal aus Essigsäureethylester um.
Ausbeute: 18.7 g (77% d. Th.)
LC-MS (Methode 1): Rₜ = 0.51 min; MS (ESIpos): m/z = 135 [M+H]⁺;
¹H-NMR (400 MHz, DMSO-d₆) = 8.58 (s, 1H), 8.33 (s, 1H), 7.74 (d, 1H), 6.76 (br. s, 1H), 4.42 (s, 2H).

### Beispiel 15A

### 6-Hydrazinopyridin-3-carbonsäure-tert.-butylester

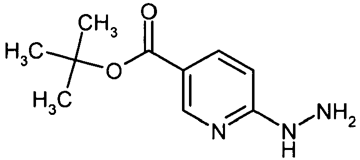

18.0 g (84.2 mmol) 6-Chlorpyridin-3-carbonsäure-*tert*.-butylester werden in 85 ml Ethanol vorgelegt. Man gibt 42.2 g (842.0 mmol) Hydrazinhydrat hinzu und rührt 2 h bei 100°C. Man engt danach ein und nimmt den Rückstand in einem Gemisch aus Essigsäureethylester und Wasser auf. Man trennt die Phasen, wäscht die organische Phase einmal mit Wasser und einmal mit gesättigter Natriumchlorid-Lösung, trocknet über Magnesiumsulfat und engt wieder ein. Der Rückstand wird in Petrolether verrührt, der gebildete Feststoff abfiltriert und im Hochvakuum getrocknet.
Ausbeute: 16.4 g (78% d. Th.)
LC-MS (Methode 1): Rₜ = 2.30 min; MS (ESIpos): m/z = 210 [M+H]⁺;
¹H-NMR (400 MHz, DMSO-d₆): δ = 8.49 (d, 1H), 8.30 (s, 1H), 7.82 (dd, 1H), 6.70 (d, 1H), 4.35 (s, 2H), 1.50 (s, 9H).

### Beispiel 16A

### 2-Hydrazino-5-(methoxymethyl)pyridin

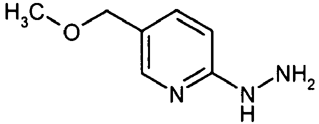

2.2 g (14.0 mmol) der Verbindung aus Beispiel 12A werden in 10 ml Ethanol vorgelegt. Man gibt 7.0 g (140.0 mmol) Hydrazinhydrat hinzu und rührt 16 h unter Rückfluss. Anschließend wird das Reaktionsgemisch nochmals 2 h in einer *single mode*-Mikrowelle (CEM Explorer) bei 150°C umgesetzt. Man engt danach am Rotationsverdampfer ein, nimmt in Essigsäureethylester auf, wäscht einmal mit gesättigter Natriumhydrogencarbonat-Lösung, trocknet über Magnesiumsulfat, engt wieder ein und trocknet den Rückstand im Hochvakuum.
Ausbeute: 970 mg (45% d. Th.)
LC-MS (Methode 7): Rₜ = 0.20 min; MS (ESIpos): m/z = 154 [M+H]⁺;
¹H-NMR (400 MHz, CDCl₃): δ = 8.09 (d, 1H), 7.50 (dd, 1H), 6.70 (d, 1H), 6.00 (br. s, 1H), 4.32 (s, 2H), 3.75 (br. s., 2H), 3.35 (s, 3H).

### Beispiel 17A

### 5-(tert.-Butoxymethyl)-2-hydrazinopyridin

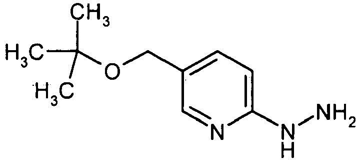

7.9 g (40.0 mmol) der Verbindung aus Beispiel 13A werden in 45 ml Ethanol gelöst. Man verteilt die Lösung auf drei Reaktionsgefäße und gibt jeweils 6.6 g (131.9 mmol) Hydrazinhydrat hinzu. Man setzt jedes Reaktionsgemisch für jeweils 4 h bei 170°C in einer *single mode*-Mikrowelle (CEM Explorer) um. Man vereinigt danach die drei Gemische und entfernt das Lösungsmittel. Der Rückstand wird in Essigsäureethylester aufgenommen und einmal mit gesättigter Natriumhydrogencarbonat-Lösung gewaschen. Die wässrige Phase wird einmal mit Essigsäureethylester rückextrahiert. Die beiden Essigsäureethylester-Phasen werden vereinigt und einmal mit gesättigter Natriumchlorid-Lösung gewaschen. Man trocknet über Magnesiumsulfat und entfernt das Lösungsmittel. Der Rückstand wird in Petrolether verrührt, der erhaltene Feststoff abfiltriert und im Hochvakuum getrocknet.
Ausbeute: 1.6 g (21% d. Th.)
LC-MS (Methode 5): Rₜ = 0.77 min; MS (ESIpos): m/z = 196 [M+H]⁺;
¹H-NMR (400 MHz, CDCl₃): δ = 8.09 (d, 1H), 7.50 (d, 1H), 6.68 (d, 1H), 5.75 (br. s, 1H), 4.32 (s, 2H), 3.85 (br. s, 2H), 1.28 (s, 9H).

### Beispiel 18A

### (4-Chlor-6-hydrazinopyridin-3-yl)methanol

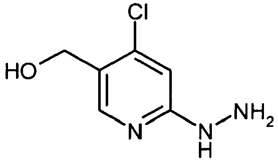

500 mg (2.8 mmol) (4,6-Dichlorpyridin-3-yl)methanol werden in 2 ml Ethanol vorgelegt. Man gibt 7.3 mg (14.0 mmol) Hydrazinhydrat hinzu und rührt 20 h bei 100°C. Man lässt danach auf RT abkühlen, filtriert den gebildeten Feststoff ab, verwirft ihn, engt die Mutterlauge am Rotationsverdampfer ein und erhält so die Titelverbindung im Gemisch mit ca. 10% [4,6-Bis(hydrazino)pyridin-3-yl]methanol.
Ausbeute: 450 mg (92% d. Th.)
LC-MS (Methode 6): Rₜ = 0.25 min; MS (ESIpos): m/z = 174 [M+H]⁺;
¹H-NMR (400 MHz, DMSO-d₆): δ = 7.75 (s, 1H), 7.41 (s, 1H), 6.90 (s, 1H), 5.20 (br. s, 1H), 4.35 (br. s, 2H).

### Beispiel 19A

### 5-(2,2-Dimethylpropoxy)-2-hydrazinopyridin

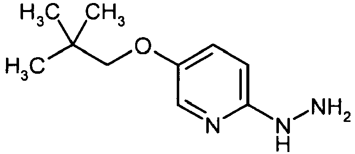

### Stufe a): 2-Chlor-5-(2,2-dimethylpropoxy)pyridin

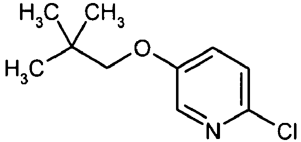

5.2 g (40.0 mmol) 6-Chlorpyridin-3-ol, 11.9 g (60.0 mmol) 1-Iod-2,2-dimethylpropan, 19.6 g (60.0 mmol) Cäsiumcarbonat und 120 ml Diethylenglykoldimethylether werden auf fünf jeweils gleich große Portionen aufgeteilt und portionsweise in einer *single mode*-Mikrowelle (CEM Explorer) bei 160°C für 4 h umgesetzt. Man vereinigt danach die erhaltenen fünf Reaktionsgemische, filtriert den Feststoff ab, wäscht den Feststoff mit Diethylenglykoldimethylether nach und vereinigt Filtrat und Waschlösungen. Der Großteil des Lösungsmittels wird entfernt und die aufkonzentrierte Lösung (ca. 50 ml) mit 300 ml Wasser versetzt. Man verrührt 30 min, filtriert den erhaltenen Feststoff ab, wäscht einmal mit Wasser und trocknet im Hochvakuum.
Ausbeute: 7.0 g (88% d. Th.)
LC-MS (Methode 6): Rₜ = 2.47 min; MS (ESIpos): m/z = 200 [M+H]⁺;
¹H-NMR (400 MHz, CDCl₃): δ = 8.05 (d, 1H), 7.25-7.15 (m, 2H), 3.61 (s, 2H), 1.03 (s, 9H).

### Stufe b): 5-(2,2-Dimethylpropoxy)-2-hydrazinopyridin

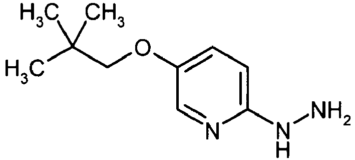

6.2 g (30.8 mmol) 2-Chlor-5-(2,2-dimethylpropoxy)pyridin werden zusammen mit 60 ml (1.2 mol) Hydrazinhydrat auf vier jeweils gleich große Portionen aufgeteilt und mit jeweils 10 ml Ethanol versetzt. Jede Portion wird in einer *single mode*-Mikrowelle (CEM Explorer) bei 170°C (200 Watt) für jeweils 12 h umgesetzt. Man vereinigt danach die vier Gemische und entfernt das Lösungsmittel. Der Rückstand wird in Essigsäureethylester aufgenommen und je einmal mit gesättigter Natriumhydrogencarbonat-Lösung und gesättigter Natriumchlorid-Lösung gewaschen. Man trocknet über Magnesiumsulfat und entfernt das Lösungsmittel im Vakuum.
Ausbeute: 6.0 g (76% d. Th.)
LC-MS (Methode 6): Rₜ = 1.28 min; MS (ESIpos): m/z = 196 [M+H]⁺;
¹H-NMR (400 MHz, CDCl₃): δ = 7.84 (s, 1H), 7.17 (dd, 1H), 6.68 (d, 1H), 5.54 (br. s, 1H), 3.80 (br. s, 2H), 3.56 (s, 2H), 1.02 (s, 9H).

### Beispiel 20A

### 2-Brom-4,5-dimethylpyridin

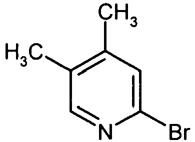

71.3 g (0.8 mol) 2-(Dimethylamino)-ethanol werden in 500 ml n-Hexan vorgelegt und auf 0°C abgekühlt. Man gibt langsam 1.0 Liter (1.6 mol) n-Butyllithium-Lösung (1.6 M in n-Hexan) hinzu und rührt 15 min bei 0°C. Man tropft dann eine Lösung von 17.9 g (166.7 mmol) 3,4-Lutidin in 500 ml n-Hexan hinzu und rührt 1 h bei 0°C. Anschließend wird auf -78°C abgekühlt und mit einer Lösung von 331.7 g (1.0 mol) Tetrabrommethan in 1.0 Liter THF versetzt. Man rührt 1 h bei -78°C nach und lässt das Reaktionsgemisch danach auf RT erwärmen. Man kühlt wieder auf 0°C ab und tropft langsam 1.5 Liter Wasser hinzu. Man trennt die Phasen, wäscht die organische Phase mit Wasser, trocknet über Magnesiumsulfat und engt im Vakuum ein. Der Rückstand wird zunächst an ca. 1 kg Kieselgel vorgereinigt (Laufmittel: Cyclohexan/Essigsäureethylester 9:1, dann 7:3). Die produkthaltigen Fraktionen werden vereinigt und im Vakuum eingeengt. Der Rückstand wird dann nochmals an Kieselgel gereinigt (Laufmittel: Cyclohexan/Essigsäureethylester 9:1). Das so erhaltene Produkt enthält ca. 10% des regioisomeren 2-Brom-3,4-dimethylpyridin.
Ausbeute: 6.7 g (20% d. Th.)
GC-MS (Methode 11): Rₜ = 4.24 min; MS (ESIpos): m/z = 187 [M+H]⁺;
¹H-NMR (400 MHz, CDCl₃): δ = 8.07 (s, 1H), 7.25 (s, 1H), 2.24 (s, 3H), 2.18 (s, 3H).

### Beispiel 21A

### 2-Hydrazino-4,5-dimethylpyridin

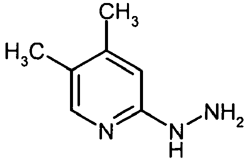

3.8 g (18.4 mmol, 90% Reinheit) der Verbindung aus Beispiel 20A werden in 12.5 ml Ethanol vorgelegt. Man gibt 8.9 ml (9.2 g, 183.8 mmol) Hydrazinhydrat hinzu und setzt in einer *single mode-*Mikrowelle (CEM Explorer) bei 170°C (100 Watt) für 2 h um. Die Reaktionslösung wird danach eingeengt, der Rückstand in Essigsäureethylester aufgenommen und jeweils einmal mit gesättigter Natriumhydrogencarbonat-Lösung und gesättigter Natriumchlorid-Lösung gewaschen. Die organische Phase wird über Natriumsulfat getrocknet, filtriert und im Vakuum eingeengt.
Ausbeute: 2.2 g (86% d. Th.)
LC-MS (Methode 6): Rₜ = 0.75 min; MS (ESIpos): m/z = 138 [M+H]⁺;
¹H-NMR (400 MHz, CDCl₃): δ = 7.86 (s, 1H), 6.51 (s, 1H), 5.61 (br. s, 1H), 3.72 (br. s, 2H), 2.20 (s, 3H), 2.12 (s, 3H).

### Ausführungsbeispiele:

### Beispiel 1

### 1-(3-Methylcyclohexyl)-3-{[2-(5-oxo-4-pyridin-3-yl-2,5-dihydro-1H-pyrazol-1-yl)pyridin-4-yl]-methyl}harnstoff

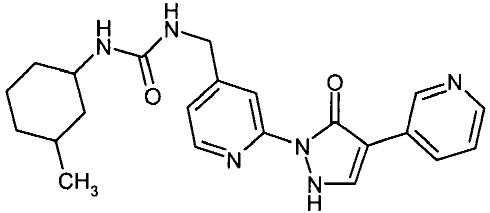

13.9 mg (100 µmol) 3-Methylcyclohexylisocyanat werden vorgelegt, mit 34.0 mg (100 µmol) der Verbindung aus Beispiel 4A, gelöst in 0.6 ml 1,2-Dichlorethan, sowie 25.8 mg (200 µmol) Diisopropylethylamin versetzt und über Nacht bei RT gerührt. Anschließend engt man ein, nimmt den Rückstand in DMSO auf, filtriert den Niederschlag ab und reinigt das Filtrat mittels präparativer HPLC (Methode 3).
Ausbeute: 5.7 mg (14% d. Th.)
LC-MS (Methode 3): Rₜ = 1.40 min; MS (ESIpos): m/z = 407 [M+H]⁺.

### Beispiel 2

### Methyl-N-({[2-(5-oxo-4-pyridin-3-yl-2,5-dihydro-1H-pyrazol-1-yl)pyridin-4-yl]methyl}carbamoyl)-L-valinat

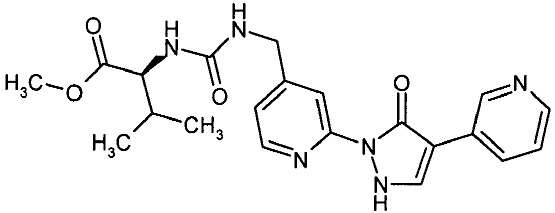

Die Titelverbindung wird in Analogie zu Beispiel 1 ausgehend von Beispiel 4A und dem entsprechenden Isocyanat erhalten.
Ausbeute: 7% d. Th.
LC-MS (Methode 3): Rₜ = 1.22 min; MS (ESIpos): m/z = 425 [M+H]⁺.

### Beispiel 3

### 2-(4-{[(Cyclohexylmethyl)amino]methyl}pyridin-2-yl)-4-pyridin-3-yl-1,2-dihydro-3H-pyrazol-3-on

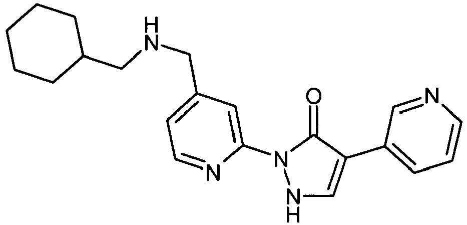

11.2 mg (100 µmol) Cyclohexancarbaldehyd werden vorgelegt, mit 34.0 mg (100 µmol) der Verbindung aus Beispiel 4A, gelöst in 0.6 ml Ethanol, sowie 25.8 mg (200 µmol) Diisopropylethylamin versetzt und über Nacht bei RT gerührt. Anschließend gibt man 5.7 mg (150 µmol) Natriumborhydrid hinzu und schüttelt 3 h bei RT. Danach setzt man 100 µl Wasser hinzu und engt ein. Man nimmt den Rückstand in DMSO auf, filtriert den Niederschlag ab und reinigt das Filtrat mittels präparativer HPLC (Methode 4).
Ausbeute: 2.6 mg (7% d. Th.)
LC-MS (Methode 4): Rₜ = 1.30 min; MS (ESIpos): m/z = 364 [M+H]⁺.

### Beispiel 4

### 2-{5-[(Methylamino)methyl]pyridin-2-yl}-4-pyridin-3-yl-1,2-dihydro-3H-pyrazol-3-on-Hydrochlorid

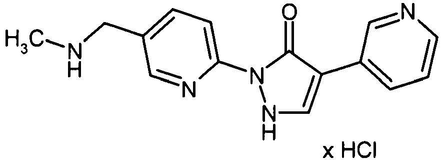

300 mg (1.4 mmol) der Verbindung aus Beispiel 2A und 207 mg (1.4 mmol) der Verbindung aus Beispiel 5A werden in 2 ml Ethanol gelöst und mit 47 mg (0.3 mmol) p-Toluolsulfonsäure versetzt. Es wird 24 h unter Rückfluss erhitzt, dann auf RT abgekühlt und direkt mittels präparativer HPLC aufgereinigt (RP18-Säule; Laufmittel: Acetonitril/Wasser-Gradient unter Zusatz von 0.1% Ameisensäure im Wasser). Die produkthaltigen Fraktionen werden im Vakuum eingeengt, der Rückstand in 1.5 ml Ethanol gelöst und mit 0.5 ml einer 4 N Lösung von Chlorwasserstoff in Dioxan versetzt. Es wird 30 min bei RT gerührt, dann im Vakuum eingeengt und der Rückstand im Hochvakuum getrocknet.
Ausbeute: 25 mg (5% d. Th.)
LC-MS (Methode 1): Rₜ = 1.68 min; MS (ESIpos): m/z = 282 [M+H]⁺;
¹H-NMR (400 MHz, DMSO-d₆): δ = 9.39-9.35 (m, 1H), 8.90 (d, 1H), 8.81 (s, 1H), 8.67 (s, 1H), 8.62 (d, 1H), 8.47 (d, 1H), 8.20 (d, 1H), 7.94 (dd, 1H), 4.23-4.19 (m, 2H), 2.59-2.56 (m, 3H).

### Beispiel 5

### 2-(5-{[(2-Methoxyethyl)amino]methyl}pyridin-2-yl)-4-pyridin-3-yl-1,2-dihydro-3H-pyrazol-3-on-Hydrochlorid

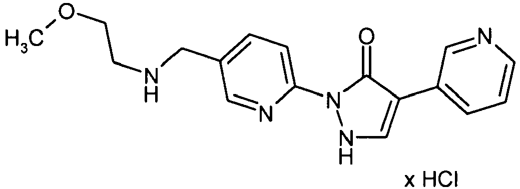

200 mg (0.9 mmol) der Verbindung aus Beispiel 2A und 178 mg (0.9 mmol) der Verbindung aus Beispiel 6A werden in 2 ml Ethanol gelöst und mit 42 mg (0.2 mmol) p-Toluolsulfonsäure versetzt. Es wird 24 h unter Rückfluss erhitzt, dann auf RT abgekühlt und direkt mittels präparativer HPLC aufgereinigt (RP18-Säule; Laufmittel: Acetonitril/Wasser-Gradient unter Zusatz von 0.1% Ameisensäure im Wasser). Die produkthaltigen Fraktionen werden im Vakuum eingeengt, der Rückstand in 1 ml Ethanol gelöst und mit 0.5 ml einer 4 N Lösung von Chlorwasserstoff in Dioxan versetzt. Es wird 30 min bei RT gerührt, dann im Vakuum eingeengt und der Rückstand im Hochvakuum getrocknet.
Ausbeute: 10 mg (3% d. Th.)
HPLC (Methode 10): Rₜ = 2.84 min; MS (ESIpos): m/z = 326 [M+H]⁺;
¹H-NMR (400 MHz, DMSO-d₆): δ = 9.47 (br. s, NH), 9.40 (s, 1H), 8.96 (d, 1H), 8.84 (s, 1H), 8.67 (s, 1H), 8.65 (d, 1H), 8.47 (d, 1H), 8.24 (d, 1H), 8.00 (dd, 1H), 4.26-4.22 (m, 2H), 3.66-3.62 (m, 2H), 3.31 (s, 3H), 3.15-3.11 (m, 2H).

### Beispiel 6

### 2-[5-(Methylsulfonyl)pyridin-2-yl]-4-pyridin-3-yl-1,2-dihydro-3H-pyrazol-3-on-Hydrochlorid

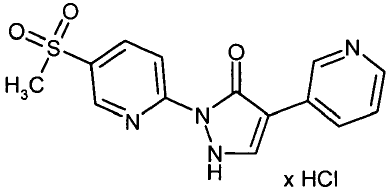

200 mg (0.9 mmol) der Verbindung aus Beispiel 2A und 170 mg (0.9 mmol) der Verbindung aus Beispiel 6A werden in 2 ml Ethanol gelöst und mit 31 mg (0.2 mmol) p-Toluolsulfonsäure versetzt. Es wird 24 h unter Rückfluss erhitzt, dann auf RT abgekühlt und direkt mittels präparativer HPLC aufgereinigt (RP18-Säule; Laufmittel: Acetonitril/Wasser-Gradient unter Zusatz von 0.1% Ameisensäure im Wasser). Die produkthaltigen Fraktionen werden im Vakuum eingeengt, der Rückstand in 1 ml Ethanol gelöst und mit 0.5 ml einer 4 N Lösung von Chlorwasserstoff in Dioxan versetzt. Es wird 30 min bei RT gerührt, dann im Vakuum eingeengt und der Rückstand im Hochvakuum getrocknet.
Ausbeute: 224 mg (66% d. Th.)
LC-MS (Methode 1): Rₜ = 2.12 min; MS (ESIpos): m/z = 317 [M+H]⁺;
¹H-NMR (400 MHz, DMSO-d₆): δ = 9.41 (s, 1H), 8.99 (s, 1H), 8.98-8.94 (m, 2H), 8.71 (d, 1H), 8.66 (d, 1H), 8.53 (dd, 1H), 8.01 (dd, 1H), 3.37 (s, 3H).

### Beispiel 7

### 4-(5-Brompyridin-3-yl)-2-(4-methylpyridin-2-yl)-1,2-dihydro-3H-pyrazol-3-on-Hydrochlorid

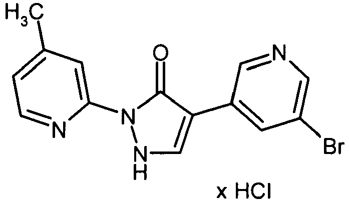

1070 mg (3.6 mmol) der Verbindung aus Beispiel 9A und 441 mg (3.6 mmol) der Verbindung aus Beispiel 10A werden in 10 ml Ethanol gelöst und mit 123 mg (0.7 mmol) p-Toluolsulfonsäure versetzt. Es wird 24 h unter Rückfluss erhitzt, dann auf RT abgekühlt und die überstehende Lösung abdekantiert. Der Rückstand wird in 10 ml Ethanol gelöst und mit 1 ml einer 4 N Lösung von Chlorwasserstoff in Dioxan versetzt. Es wird 30 min bei RT gerührt, dann im Vakuum eingeengt und der Rückstand im Hochvakuum getrocknet.
Ausbeute: 470 mg (36% d. Th.)
LC-MS (Methode 6): Rₜ = 1.99 min; MS (ESIpos): m/z = 332 [M+H]⁺;
¹H-NMR (400 MHz, DMSO-d₆): δ = 9.12 (s, 1H), 8.66-8.62 (m, 1H), 8.53 (s, 1H), 8.51 (d, 1H), 8.35 (d, 1H), 8.16 (d, 1H), 7.24 (d, 1H), 2.46 (s, 3H).

### Beispiel 8

### 5-[2-(4-Methylpyridin-2-yl)-3-oxo-2,3-dihydro-1H-pyrazol-4-yl]pyridin-3-carbonitril-Hydrochlorid

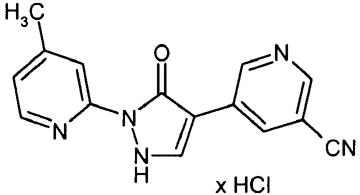

200 mg (0.5 mmol) der Verbindung aus Beispiel 7, 47 mg (0.4 mmol) Zinkcyanid und 19 mg (0.02 mmol) Tetrakis(triphenylphosphin)palladium(0) werden in 3 ml DMF 50 min lang bei 220°C in einer *single mode*-Mikrowelle (Emrys Optimizer) umgesetzt. Das Reaktionsgemisch wird danach im Vakuum eingeengt, der Rückstand in 2 ml Ethanol aufgenommen und das Gemisch mit 1 N Natronlauge auf pH 10 eingestellt. Es wird mit Essigsäureethylester extrahiert, die organische Phase über Natriumsulfat getrocknet, im Vakuum eingeengt und der Rückstand mittels präparativer HPLC aufgereinigt (RP 18-Säule; Laufmittel: Acetonitril/Wasser-Gradient unter Zusatz von 0.1 % Ameisensäure im Wasser). Die produkthaltigen Fraktionen werden im Vakuum eingeengt, der Rückstand in 1 ml Ethanol gelöst und mit 0.5 ml einer 4 N Lösung von Chlorwasserstoff in Dioxan versetzt. Es wird 30 min bei RT gerührt, dann im Vakuum eingeengt und der Rückstand im Hochvakuum getrocknet.
Ausbeute: 12 mg (7% d. Th.)
LC-MS (Methode 6): Rₜ = 1.79 min; MS (ESIpos): m/z = 278 [M+H]⁺;
¹H-NMR (400 MHz, DMSO-d₆): δ = 9.38 (s, 1H), 8.76 (s, 1H), 8.71 (s, 1H), 8.55 (s, 1H), 8.35 (d, 1H), 8.16 (d, 1H), 7.24 (d, 1H), 2.46 (s, 3H).

### Beispiel 9

### 6-[4-(5-Brompyridin-3-yl)-5-oxo-2,5-dihydro-1H-pyrazol-1-yl]pyridin-3-carbonsäure-tert.-butylester-Hydrochlorid

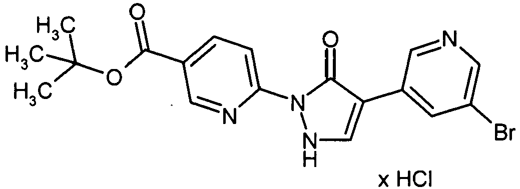

500 mg (1.7 mmol) der Verbindung aus Beispiel 9A und 350 mg (1.7 mmol) der Verbindung aus Beispiel 15A werden in 2 ml Ethanol gelöst und mit 58 mg (0.3 mmol) p-Toluolsulfonsäure versetzt. Es wird 24 h unter Rückfluss erhitzt, dann auf RT abgekühlt und mit 0.5 ml einer 4 N Lösung von Chlorwasserstoff in Dioxan versetzt. Es wird 30 min bei RT gerührt. Der Niederschlag wird abfiltriert, mit Ethanol und Diethylether nachgewaschen und im Hochvakuum getrocknet.
Ausbeute: 425 mg (56% d. Th.)
LC-MS (Methode 5): Rₜ = 1.47 min; MS (ESIpos): m/z = 417 [M+H]⁺;
¹H-NMR (400 MHz, DMSO-d₆): δ = 9.15 (s, 1H), 8.91 (s, 1H), 8.74 (d, 1H), 8.64 (d, 1H), 8.52-8.49 (m, 2H), 8.43 (d, 1H), 1.58 (s, 9H).

### Beispiel 10

### 6-[4-(5-Brompyridin-3-yl)-5-oxo-2,5-dihydro-1H-pyrazol-1-yl]pyridin-3-carbonsäure-Hydrochlorid

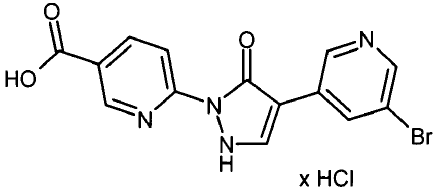

60 mg (0.1 mmol) der Verbindung aus Beispiel 9 werden in 2 ml Dichlormethan suspendiert und mit 0.5 ml TFA versetzt. Es wird 4 h bei RT gerührt, dann im Vakuum eingeengt und der Rückstand mit 0.5 ml einer 4 N Lösung von Chlorwasserstoff in Dioxan versetzt. Es wird 30 min bei RT gerührt. Der Niederschlag wird abfiltriert, mit Diethylether nachgewaschen und im Hochvakuum getrocknet.
Ausbeute: 37 mg (70% d. Th.)
LC-MS (Methode 6): Rₜ = 1.83 min; MS (ESIpos): m/z = 361 [M+H]⁺;
¹H-NMR (400 MHz, DMSO-d₆): δ = 9.16 (s, 1H), 8.97 (s, 1H), 8.75 (d, 1H), 8.66 (d, 1H), 8.52-8.47 (m, 2H), 8.46 (d, 1H).

### Beispiel 11

### 2-(5-Methylpyridin-2-yl)-4-pyridin-3-yl-1,2-dihydro-3H-pyrazol-3-on-Hydrochlorid

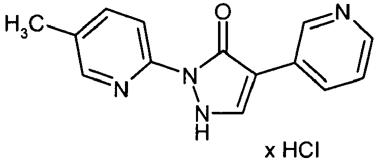

100 mg (0.5 mmol) der Verbindung aus Beispiel 9A und 62 mg (0.5 mmol) der Verbindung aus Beispiel 11A werden in 1.5 ml Ethanol gelöst und mit 16 mg (0.1 mmol) p-Toluolsulfonsäure versetzt. Es wird 24 h unter Rückfluss erhitzt, dann auf RT abgekühlt und im Vakuum eingeengt. Der Rückstand wird in 2 ml Ethanol aufgenommen und mit 1 ml einer 4 N Lösung von Chlorwasserstoff in Dioxan versetzt. Es wird 30 min bei RT gerührt. Der Niederschlag wird abfiltriert, mit Ethanol und Diethylether nachgewaschen und im Hochvakuum getrocknet.
Ausbeute: 66 mg (50% d. Th.)
LC-MS (Methode 6): Rₜ = 1.07 min; MS (ESIpos): m/z = 253 [M+H]⁺;
¹H-NMR (400 MHz, DMSO-d₆): δ = 9.31 (s, 1H), 8.82 (d, 1H), 8.64 (s, 1H), 8.57 (d, 1H), 8.34 (d, 1H), 8.27 (d, 1H), 7.93 (dd, 1H), 7.89 (dd, 1H), 2.36 (s, 3H).

### Beispiel 12

### 6-(5-Oxo-4-pyridin-3-yl-2,5-dihydro-1H-pyrazol-1-yl)pyridin-3-carbonitril-Hydrochlorid

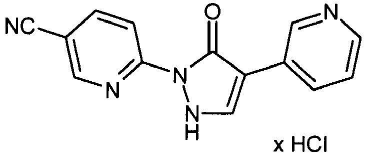

11.8 g (53.7 mmol) der Verbindung aus Beispiel 2A und 7.2 g (53.7 mmol) der Verbindung aus Beispiel 14A werden in 175 ml Ethanol vorgelegt. Man gibt 2.0 g (10.7 mmol) p-Toluolsulfonsäure-Monohydrat hinzu und rührt 16 h unter Rückfluss. Anschließend kühlt man auf 0°C ab, filtriert den gebildeten Feststoff ab und trocknet ihn im Hochvakuum. Man verrührt den Feststoff 30 min in einer 4 N Lösung von Chlorwasserstoff in Dioxan, filtriert den Niederschlag erneut ab und trocknet im Hochvakuum.
Ausbeute: 10.8 g (67% d. Th.)
LC-MS (Methode 1): Rₜ = 2.22 min; MS (ESIpos): m/z = 264 [M+H]⁺;
¹H-NMR (400 MHz, DMSO-d₆): δ = 9.40 (s, 1H), 9.02-8.96 (m, 3H), 8.68-8.61 (m, 2H), 8.49 (dd, 1H), 8.00 (dd, 1H).

### Beispiel 13

### 2-[5-(Aminomethyl)pyridin-2-yl]-4-pyridin-3-yl-1,2-dihydro-3H-pyrazol-3-on-Dihydrochlorid

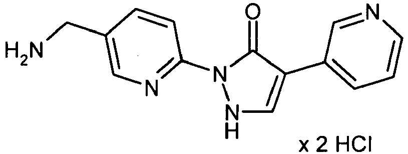

10.0 g (38.0 mmol) der Verbindung aus Beispiel 12 werden in 300 ml Essigsäure vorgelegt. Man gibt 20 ml einer 4 N Lösung von Chlorwasserstoff in Dioxan sowie 5.0 g (4.7 mmol) Palladium auf Kohle (10%) hinzu und hydriert über 96 h bei RT unter Normaldruck. Anschließend versetzt man mit Wasser, bis der gebildetete organische Feststoff wieder gelöst ist, und filtriert den Katalysator über Kieselgur ab. Man engt das Filtrat ein und reinigt den Rückstand säulenchromatographisch an Kieselgel (Laufmittel: Cyclohexan/Essigsäureethylester-Gemisch). Anschließend verrührt man das erhaltene Produkt 30 min in einer 4 N Lösung von Chlorwasserstoff in Dioxan, filtriert den Feststoff ab und trocknet ihn im Hochvakuum. Der Feststoff wird schließlich in ca. 300 ml Wasser gelöst und die Lösung lyophilisiert.
Ausbeute: 8.7 g (67% d. Th.)
LC-MS (Methode 6): Rₜ = 0.71 min; MS (ESIpos): m/z = 268 [M+H]⁺;
¹H-NMR (400 MHz, DMSO-d₆): δ = 9.42 (s, 1H), 9.04 (d, 1H), 8.87 (s, 1H), 8.68-8.53 (m, 4H), 8.45 (d, 1H), 8.20 (d, 1H), 8.05 (dd, 1H), 4.12 (d, 2H).

### Beispiel 14

### 2,2-Dimethyl-N-{[6-(5-oxo-4-pyridin-3-yl-2,5-dihydro-1H-pyrazol-1-yl)pyridin-3-yl]methyl}-propanamid-Hydrochlorid

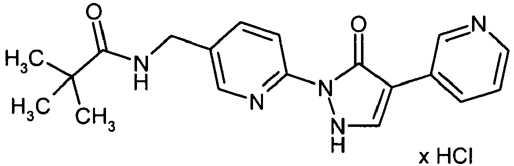

100 mg (0.3 mmol) der Verbindung aus Beispiel 13 werden in 3 ml Dichlormethan suspendiert. Man gibt 152 mg (1.2 mmol) *N*,*N-*Diisopropylethylamin (Hünig-Base) hinzu und rührt 5 min bei RT. Anschließend gibt man 43 mg (0.4 mmol) 2,2-Dimethylpropansäurechlorid hinzu und rührt 16 h bei RT. Man engt dann am Rotationsverdampfer ein, nimmt den Rückstand in 5 ml Dioxan auf und versetzt mit 1 ml 1 N Natronlauge. Dieses Gemisch wird anschließend mittels präparativer HPLC aufgereinigt (RP18-Säule; Laufmittel: Acetonitril/Wasser-Gradient unter Zusatz von 0.1% TFA im Wasser). Die produkthaltigen Fraktionen werden vereinigt und am Rotationsverdampfer eingeengt. Der Rückstand wird in 1 N Salzsäure gelöst und die Lösung lyophilisiert. Anschließend verrührt man 30 min in *tert*.-Butylmethylether, filtriert den Feststoff ab und trocknet ihn im Hochvakuum.
Ausbeute: 71 mg (62% d. Th.)
LC-MS (Methode 1): Rₜ = 2.37 min; MS (ESIpos): m/z = 352 [M+H]⁺;
¹H-NMR (400 MHz, DMSO-d₆): δ = 9.39 (s, 1H), 9.00 (d, 1H), 8.87 (s, 1H), 8.63 (d, 1H), 8.33 (m, 2H), 8.27 (t, 1H), 8.05 (dd, 1H), 7.93 (dd, 1H), 4.33 (d, 2H), 1.12 (s, 9H).

### Beispiel 15

### 6-(5-Oxo-4-pyridin-3-yl-2,5-dihydro-1H-pyrazol-1-yl)pyridin-3-carbonsäure-tert.-butylester

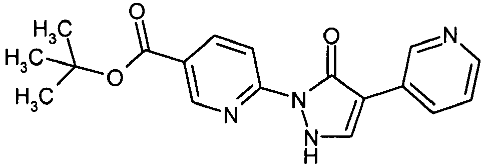

2.2 g (10.0 mmol) der Verbindung aus Beispiel 2A werden in 50 ml Ethanol vorgelegt. Man gibt 2.1 g (10.0 mmol) der Verbindung aus Beispiel 15A sowie 380 mg (2.0 mmol) p-Toluolsulfonsäure hinzu und rührt 16 h unter Rückfluss. Man kühlt dann auf 0°C ab, filtriert den gebildeten Feststoff ab und wäscht ihn einmal mit wenig Ethanol (Charge 1). Die Mutterlauge wird eingeengt und der Rückstand in wenig Ethanol verrührt. Man filtriert den erhaltenen Feststoff ab und wäscht einmal mit Ethanol (Charge 2). Die beiden Feststoff-Chargen werden vereinigt und im Hochvakuum getrocknet. Anschließend verrührt man 30 min in *tert*.-Butylmethylether, filtriert den Feststoff erneut ab und trocknet im Hochvakuum.
Ausbeute: 2.7 g (79% d. Th.)
LC-MS (Methode 5): Rₜ = 0.93 min; MS (ESIpos): m/z = 339 [M+H]⁺;
¹H-NMR (400 MHz, DMSO-d₆): δ = 9.11 (d, 1H), 8.91 (d, 1H), 8.57 (s, 1H), 8.51-8.49 (m, 1H), 8.40 (dd, 1H), 8.37-8.35 (m, 1H), 8.28 (d, 1H), 7.37 (dd, 1H), 1.58 (s, 9H).

### Beispiel 16

### 6-(5-Oxo-4-pyridin-3-yl-2,5-dihydro-1H-pyrazol-1-yl)pyridin-3-carbonsäure-tert.-butylester-Hydrochlorid

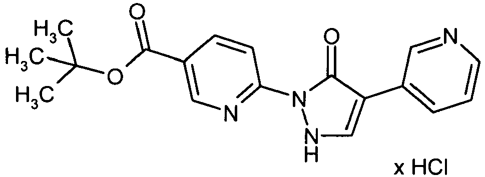

100 mg (0.3 mmol) der Verbindung aus Beispiel 15 werden 30 min lang in einer 4 N Lösung von Chlorwasserstoff in Dioxan bei RT verrührt. Man filtriert den Feststoff danach ab und trocknet ihn im Hochvakuum.
Ausbeute: 90 mg (81 % d. Th.)
LC-MS (Methode 1): Rₜ = 3.04 min; MS (ESIpos): m/z = 339 [M+H]⁺;
¹H-NMR (400 MHz, DMSO-d₆): δ = 9.39 (s, 1H), 8.99-8.86 (m, 3H), 8.65 (d, 1H), 8.55 (d, 1H), 8.45 (d, 1H), 8.00 (dd, 1H), 1.57 (s, 9H).

### Beispiel 17

### 6-(5-Oxo-4-pyridin-3-yl-2,5-dihydro-1H-pyrazol-1-yl)pyridin-3-carbonsäureamid-Hydrochlorid

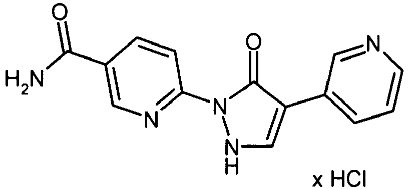

### Stufe a):

### 6-(5-Oxo-4-pyridin-3-yl-2,5-dihydro-1H-pyrazol-1-yl)pyridin-3-carbonsäurechlorid

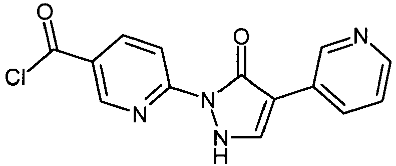

2.5 g (8.9 mmol) 6-(5-Oxo-4-pyridin-3-yl-2,5-dihydro-1*H-*pyrazol-1-yl)pyridin-3-carbonsäure [WO 2006/114213, Beispiel 39] werden in 50 ml Dichlormethan vorgelegt. Man gibt unter Rühren 0.1 ml DMF und dann langsam 2.2 g (17.7 mmol) Oxalsäuredichlorid bei RT hinzu. Nach Abklingen der Gasentwicklung wird 1 h unter Rückfluss gerührt. Anschließend engt man am Rotationsverdampfer ein, trocknet den Rückstand im Hochvakuum und setzt das so erhaltene Säurechlorid direkt in der Folgestufe weiter um.

### Stufe b):

### 6-(5-Oxo-4-pyridin-3-yl-2,5-dihydro-1H-pyrazol-1-yl)pyridin-3-carbonsäureamid-Hydrochlorid

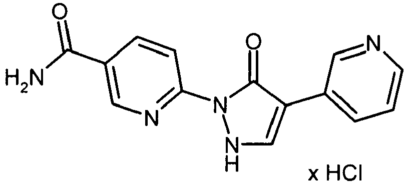

337 mg (1.0 mmol) 6-(5-Oxo-4-pyridin-3-yl-2,5-dihydro-1*H-*pyrazol-1-yl)pyridin-3-carbonsäure-chlorid werden in 5 ml Dioxan suspendiert. Man gibt langsam unter Rühren 4 ml einer 5 N Lösung von Ammoniak in Dioxan hinzu und rührt 16 h bei RT. Man engt danach ein, nimmt den Rückstand in DMSO auf und reinigt mittels präparativer HPLC (RP18-Säule; Laufmittel: Acetonitril/ Wasser-Gradient unter Zusatz von 0.1% TFA im Wasser). Die produkthaltigen Fraktionen werden vereinigt und am Rotationsverdampfer eingeengt. Der Rückstand wird in 1 N Salzsäure gelöst und die Lösung anschließend lyophilisiert.
Ausbeute: 47 mg (15% d. Th.)
LC-MS (Methode 1): Rₜ = 2.01 min; MS (ESIpos): m/z = 282 [M+H]⁺;
¹H-NMR (400 MHz, DMSO-d₆): δ = 9.41 (s, 1H), 9.00-8.95 (m, 2H), 8.48 (s, 1H), 8.65 (d, 1H), 8.56-8.42 (m, 2H), 8.23 (s, 1H), 8.03 (t, 1H), 7.63 (s, 1H).

### Beispiel 18

### N-tert.-Butyl-6-(5-oxo-4-pyridin-3-yl-2,5-dihydro-1H-pyrazol-1-yl)pyridin-3-carboxamid-Hydrochlorid

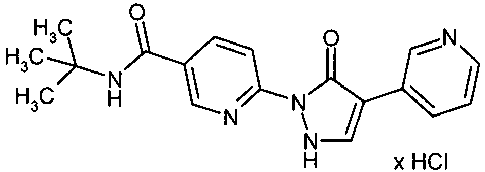

Man legt 337 mg (1.0 mmol) 6-(5-Oxo-4-pyridin-3-yl-2,5-dihydro-1*H*-pyrazol-1-yl)pyridin-3-carbonsäurechlorid aus Beispiel 17 (Stufe a) vor, gibt eine Lösung von 293 mg (4.0 mmol) *tert*.-Butylamin in 15 ml THF hinzu und rührt 1 h bei RT. Man engt danach am Rotationsverdampfer ein und reinigt den Rückstand mittels präparativer HPLC (RP18-Säule; Laufmittel: Acetonitril/ Wasser-Gradient unter Zusatz von 0.1% TFA im Wasser). Die produkthaltigen Fraktionen werden vereinigt und eingeengt. Der Rückstand wird in 1 N Salzsäure gelöst und die Lösung anschließend lyophilisiert.
Ausbeute: 47 mg (12% d. Th.)
LC-MS (Methode 1): Rₜ = 2.50 min; MS (ESIpos): m/z = 338 [M+H]⁺;
¹H-NMR (400 MHz, DMSO-d₆): δ = 9.41 (s, 1H), 8.99 (d, 1H), 8.88 (s, 2H), 8.64 (d, 1H), 8.51-8.39 (m, 2H), 8.09 (s, 1H), 8.03 (t, 1H), 1.40 (s, 9H).

### Beispiel 19

### 2-[5-(Methoxymethyl)pyridin-2-yl]-4-pyridin-3-yl-1,2-dihydro-3H-pyrazol-3-on-Hydrochlorid

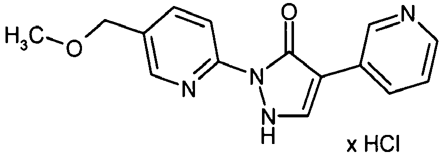

661 mg (3.0 mmol) der Verbindung aus Beispiel 2A und 460 mg (3.0 mmol) der Verbindung aus Beispiel 16A werden in 10 ml Ethanol vorgelegt. Man gibt 114 mg (0.6 mmol) p-Toluolsulfonsäure hinzu und rührt 16 h unter Rückfluss. Man lässt dann auf RT abkühlen, filtriert den gebildeten Feststoff ab und wäscht ihn einmal mit Ethanol (Charge 1). Die Mutterlauge wird eingeengt und der Rückstand mittels präparativer HPLC (RP18-Säule; Laufmittel: Acetonitril/Wasser-Gradient unter Zusatz von 0.1% TFA im Wasser) gereinigt (Charge 2). Beide Chargen werden vereinigt, in 1 N Salzsäure gelöst und die Lösung anschließend lyophilisiert.
Ausbeute: 625 mg (65% d. Th.)
LC-MS (Methode 7): Rₜ = 1.08 min; MS (ESIpos): m/z = 283 [M+H]⁺;
¹H-NMR (400 MHz, DMSO-d₆): δ = 9.41 (s, 1H), 9.01 (d, 1H), 8.80 (s, 1H), 8.65 (d, 1H), 8.44 (d, 1H), 8.40 (d, 1H), 8.06-8.04 (m, 2H), 4.50 (s, 2H), 3.34 (s, 3H).

### Beispiel 20

### 2-[5-(tert.-Butoxymethyl)pyridin-2-yl]-4-pyridin-3-yl-1,2-dihydro-3H-pyrazol-3-on

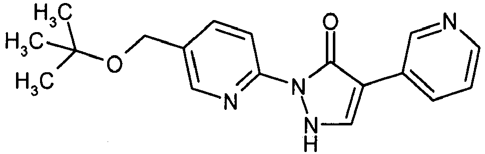

661 mg (3.0 mmol) der Verbindung aus Beispiel 2A und 586 mg (3.0 mmol) der Verbindung aus Beispiel 17A werden in 10 ml Ethanol vorgelegt. Man gibt 114 mg (0.6 mmol) p-Toluolsulfonsäure hinzu und rührt 16 h unter Rückfluss. Man lässt danach auf RT abkühlen, filtriert den gebildeten Feststoff ab, wäscht ihn einmal mit Ethanol und trocknet im Hochvakuum. Der Feststoff wird dann 30 min in *tert*.-Butylmethylether verrührt, erneut abfiltriert und im Hochvakuum getrocknet (Charge 1). Die zuvor erhaltenen Filtrate und Waschlösungen werden vereinigt, eingeengt und der Rückstand in gesättigter Natriumhydrogencarbonat-Lösung verrührt. Man filtriert den erhaltenen Feststoff ab, wäscht einmal mit Wasser und reinigt den Filterrückstand dann mittels präparativer HPLC (RP18-Säule; Laufmittel: Acetonitril/Wasser-Gradient unter Zusatz von 0.1% TFA im Wasser). Die Produktfraktionen werden vereinigt und eingeengt (Charge 2).
Ausbeute: zusammen 487 mg (50% d. Th.)
LC-MS (Methode 5): Rₜ = 0.82 min; MS (ESIpos): m/z = 325 [M+H]⁺;
¹H-NMR (400 MHz, DMSO-d₆): δ = 9.08 (s, 1H), 8.41 (s, 2H), 8.37 (d, 1H), 8.24 (d, 2H), 7.98 (d, 1H), 8.38 (dd, 1H), 4.49 (s, 2H), 1.25 (s, 9H).

### Beispiel 21

### 2-[5-(tert.-Butoxymethyl)pyridin-2-yl]-4-pyridin-3-yl-1,2-dihydro-3H-pyrazol-3-on-Hydrochlorid

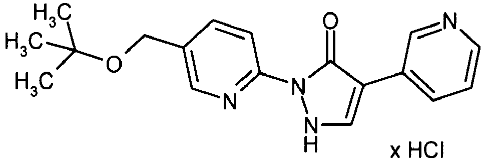

400 mg (1.2 mmol) der Verbindung aus Beispiel 20 werden 30 min lang in 20 ml einer 4 N Lösung von Chlorwasserstoff in Dioxan bei RT verrührt. Man filtriert den Feststoff ab und trocknet ihn im Hochvakuum. Anschließend wird in Wasser gelöst und die Lösung lyophilisiert. Das Lyophilisat wird erneut 30 min mit 10 ml einer 4 N Lösung von Chlorwasserstoff in Dioxan verrührt. Man filtriert den Feststoff ab, wäscht einmal mit *tert*.-Butylmethylether und trocknet im Hochvakuum.
Ausbeute: 306 mg (69% d. Th.)
LC-MS (Methode 5): Rₜ = 0.81 min; MS (ESIpos): m/z = 325 [M+H]⁺;
¹H-NMR (400 MHz, DMSO-d₆): δ = 9.38 (s, 1H), 8.94 (d, 1H), 8.73 (s, 1H), 8.61 (d, 1H), 8.43 (s, 1H), 4.35 (d, 1H), 8.01-7.99 (m, 2H), 4.49 (s, 2H), 1.25 (s, 9H).

### Beispiel 22

### 4-Pyridin-3-yl-2-[5-(1H-pyrrol-1-ylmethyl)pyridin-2-yl]-1,2-dihydro-3H-pyrazol-3-on-Hydrochlorid

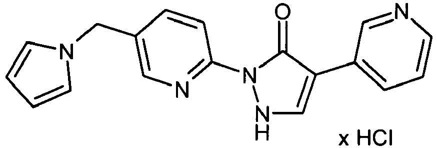

565 mg (9.4 mmol) Essigsäure werden in 0.3 ml Wasser vorgelegt. Man gibt langsam 744 mg (9.4 mmol) Pyridin hinzu. Anschließend gibt man 191 mg (0.6 mmol) der Verbindung aus Beispiel 13 sowie 74 mg (0.6 mmol) 2,5-Dimethoxytetrahydrofuran hinzu und rührt 16 h bei 100°C. Man engt dann am Rotationsverdampfer ein, versetzt mit 3 ml Acetonitril sowie 3 ml 1 N Salzsäure und rührt 30 min bei RT nach. Man filtriert den gebildeten Feststoff ab, wäscht einmal mit Wasser und trocknet im Hochvakuum.
Ausbeute: 150 mg (76% d. Th.)
LC-MS (Methode 5): Rₜ = 0.74 min; MS (ESIpos): m/z = 318 [M+H]⁺;
¹H-NMR (400 MHz, DMSO-d₆): δ = 9.35 (s, 1H), 8.88 (d, 1H), 8.73 (s, 1H), 8.60 (d, 1H), 8.48 (s, 1H), 8.35 (d, 1H), 7.97-7.86 (m, 2H), 6.90 (s, 2H), 6.05 (s, 2H), 5.70 (s, 2H).

### Beispiel 23

### 2-[4-Chlor-5-(hydroxymethyl)pyridin-2-yl]-4-pyridin-3-yl-1,2-dihydro-3H-pyrazol-3-on-Trifluoracetat

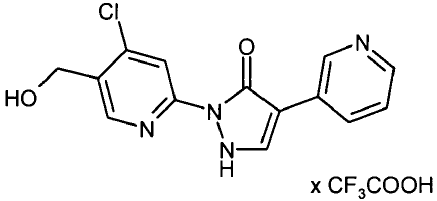

345 mg (1.6 mmol) der Verbindung aus 2A, 272 mg (1.6 mmol) der Verbindung aus Beispiel 18A und 60 mg (0.3 mmol) p-Toluolsulfonsäure werden in einem Gemisch aus 10 ml THF und 5 ml Ethanol vorgelegt. Man setzt 3 h bei 150°C in einer *single mode*-Mikrowelle (CEM Explorer) um. Anschließend engt man am Rotationsverdampfer ein und nimmt den Rückstand in einem Gemisch aus 5 ml Methanol und 4 ml 1 N Salzsäure auf. Man reinigt zweimal nacheinander mittels präparativer HPLC (RP18-Säule; Laufmittel: Acetonitril/Wasser-Gradient unter Zusatz von 0.1% TFA im Wasser).
Ausbeute: 16 mg (2% d. Th.)
LC-MS (Methode 6): Rₜ = 1.04 min; MS (ESIpos): m/z = 303 [M+H]⁺;
¹H-NMR (400 MHz, DMSO-d₆): δ = 9.38 (s, 1H), 8.64 (d, 1H), 8.48 (s, 1H), 8.33 (d, 1H), 8.20 (s, 1H), 7.96 (s, 1H), 7.77 (dd, 1H), 4.57 (s, 2H).

### Beispiel 24

### 2-[4-Chlor-5-(hydroxymethyl)pyridin-2-yl]-4-pyridin-3-yl-1,2-dihydro-3H-pyrazol-3-on-Hydrochlorid

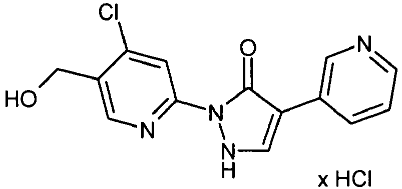

12 mg (0.03 mmol) der Verbindung aus Beispiel 23 werden 30 min lang in 1.5 ml einer 4 N Lösung von Chlorwasserstoff in Dioxan verrührt. Man dekantiert danach ab, verrührt den Rückstand in *tert*.-Butylmethylether, dekantiert erneut ab und trocknet den verbleibenden Feststoff zunächst an der Luft und dann im Hochvakuum.
Ausbeute: 10 mg (95% d. Th.)
LC-MS (Methode 6): Rₜ = 1.06 min; MS (ESIpos): m/z = 303 [M+H]⁺;
¹H-NMR (400 MHz, DMSO-d₆): δ = 9.31 (s, 1H), 8.90 (d, 1H), 8.66 (s, 1H), 8.63 (d, 2H), 8.03 (dd, 1H), 7.69 (s, 1H), 4.61 (s, 2H).

### Beispiel 25

### 1-(2,4-Difluorphenyl)-3-{[2-(5-oxo-4-pyridin-3-yl-2,5-dihydro-1H-pyrazol-1-yl)pyridin-4-yl]-methyl}harnstoff

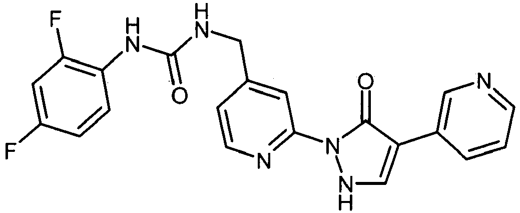

2.7 g (10 mmol) der Verbindung aus Beispiel 4A werden in 60 ml 1,2-Dichlorethan gelöst und als Stammlösung bereitgestellt.

16 mg (0.1 mmol) 2,4-Difluor-1-isocyanatobenzol werden vorgelegt und mit 600 µl (0.1 mmol) der obigen Stammlösung sowie 26 mg (0.2 mmol) *N*,*N*-Diisopropylethylamin (Hünig-Base) versetzt. Man rührt die Reaktionsmischung 16 h bei RT. Zur Aufarbeitung wird das Lösungsmittel im Vakuum entfernt, der Rückstand in DMSO aufgenommen, filtriert und das Filtrat direkt mittels präparativer LC-MS (Methode 8) aufgereinigt. Die Produktfraktionen werden im Vakuum aufkonzentriert und der Rückstand getrocknet.
Ausbeute: 3 mg (10% d. Th.)
LC-MS (Methode 8): Rₜ = 1.33 min; MS (ESIpos): m/z = 423 [M+H]⁺.

Analog zur Arbeitsvorschrift von Beispiel 25 werden die in Tabelle 1 aufgeführten Verbindungen aus 0.1 mmol der Verbindung aus Beispiel 4A und 0.1 mmol des entsprechenden Isocyanats hergestellt:

**Tabelle 1**

| **Beispiel Nr.** | **Struktur** | **Ausbeute (% d. Th.)** | **MS (ESI): [M+H]⁺; LC-MS: Rₜ (Methode 8)** |
|---|---|---|---|
| **26** | 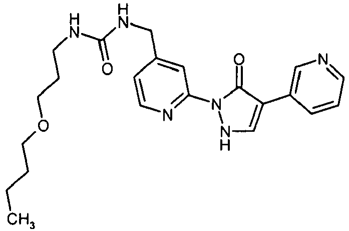 | 9% | m/z = 425; 1.32 min |
| **27** | 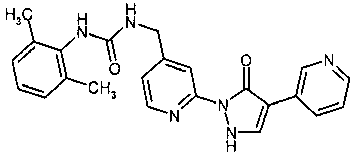 | 7% | m/z = 415; 1.31 min |
| **28** | 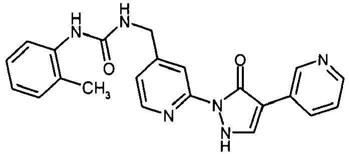 | 11% | m/z = 401; 1.28 min |
| **29** | 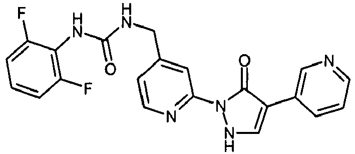 | 10% | m/z = 423; 1.20 min |
| **30** | 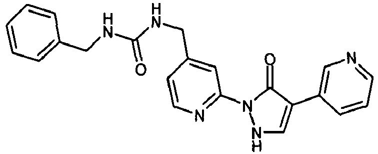 | 8% | m/z = 401; 1.25 min |
| **31** | 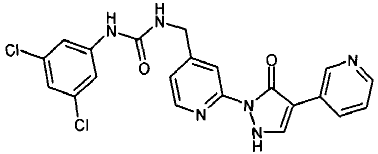 | 13% | m/z = 456; 1.57 min |
| **32** | 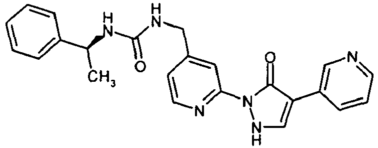 | 18% | m/z = 415; 1.30 min |
| **33** | 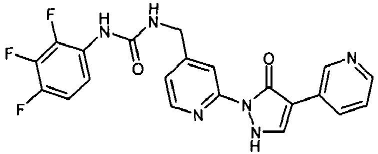 | 17% | m/z = 441; 1.38 min |
| **34** | 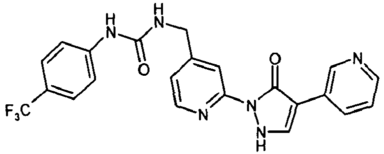 | 15% | m/z = 455; 1.52 min |
| **35** | 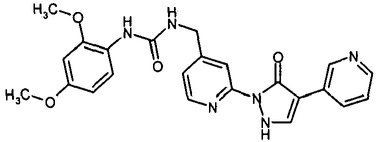 | 10% | m/z = 447; 1.31 min |
| **36** | 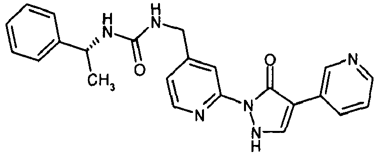 | 16% | m/z = 415; 1.30 min |
| **37** | 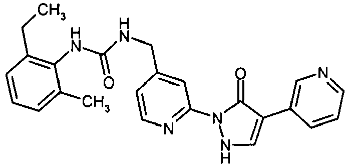 | 7% | m/z = 429; 1.37 min |
| **38** | 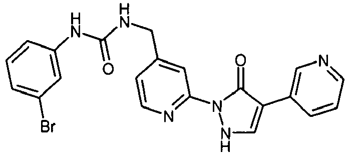 | 18% | m/z = 465; 1.44 min |
| **39** | 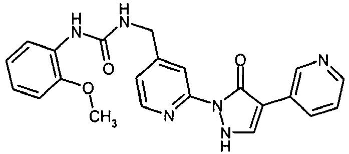 | 11% | m/z = 417; 1.33 min |
| **40** | 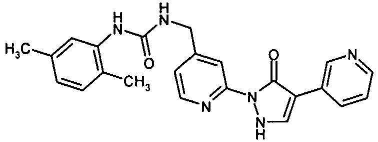 | 8% | m/z = 415; 1.36 min |
| **41** | 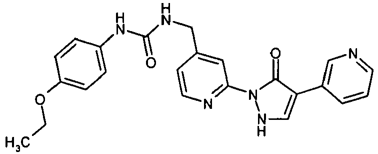 | 14% | m/z = 431; 1.34 min |
| **42** | 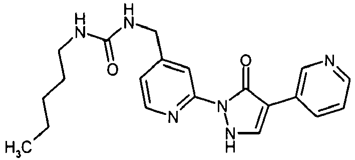 | 15% | m/z = 381; 1.31 min |
| **43** | 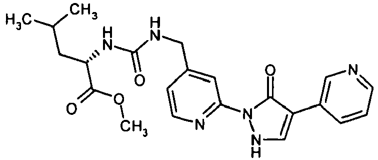 | 11% | m/z = 439; 1.33 min |
| **44** | 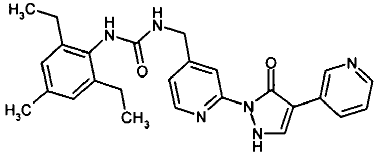 | 14% | m/z = 457; 1.51 min |
| **45** | 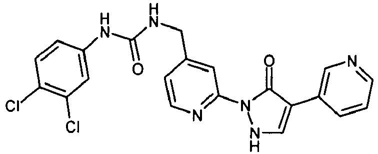 | 14% | m/z = 455; 1.52 min |
| **46** | 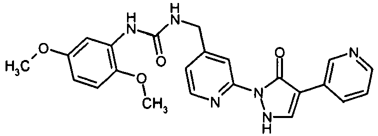 | 10% | m/z = 447; 1.33 min |
| **47** | 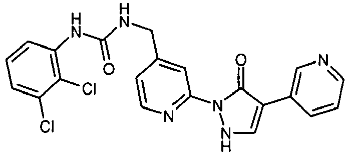 | 20% | m/z = 455; 1.47 min |
| **48** | 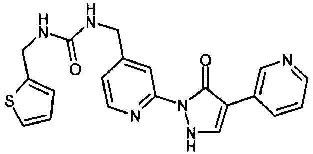 | 4% | m/z = 407; 1.22 min |
| **49** | 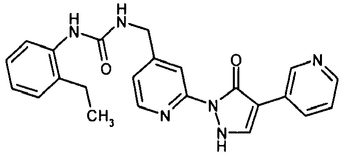 | 15% | m/z = 415; 1.35 min |
| **50** | 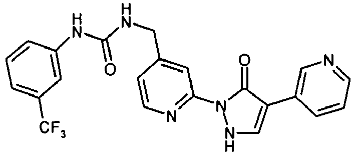 | 15% | m/z = 455; 1.48 min |
| **51** | 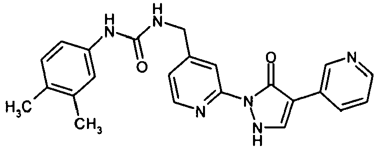 | 17% | m/z = 415; 1.42 min |
| **52** | 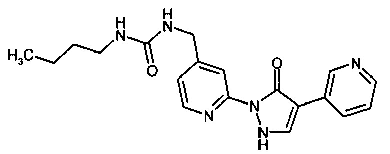 | 3% | m/z = 367; 1.21 min |
| **53** | 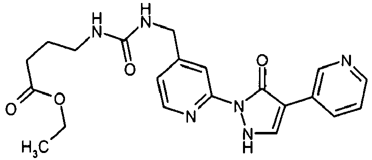 | 13% | m/z = 425; 1.19 min |
| **54** | 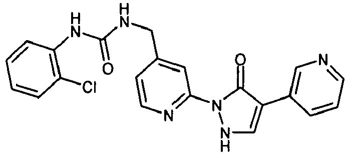 | 16% | m/z = 421; 1.36 min |
| **55** | 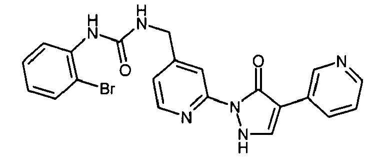 | 12% | m/z = 465; 1.37 min |
| **56** | 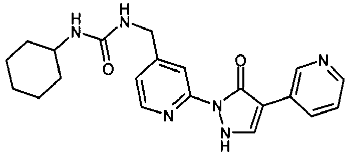 | 8% | m/z = 393; 1.29 min |
| **57** | 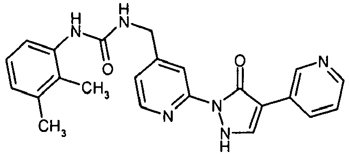 | 10% | m/z = 415; 1.35 min |
| **58** | 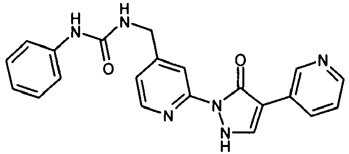 | 15% | m/z = 387; 1.28 min |
| **59** | 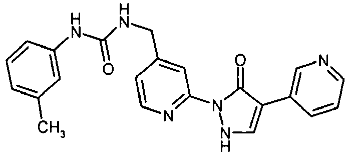 | 19% | m/z = 401; 1.35 min |
| **60** | 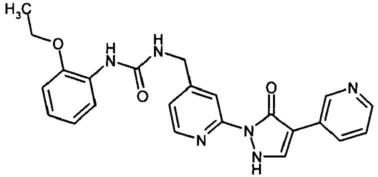 | 17% | m/z = 431; 1.40 min |
| **61** | 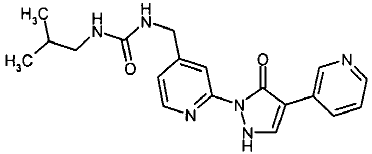 | 14% | m/z = 367; 1.20 min |
| **62** | 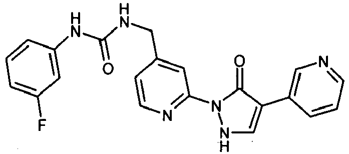 | 10% | m/z = 405; 1.33 min |
| **63** | 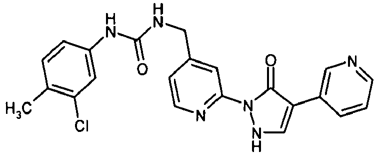 | 26% | m/z = 435; 1.49 min |
| **64** | 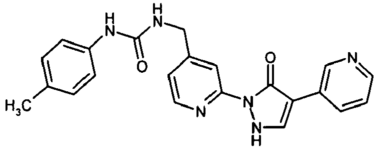 | 22% | m/z = 401; 1.35 min |
| **65** | 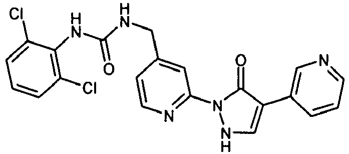 | 8% | m/z = 455; 1.28 min |
| **66** | 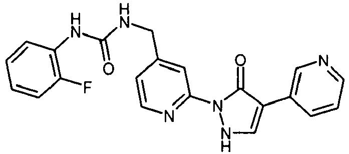 | 12% | m/z = 405; 1.31 min |
| **67** | 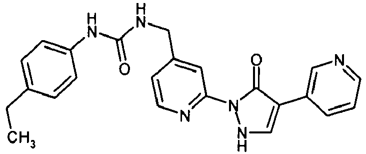 | 6% | m/z = 415; 1.44 min |
| **68** | 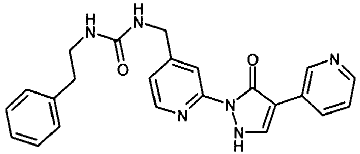 | 13% | m/z = 415; 1.30 min |
| **69** | 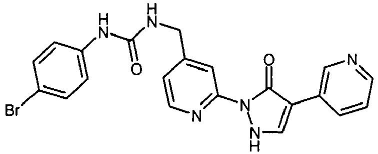 | 7% | m/z = 465; 1.44 min |

### Beispiel 70

### [6-(5-Oxo-4-pyridin-3-yl-2,5-dihydro-1H-pyrazol-1-yl)pyridin-3-yl]methyl(2,3-dimethylphenyl)-carbamat

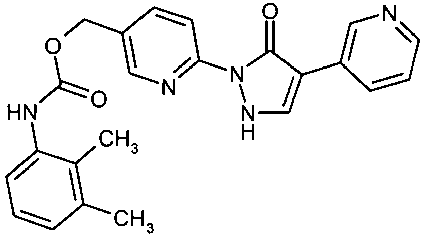

2.7 g (10 mmol) 2-[5-(Hydroxymethyl)pyridin-2-yl]-4-pyridin-3-yl-1,2-dihydro-3*H-*pyrazol-3-on [Herstellung siehe WO 2006/114213] werden in 60 ml 1,2-Dichlorethan gelöst und als Stammlösung bereitgestellt.

15 mg (0.1 mmol) 1-Isocyanato-2,3-dimethylbenzol werden vorgelegt und mit 600 µl(0.1 mmol) der obigen Stammlösung sowie 26 mg (0.2 mmol) *N,N-*Diisopropylethylamin (Hünig-Base) versetzt. Man rührt die Reaktionsmischung 16 h bei RT. Zur Aufarbeitung wird das Lösungsmittel im Vakuum entfernt, der Rückstand in DMSO aufgenommen, filtriert und das Filtrat direkt mittels präparativer LC-MS (Methode 9) aufgereinigt. Die Produktfraktionen werden im Vakuum aufkonzentriert und der Rückstand getrocknet.
Ausbeute: 0.6 mg (2% d. Th.)
LC-MS (Methode 9): Rₜ = 1.58 min; MS (ESIpos): m/z = 416 [M+H]⁺.

Analog zur Arbeitsvorschrift von Beispiel 70 werden die in Tabelle 2 aufgeführten Verbindungen aus 0.1 mmol 2-[5-(Hydroxymethyl)pyridin-2-yl]-4-pyridin-3-yl-1,2-dihydro-3*H*-pyrazol-3-on und 0.1 mmol des entsprechenden Isocyanats hergestellt:

**Tabelle 2**

| **Beispiel Nr.** | **Struktur** | **Ausbeute (% d. Th.)** | **MS (ESI): [M+H]⁺; LC-MS: R, (Methode 9)** |
|---|---|---|---|
| **71** | 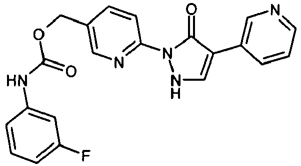 | 4% | m/z = 406; 1.55 min |
| **72** | 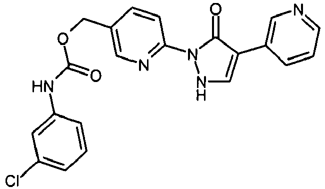 | 2% | m/z = 422; 1.63 min |
| **73** | 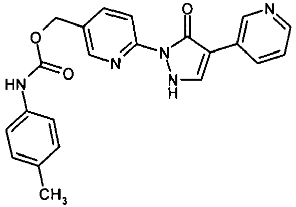 | 1% | m/z = 402; 1.57 min |
| **74** | 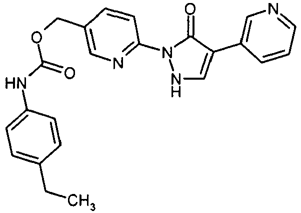 | 3% | m/z = 416; 1.67 min |
| **75** | 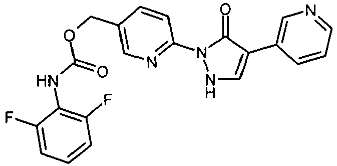 | 2% | m/z = 424; 1.44 min |
| **76** | 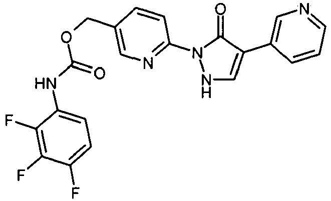 | 2% | m/z = 442; 1.58 min |
| **77** | 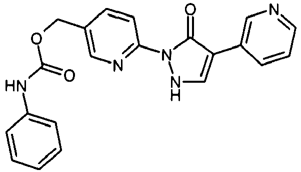 | 2% | m/z = 388; 1.48 min |
| **78** | 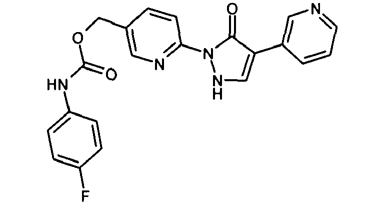 | 4% | m/z = 406; 1.52 min |
| **79** | 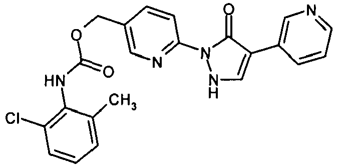 | 2% | m/z = 436; 1.53 min |
| **80** | 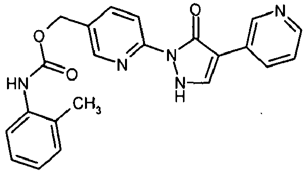 | 1% | m/z = 402; 1.51 min |
| **81** | 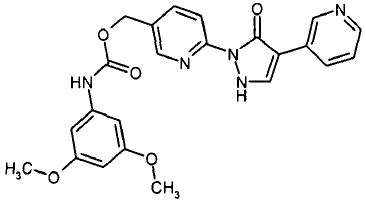 | 2% | m/z = 448; 1.55 min |

### Beispiel 82

### 4-Pyridin-3-yl-2-(4-{[(pyridin-2-ylmethyl)amino]methyl}pyridin-2-yl)-1,2-dihydro-3H-pyrazol-3-on

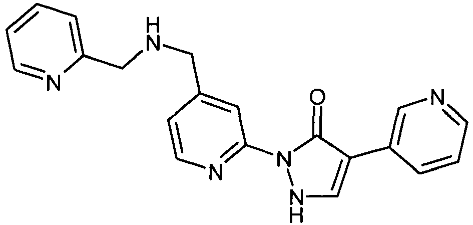

2.7 g (10 mmol) der Verbindung aus Beispiel 4A werden in 60 ml Ethanol gelöst und als Stammlösung bereitgestellt.

11 mg (0.1 mmol) Pyridin-2-carbaldehyd werden vorgelegt und mit 600 µl (0.1 mmol) der obigen Stammlösung sowie 26 mg (0.2 mmol) *N*,*N-*Diisopropylethylamin (Hünig-Base) versetzt. Man rührt die Reaktionsmischung 16 h bei RT, gibt dann 4 mg (0.1 mmol) Natriumborhydrid hinzu und schüttelt weitere 3 h bei RT. Zur Aufarbeitung gibt man 100 µl Wasser hinzu, entfernt das Lösungsmittel im Vakuum und löst den Rückstand in DMSO. Nach Filtration wird das Filtrat mittels präparativer LC-MS (Methode 9) aufgereinigt. Die Produktfraktionen werden im Vakuum aufkonzentriert und der Rückstand getrocknet.
Ausbeute: 0.7 mg (2% d. Th.)
LC-MS (Methode 9): Rₜ = 1.56 min; MS (ESIpos): m/z = 358 [M+H]⁺.

Analog zur Arbeitsvorschrift von Beispiel 82 werden die in Tabelle 3 aufgeführten Verbindungen aus 0.1 mmol der Verbindung aus Beispiel 4A und 0.1 mmol des entsprechenden Aldehyds hergestellt:

**Tabelle 3**

| **Beispiel Nr.** | **Struktur** | **Ausbeute (% d. Th.)** | **MS (ESI): [M+H]⁺; LC-MS: R, (Methode 9)** |
|---|---|---|---|
| **83** | 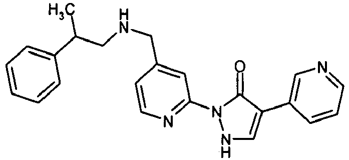 | 2% | m/z = 386; 1.31 min |
| **84** | 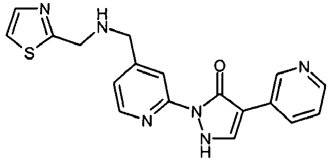 | 2% | m/z = 364; 1.59 min |
| **85** | 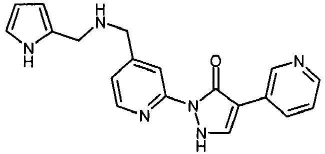 | 4% | m/z = 347; 1.36 min |

### Beispiel 86

### 2-(4,5-Dimethylpyridin-2-yl)-4-pyridin-3-yl-1,2-dihydro-3H-pyrazol-3-on

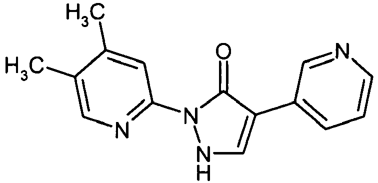

220 mg (1.0 mmol) der Verbindung aus Beispiel 2A werden in 5 ml Ethanol vorgelegt. Man gibt 137 mg (1.0 mmol) der Verbindung aus Beispiel 21A sowie 38 mg (0.2 mmol) p-Toluolsulfonsäure hinzu und rührt 16 h unter Rückfluss. Man lässt danach auf RT abkühlen, filtriert den gebildeten Feststoff ab, wäscht mit *tert*.-Butylmethylether und trocknet den Feststoff im Hochvakuum. Man erhält so 28 mg der Titelverbindung. Die Mutterlauge wird verwahrt und zur Darstellung des Hydrochlorids verwendet (siehe Beispiel 87).
Ausbeute: 28 mg (11% d. Th.)
LC-MS (Methode 6): Rₜ = 1.10 min; MS (ESIpos): m/z = 267 [M+H]⁺;
¹H-NMR (400 MHz, DMSO-d₆): δ = 9.05 (s, 1H), 8.37-8.28 (m, 2H), 8.24-8.15 (m, 2H), 8.09-7.95 (s, 1H), 7.36 (dd, 1H), 2.36 (s, 3H), 2.25 (s, 3H).

### Beispiel 87

### 2-(4,5-Dimethylpyridin-2-yl)-4-pyridin-3-yl-1,2-dihydro-3H-pyrazol-3-on-Hydrochlorid

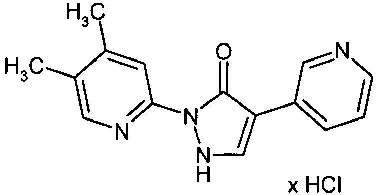

Die im Zuge der Darstellung von Beispiel 86 erhaltene Mutterlauge (siehe oben) wird am Rotationsverdampfer eingeengt und der Rückstand mittels präparativer HPLC gereinigt (RP18-Säule; Laufmittel: Acetonitril/Wasser-Gradient unter Zusatz von 0.1% TFA im Wasser). Der nach Einengen der Produktfraktionen erhaltene Rückstand wird 30 min in 5 ml einer 4 N Lösung von Chlorwasserstoff in Dioxan verrührt. Man filtriert den Feststoff ab, wäscht zweimal mit *tert*.-Butyl-methylether und trocknet im Hochvakuum.
Ausbeute: 100 mg (33% d. Th., bezogen auf 1.0 mmol der Verbindung aus Beispiel 21 A)
LC-MS (Methode 6): Rₜ = 1.12 min; MS (ESIpos): m/z = 267 [M+H]⁺;
¹H-NMR (400 MHz, DMSO-d₆): δ = 9.32 (s, 1H), 8.94 (d, 1H), 8.61 (s, 1H), 8.57 (d, 1H), 8.23 (s, 1H), 8.21 (s, 1H), 8.00 (dd, 1H), 2.41 (s, 3H), 2.28 (s, 3H).

### Beispiel 88

### 2-[5-(2,2-Dimethylpropoxy)pyridin-2-yl]-4-pyridin-3-yl-1,2-dihydro-3H-pyrazol-3-on

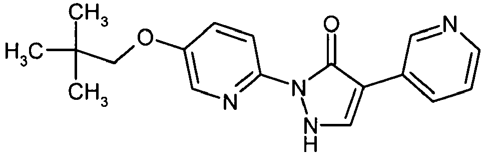

220 mg (1.0 mmol) der Verbindung aus Beispiel 2A werden in 5 ml Ethanol vorgelegt. Man gibt 260 mg (Reinheit 75%, 1.0 mmol) der Verbindung aus Beispiel 19A sowie 38 mg (0.2 mmol) p-Toluolsulfonsäure hinzu und rührt 16 h unter Rückfluss. Man lässt danach auf RT abkühlen, filtriert den gebildeten Feststoff ab, wäscht mit *tert*.-Butylmethylether und trocknet den Feststoff im Hochvakuum. Man erhält so 37 mg der Titelverbindung. Die Mutterlauge wird verwahrt und zur Darstellung des Hydrochlorids verwendet (siehe Beispiel 89).
Ausbeute: 37 mg (11% d. Th.)
LC-MS (Methode 5): Rₜ = 1.05 min; MS (ESIpos): m/z = 325 [M+H]⁺;
¹H-NMR (400 MHz, DMSO-d₆): δ = 9.08 (s, 1H), 8.45-8.35 (m, 2H), 8.32-8.15 (m, 3H), 7.70 (dd, 1H), 7.37 (dd; 1H), 3.76 (s, 2H), 1.01 (s, 9H).

### Beispiel 89

### 2-[5-(2,2-Dimethylpropoxy)pyridin-2-yl]-4-pyridin-3-yl-1,2-dihydro-3H-pyrazol-3-on-Hydrochlorid

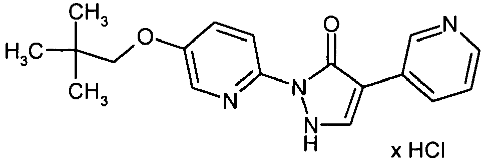

Die im Zuge der Darstellung von Beispiel 88 erhaltene Mutterlauge (siehe oben) wird am Rotationsverdampfer eingeengt und der Rückstand mittels präparativer HPLC gereinigt (RP18-Säule; Laufmittel: Acetonitril/Wasser-Gradient unter Zusatz von 0.1% TFA im Wasser). Der nach Einengen der Produktfraktionen erhaltene Rückstand wird 30 min in 5 ml einer 4 N Lösung von Chlorwasserstoff in Dioxan verrührt. Man filtriert den Feststoff ab, wäscht zweimal mit *tert*.-Butyl-methylether und trocknet im Hochvakuum.
Ausbeute: 160 mg (44% d. Th., bezogen auf 1.0 mmol der Verbindung aus Beispiel 19A)
LC-MS (Methode 2): Rₜ = 1.52 min; MS (ESIpos): m/z = 325 [M+H]⁺;
¹H-NMR (400 MHz, DMSO-d₆): δ = 9.39 (s, 1H), 8.96 (d, 1H), 8.74 (s, 1H), 8.64 (d, 1H), 8.30 (d, 1H), 8.21 (d, 1H), 8.00 (dd, 1H), 7.70 (dd, 1H), 3.78 (s, 2H), 1.01 (s, 9H).

### B. Bewertung der pharmakologischen Wirksamkeit

Die pharmakologischen Eigenschaften der erfindungsgemäßen Verbindungen können in folgenden Assays gezeigt werden:

### Abkürzungen:

- DMEM: Dulbecco's Modified Eagle Medium
- FCS: Fetal Calf Serum
- TMB: 3,3',5,5'-Tetramethylbenzidin
- Tris: Tris(hydroxymethyl)-aminomethan

### 1. In vitro-Tests zur Bestimmung der Aktivität und Selektivität von HIF-Prolyl-4-Hydroxylase-Inhibitoren

### 1.a) Hemmung der Aktivität von HIF-Prolylhydroxylase:

Hydroxylierter HIF bindet spezifisch an den von Hippel-Lindau Protein-Elongin B-Elongin C-Komplex (VBC-Komplex). Diese Interaktion tritt nur auf, wenn HIF an einem konservierten Prolylrest hydroxyliert ist. Sie ist die Grundlage für die biochemische Bestimmung der HIF-Prolylhydroxylase-Aktivität. Der Test wird wie beschrieben durchgeführt [Oehme F., Jonghaus W., Narouz-Ott L., Huetter J., Flamme I., Anal. Biochem. 330 (1), 74-80 (2004)]:
Eine klare, mit NeutrAvidin HBC beschichtete 96-Loch-Mikrotiterplatte (Fa. Pierce) wird für 30 Minuten mit Blocker-Casein inkubiert. Anschließend wird die Platte dreimal mit je 200 µl Waschpuffer (50 mM Tris, pH 7.5, 100 mM NaCl, 10% (v/v) Blocker-Casein, 0.05% (v/v) Tween 20) pro Loch gewaschen. Das Peptid Biotin-DLDLEMLAPYIPMDDDFQL (Fa. Eurogentec, 4102 Seraing, Belgien) wird in einer Konzentration von 400 nM in 100 µl Waschpuffer zugegeben. Dieses Peptid dient als Substrat für die Prolylhydroxylierung und wird an die Mikrotiterplatte gebunden. Nach 60 Minuten Inkubation wird die Platte dreimal mit Waschpuffer gewaschen, 30 Minuten mit 1 mM Biotin in Blocker-Casein inkubiert und dann erneut dreimal mit Waschpuffer gewaschen.

Zur Durchführung der Prolylhydroxylase-Reaktion wird das an die Platte gebundene Peptidsubstrat 1 bis 60 Minuten mit einem Prolylhydroxylase-haltigen Zelllysat inkubiert. Die Reaktion findet in 100 µl Reaktionspuffer (20 mM Tris, pH 7.5, 5 mM KCl, 1.5 mM MgCl₂, 1 µM-1 mM 2-Oxo-glutarat, 10 µM FeSO₄, 2 mM Ascorbat) bei Raumtemperatur statt. Die Reaktionsmischung enthält außerdem den zu testenden Hemmstoff der Prolylhydroxylase in verschiedenen Konzentrationen. Die Testsubstanz wird bevorzugt, aber nicht ausschließlich bei Konzentrationen zwischen 1 nM und 100 µM eingesetzt. Durch dreimaliges Waschen der Platte mit Waschpuffer wird die Reaktion gestoppt.

Zur quantitativen Bestimmung der Prolylhydroxylierung wird ein Fusionsprotein, das sowohl Thioredoxin aus E. coli als auch den VBC-Komplex enthält, in 80 µl Bindungspuffer (50 mM Tris, pH 7.5, 120 mM NaCl) zugegeben. Nach 15 Minuten werden 10 µl einer Lösung von polyklonalem Anti-Thioredoxin-Antikörper aus Kaninchen in Bindungspuffer zugefügt. Nach weiteren 30 Minuten setzt man 10 µl einer Lösung von mit Meerrettich-Peroxidase gekoppeltem Anti-Kaninchen-Immunglobulin in Bindungspuffer hinzu. Nach 30 Minuten Inkubation bei Raumtemperatur wird dreimal mit Waschpuffer gewaschen, um ungebundenen VBC-Komplex und Antikörper zu entfernen. Um die Menge an gebundenem VBC-Komplex zu bestimmen, wird 15 Minuten mit TMB inkubiert. Die Farbreaktion wird durch Zugabe von 100 µl 1 M Schwefelsäure beendet. Durch Messung der optischen Dichte bei 450 nm wird die Menge an gebundenem VBC-Komplex bestimmt. Sie ist proportional zur Menge an hydroxyliertem Prolin im Peptidsubstrat.

Zur Detektion der Prolylhydroxylierung kann alternativ ein mit Europium (Fa. Perkin Elmer) gekoppelter VBC-Komplex verwendet werden. In diesem Fall wird die Menge an gebundenem VBC-Komplex durch zeitaufgelöste Fluoreszenz bestimmt. Außerdem ist die Verwendung von mit [³⁵S]-Methionin markiertem VBC-Komplex möglich. Hierfür kann der radioaktiv markierte VBC-Komplex *durch in vitro*-Transkription-Translation in Retikulozytenlysat hergestellt werden.

Die Ausführungsbeispiele hemmen die Aktivität der HIF-Prolylhydroxylase in diesem Test mit einem IC₅₀-Wert von ≤ 30 µM. In der nachstehenden Tabelle 1 sind repräsentative IC₅₀-Werte zu den Ausführungsbeispielen wiedergegeben:

**Tabelle 1**

| **Beispiel Nr.** | **IC₅₀ [µM]** |
|---|---|
| 5 | 6.3 |
| 8 | 5.2 |
| 10 | 0.26 |
| 18 | 0.91 |
| 22 | 3.24 |
| 65 | 0.9 |
| 80 | 1.6 |
| 89 | 3.7 |

### 1.b) Zellulärer, funktioneller in vitro-Test:

Die Quantifizierung der Wirksamkeit der erfindungsgemäßen Verbindungen erfolgt mit Hilfe einer rekombinanten Zelllinie. Die Zelle leitet sich ursprünglich von einer humanen Lungencarcinom-- Zelllinie ab (A549, ATCC: American Type Culture Collection, Manassas, VA 20108, USA). Die Testzelllinie wird stabil mit einem Vektor transfiziert, der das Reportergen der *Photinus pyralis-*Luciferase (im folgenden Luciferase genannt) unter der Kontrolle eines artifiziellen Minimalpromotors enthält. Der Minimalpromotor besteht aus zwei Hypoxie-responsiblen Elementen stromaufwärts einer TATA-Box [Oehme F., Ellinghaus P., Kolkhof P., Smith T.J., Ramakrishnan S., Hütter J., Schramm M., Flamme I., Biochem. Biophys. Res. Commun. 296 (2), 343-9 (2002)]. Unter Einwirkung von Hypoxie (z.B. Kultivierung in Gegenwart von 1% Sauerstoff für 24 Stunden) oder unter Einwirkung unselektiver Dioxygenase-Inhibitoren (z.B. Desferroxamin in einer Konzentration von 100 µM, Cobaltchlorid in einer Konzentration von 100 µM oder *N*-Oxalylglycindiethylester in einer Konzentration von 1 mM) produziert die Testzelllinie Luciferase, die mit Hilfe geeigneter Biolumineszenz-Reagenzien (z.B. Steady-Glo^{®} Luciferase Assay System, Promega Corporation, Madison, WI 53711, USA) und eines geeigneten Luminometers detektiert und quantifiziert werden kann.

Testablauf: Die Zellen werden am Tag vor dem Test in einer exakt bemessenen Menge Kulturmedium (DMEM, 10% FCS, 2 mM Glutamin) in 384- oder 1536-Loch-Mikrotiterplatten ausplattiert und in einem Zellinkubator (96% Luftfeuchtigkeit, 5% v/v CO₂, 37°C) gehalten. Am Testtag werden dem Kulturmedium die Testsubstanzen in abgestuften Konzentrationen zugesetzt. In als Negativkontrolle dienenden Ansätzen wird den Zellen keine Testsubstanz zugesetzt. Als Positivkontrolle zur Bestimmung der Empfindlichkeit der Zelle für Inhibitoren wird z.B. Desferroxamin in einer Endkonzentration von 100 µM zugesetzt. Sechs bis 24 Stunden nach Übertragung der Testsubstanzen in die Löcher der Mikrotiterplatten wird das resultierende Lichtsignal im Luminometer gemessen. Anhand der Meßwerte wird eine Dosiswirkungsbeziehung aufgestellt, die als Grundlage für die Ermittlung der halbmaximalen Wirkkonzentration (als EC₅₀-Wert bezeichnet) dient.

### 1.c) Zellulärer, funktioneller in vitro-Test zur Veränderung der Genexpression:

Um die Veränderung der Expression spezifischer mRNAs in menschlichen Zelllinien nach Behandlung mit Testsubstanzen zu untersuchen, werden folgende Zelllinien auf 6- oder 24-Lochplatten kultiviert: humane Hepatomzellen (HUH, JCRB Cell Bank, Japan), humane embryonale Nierenfibroblasten (HEK/293, ATCC, Manassas, VA 20108, USA), humane Cervixcarcinomzellen (HeLa, ATCC, Manassas, VA 20108, USA), humane Nabelschnurvenenendothelzellen (HUVEC, Cambrex, East Rutherford, New Jersey 07073, USA). 24 Stunden nach Zugabe der Testsubstanzen werden die Zellen mit Phosphat-gepufferter Saline gewaschen und aus ihnen die Gesamt-RNA unter Verwendung einer geeigneten Methode gewonnen (z.B. Trizol^{®}-Reagenz, Invitrogen GmbH, 76131 Karlsruhe, Deutschland).

Für ein typisches Analyseexperiment wird je 1 µg der so gewonnenen Gesamt-RNA mit DNase I verdaut und unter Verwendung einer geeigneten Reversen-Transkriptase-Reaktion (ImProm-II Reverse Transcription System, Promega Corporation, Madison, WI 53711, USA) in eine komplementäre DNA (cDNA) übersetzt. 2.5% des so gewonnenen cDNA-Ansatzes werden jeweils für die Polymerase-Kettenreaktion verwendet. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht. Die hierbei benutzten Primer-Sonden-Kombinationen werden mittels der Primer Express 1.5 Software (Fa. Applied Biosystems, Inc.) generiert. Im einzelnen werden die mRNAs von Erythropoetin, Carboanhydrase IX, Lactatdehydrogenase A und vaskulärem Endothelzellwachstumsfaktor untersucht.

Substanzen gemäß der vorliegenden Erfindung führen zu einem signifikanten, dosisabhängigen Anstieg der mRNA Hypoxie-induzierter Gene in Zellen menschlichen Ursprungs.

### 2. In vivo-Tests zum Nachweis der Wirkung im Kardiovaskularsystem

### 2.a) In vivo-Test zur Veränderung der Genexpression:

Mäusen oder Ratten werden die in geeigneten Lösungsmitteln gelösten Prüfverbindungen entweder oral durch Schlundsonden-Applikation, intraperitoneal oder intravenös verabfolgt. Typische Dosierungen sind 0.1, 0.5, 1, 5, 10, 20, 50, 100 und 300 mg Substanz pro kg Körpergewicht und Verabfolgung. Kontrolltiere erhalten nur Lösungsmittel. 4, 8 oder 24 Stunden nach Gabe der Prüfsubstanz werden die Tiere mit einer Überdosis Isofluran und anschließendem Genickbruch getötet und die zu untersuchenden Organe entnommen. Teile der Organe werden in flüssigem Stickstoff schockgefroren. Aus den Organteilen wird wie unter B.1.a) beschrieben Gesamt-RNA gewonnen und diese in eine cDNA übersetzt. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht.

Substanzen gemäß der vorliegenden Erfindung führen im Vergleich mit der Placebo-Kontrolle nach oraler oder parenteraler Verabreichung zu einem signifikanten, dosisabhängigen Anstieg der mRNA des Erythropoetins in der Niere.

### 2.b) Bestimmung des Erythropoetin-Spiegels im Serum:

Mäusen oder Ratten wird die Prüfsubstanz in einem geeigneten Lösungsmittel entweder intra-peritoneal oder oral einmal oder zweimal täglich verabreicht. Typische Dosierungen sind 0.1, 0.5, 1, 5, 10, 20, 50, 100 und 300 mg Substanz pro kg Körpergewicht und Verabfolgung. Placebo-Kontrolltiere erhalten nur Lösungsmittel. Vor der Applikation und vier Stunden nach der letzten Substanzgabe wird den Tieren in Kurznarkose aus dem retroorbitalen Venenplexus oder der Schwanzvene 50 µl Blut entnommen. Das Blut wird durch Zusatz von Lithium-Heparin ungerinnbar gemacht. Durch Zentrifugieren wird das Blutplasma gewonnen. In dem Blutplasma wird mit Hilfe eines Erythropoetin-ELISA (Quantikine^{®} mouse Epo Immunoassay, R&D Systems, Inc., Minneapolis, USA) entsprechend der Anleitung des Herstellers der Gehalt an Erythropoetin bestimmt. Die Meßwerte werden anhand einer für Maus-Erythropoetin erhobenen Referenzmessung in pg/ml umgerechnet.

Substanzen gemäß der vorliegenden Erfindung führen nach oraler und parenteraler Verabreichung zu einem signifikanten, dosisabhängigen Anstieg des Plasma-Erythropoetins gegenüber dem Ausgangswert und der Placebo-Kontrolle.

### 2.c) Bestimmung der zellulären Zusammensetzung des peripheren Blutes:

Mäusen oder Ratten wird die Prüfsubstanz in einem geeigneten Lösungsmittel entweder intra-peritoneal oder oral einmal oder zweimal täglich über mehrere Tage verabreicht. Typische Dosierungen sind z.B. 0.1, 0.5, 1, 5, 10, 20, 50, 100 und 300 mg Substanz pro kg Körpergewicht und Verabfolgung. Kontrolltiere erhalten nur Lösungsmittel. Am Versuchsende wird den Tieren in Kurznarkose aus dem Venenplexus des Augenwinkels oder der Schwanzvene Blut entnommen und durch Zusatz von Natriumcitrat ungerinnbar gemacht. In einem geeigneten elektronischen Messgerät werden in den Blutproben die Konzentrationen von Erythrozyten, Leukozyten und Thrombozyten bestimmt. Die Konzentration der Retikulozyten wird anhand von Blutausstrichen, die mit einer dafür geeigneten Farblösung (Fa. KABE Labortechnik, Nümbrecht) gefärbt werden, durch mikroskopische Durchmusterung von je 1000 Erythrozyten bestimmt. Für die Bestimmung des Hämatokrits wird Blut aus dem retroorbitalen Venenplexus mittels einer Hämatokritkapillare entnommen und der Hämatokritwert nach Zentrifugieren der Kapillare in einer dafür geeigneten Zentrifuge manuell abgelesen.

Substanzen gemäß der vorliegenden Erfindung führen nach oraler und parenteraler Verabreichung zu einem signifikanten, dosisabhängigen Anstieg des Hämatokrits, der Erythrozytenzahl und der Retikulozyten gegenüber dem Ausgangswert und der Placebo-Kontrolle.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 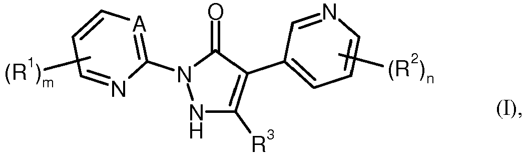 in welcher
A für CH steht,
R¹ für einen Substituenten ausgewählt aus der Reihe (C₃-C₆)-Cycloalkyl, 4- bis 6-gliedriges Heterocycloalkyl, Phenyl, 5- oder 6-gliedriges Heteroaryl, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹ und -NR²⁵R²⁶ steht, worin
(i) (C₃-C₆)-Cycloalkyl, 4- bis 6-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, Halogen, Cyano, Oxo, Hydroxy, Amino, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert sein können,
wobei der zuletzt genannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl, 4- bis 6-gliedrigem Heterocycloalkyl, Phenyl und/oder 5- oder 6-gliedrigem Heteroaryl substituiert sein kann,
(*ii*) R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
(*iii*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und/oder worin
(*iv*) R⁶ und R⁷, R⁹ und R¹⁰, R¹¹ und R¹², R¹³ und R¹⁴, R¹⁵ und R¹⁶, R¹⁸ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
R² für einen Substituenten ausgewählt aus der Reihe -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} und -NR^{25A}R^{26A} steht, worin
(i) R^{9A}, R^{12A}, R^{14A}, R^{16A}, R^{19A} und R^{25A} unabhängig voneinander für (C₁-C₆)-Alkyl, welches jeweils mit Halogen, Cyano, Trifluormethyl, Hydroxy, Tri-fluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*ii*) R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxy-carbonyl substituiert sein kann, stehen,
und/oder worin
(*iii*) R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
m für die Zahl 1 steht,
n für die Zahl 0 oder 1 steht
und
R³ für Wasserstoff steht,
sowie ihre Salze, Solvate und Solvate der Salze.

2. Verbindung der Formel (I), in welcher
A für CH steht,
R¹ für einen Substituenten ausgewählt aus der Reihe (C₃-C₆)-Cycloalkyl, 4- bis 6-gliedriges Heterocycloalkyl, Phenyl, 5- oder 6-gliedriges Heteroaryl, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹ und -NR²⁵R²⁶ steht, worin
(i) (C₃-C₆)-Cycloalkyl, 4- bis 6-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, Halogen, Cyano, Oxo, Hydroxy, Amino, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert sein können,
wobei der zuletzt genannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxy-carbonyl, (C₁-C₄)-Alkoxycarbonyl, (C₃-C₆)-Cycloalkyl, 4- bis 6-gliedrigem Heterocycloalkyl, Phenyl und/oder 5- oder 6-gliedrigem Heteroaryl substituiert sein kann,
*(ii)* R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
*(iii)* R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und/oder worin
(*iv*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
R² für einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino, Hydroxycarbonyl und -C(=O)-NH-R^{7A} steht, worin
(C₁-C₆)-Alkyl und (C₁-C₆)-Alkoxy ihrerseits mit Hydroxy substituiert sein können
und
R^{7A} Wasserstoff oder (C₁-C₄)-Alkyl bedeutet,
m für die Zahl 1 steht,
n für die Zahl 0 oder 1 steht
und
R³ für Wasserstoff steht,
sowie ihre Salze, Solvate und Solvate der Salze.

3. Verbindung der Formel (I), in welcher
A für CH steht,
R¹ für einen Substituenten ausgewählt aus der Reihe (C₁-C₆)-Alkyl, Trifluormethyl, Halogen, Cyano, Nitro, Hydroxy, (C₁-C₆)-Alkoxy, Amino, Hydroxycarbonyl, (C₁-C₆)-Alkoxycarbonyl und -C(=O)-NH-R⁷ steht, worin
(C₁-C₆)-Alkyl seinerseits mit Hydroxy, (C₁-C₄)-Alkoxy, Amino, Mono-(C₁-C₄)-alkylamin, Di-(C₁-C₄)-alkylamino oder einer Gruppe der Formel -NH-C(=O)-R¹², -NH-C(=O)-NH-R¹⁶ oder -NH-SO₂-R¹⁹ substituiert sein kann, worin
R¹², R¹⁶ und R¹⁹ jeweils (C₁-C₆)-Alkyl, welches mit Hydroxy oder (C₁-C₄)-Alkoxy substituiert sein kann, bedeuten,
und
R⁷ Wasserstoff oder (C₁-C₆)-Alkyl, das mit Hydroxy oder (C₁-C₄)-Alkoxy substituiert sein kann, bedeutet,
R² für einen Substituenten ausgewählt aus der Reihe -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} und -NR^{25A}R^{26A} steht, worin
(*i*) R^{9A}, R^{12A}, R^{14A}, R^{16A}, R^{19A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*ii*) R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
und/oder worin
(*iii*) R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
m für die Zahl 1 steht,
n für die Zahl 1 steht
und
R³ für Wasserstoff steht,
sowie ihre Salze, Solvate und Solvate der Salze.

4. Verbindung der Formel (I), in welcher
A für CH steht,
R¹ für (C₁-C₆)-Alkyl steht, welches
(i) ein- oder zweifach, gleich oder verschieden, mit einem Rest ausgewählt aus der Reihe Halogen, Cyano, Trifluormethyl, (C₃-C₆)-Cycloalkyl, 4- bis 6-gliedriges Heterocycloalkyl, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist
und darüber hinaus mit Hydroxy, (C₁-C₄)-Alkoxy, Amino, Mono-(C₁-C₄)-alkylamino, Di-(C₁-C₄)-alkylamino oder (C₁-C₄)-Acylamino substituiert sein kann,
oder
(*ii*) zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkylamino und (C₁-C₄)-Acylamino substituiert ist,
worin
(*a*) die oben genannten Cycloalkyl- und Heterocycloalkyl-Reste ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*b*) R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono(C₁-C₄)-alkylamino, Di-(C₁-C₄)-alkylamino, Hydroxycarbonyl und/oder (C₁-C₄)-Alkoxycarbonyl substituiert sein kann,
(c) R⁶, R⁹ und R¹⁴ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
(*d*) R¹², R¹⁶ und R¹⁹ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit einem Rest ausgewählt aus der Reihe Halogen, Cyano, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist,
*(e)* R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit einem Rest ausgewählt aus der Reihe Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist,
und/oder worin
(*f*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
R² für einen Substituenten ausgewählt aus der Reihe Halogen, Cyano, (C₁-C₆)-Alkyl, Trifluormethyl, Hydroxy, Trifluormethoxy, Amino, Hydroxycarbonyl, Aminocarbonyl, -C(=O)-NR^{6A}R^{7A} -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A}, -OR^{23A} und -NR^{25A}R^{26A} steht, worin
(i) (C₁-C₆)-Alkyl mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann,
(*ii*) R^{6A}, R^{9A}, R^{12A}, R^{14A}, R^{16A}, R^{19A}, R^{23A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
(*iii*) R^{7A}, R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
und/oder worin
(*iv*) R^{6A} und R^{7A}, R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
m für die Zahl 1 steht,
n für die Zahl 0 oder 1 steht
und
R³ für Wasserstoff steht,
sowie ihre Salze, Solvate und Solvate der Salze.

5. Verbindung der Formel (I), in welcher
A für CH steht,
R¹ für (C₁-C₆)-Alkoxy steht, welches ein- oder zweifach, gleich oder verschieden, mit einem Rest ausgewählt aus der Reihe Halogen, Cyano, Trifluormethyl, (C₃-C₆)-Cycloalkyl, 4- bis 6-gliedriges Heterocycloalkyl, Hydroxy, Amino, Hydroxycarbonyl, Aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist, worin
(i) (C₃-C₆)-Cycloalkyl und 4- bis 6-gliedriges Heterocycloalkyl ihrerseits jeweils ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*ii*) R⁶, R⁹, R¹², R¹⁴, R¹⁶, R¹⁹, R²³ und R²⁵ unabhängig voneinander bei jedem einzelnen Auftreten für einen Rest ausgewählt aus der Reihe (C₁-C₆)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
*(iii)* R⁷, R¹⁰, R¹¹, R¹³, R¹⁵, R¹⁷, R¹⁸ und R²⁶ unabhängig voneinander bei jedem einzelnen Auftreten für einen Rest ausgewählt aus der Reihe Wasserstoff und (C₁-C₆)-Alkyl stehen,
wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und/oder worin
(*iv*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
R² für einen Substituenten ausgewählt aus der Reihe Halogen, Cyano, (C₁-C₆)-Alkyl, Trifluormethyl, Hydroxy, Trifluormethoxy, Amino, Hydroxycarbonyl, Aminocarbonyl, -C(=O)-NR^{6A}R^{7A}, -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A}, -OR^{23A} und -NR^{25A}R^{26A} steht, worin
(i) (C₁-C₆)-Alkyl mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann,
*(ii)* R^{6A}, R^{9A}, R^{12A}, R^{14A}, R^{16A}, R^{19A}, R^{23A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
*(iii)* R^{7A}, R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
und/oder worin
(*iv*) R^{6A} und R^{7A}, R^{9A} und R^{10A}, R^{11A} und R^{12A}, R^{13A} und R^{14A}, R^{15A} und R^{16A}, R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
m für die Zahl 1 steht,
n für die Zahl 0 oder 1 steht
und
R³ für Wasserstoff steht,
sowie ihre Salze, Solvate und Solvate der Salze.

6. Verbindung der Formel (I), in welcher
A für CH steht,
R¹ für die Gruppe -C(=O)-NR⁶R⁷ steht,
worin (i)
R⁶ (C₃-C₆)-Cycloalkyl oder 4- bis 6-gliedriges Heterocycloalkyl bedeutet, welche jeweils ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
oder
(C₁-C₆)-Alkyl bedeutet, welches
*(a)* ein- oder zweifach, gleich oder verschieden, mit einem Rest ausgewählt aus der Reihe Halogen, Cyano, Trifluormethyl, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist
und darüber hinaus mit Hydroxy oder (C₁-C₄)-Alkoxy substituiert sein kann,
oder
(b) zweifach mit Hydroxy und/oder (C₁-C₄)-Alkoxy substituiert ist,
und
R⁷ Wasserstoff bedeutet,
oder worin (*ii*)
R⁶ (C₁-C₆)-Alkyl, (C₃-C₆)-Cycloalkyl oder 4- bis 6-gliedriges Heterocycloalkyl bedeutet, wobei (C₃-C₆)-Cycloalkyl und 4- bis 6-gliedriges Heterocycloalkyl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxy-carbonyl und/oder (C₁-C₄)-Alkoxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl und/oder 4- bis 6-gliedrigem Heterocycloalkyl substituiert sein kann,
und
R⁷ (C₁-C₆)-Alkyl bedeutet, welches ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/ oder (C₁-C₄)-Alkoxycarbonyl substituiert sein kann,
oder worin *(iii)*
R⁶ und R⁷ gemeinsam mit dem Stickstoffatom, an das sie gebunden sind, einen 5-oder 6-gliedrigen Heterocycloalkyl-Ring bilden, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono(C₁-C₄)-alkylamino, Di-(C₁-C₄)-alkylamino, Hydroxycarbonyl und/oder (C₁-C₄)-Alkoxycarbonyl substituiert sein kann,
R² für einen Substituenten ausgewählt aus der Reihe Halogen, Cyano, (C₁-C₆)-Alkyl, Trifluormethyl, Hydroxy, Trifluormethoxy, Amino, Hydroxycarbonyl, Aminocarbonyl, -C(=O)-NR^{6A}R^{7A} -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A}, -OR^{23A} und -NR^{25A}R^{26A} steht, worin
(i) (C₁-C₆)-Alkyl mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann,
*(ii)* R^{6A}, R^{9A}, R^{12A}, R^{14A}, R^{16A}, R^{19A}, R^{23A} und R^{25A} unabhängig voneinander für (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
*(iii)* R^{7A}, R^{10A}, R^{11A}, R^{13A}, R^{15A}, R^{17A}, R^{18A} und R^{26A} unabhängig voneinander für Wasserstoff oder (C₁-C₆)-Alkyl, welches jeweils mit Halogen, Cyano, Trifluormethyl, Hydroxy, Trifluormethoxy, (C₁-C₄)-Alkoxy, Amino, Mono-(C₁-C₄)-alkylamino, Di-(C₁-C₄)-alkylamino, Hydroxycarbonyl oder (C₁-C₄)-Alkoxycarbonyl substituiert sein kann, stehen,
und/oder worin
(*iv*) R^{6A} und R^{7A}, R^{9A} und R^{10A}, R^{11A} und R^{12A}, R^{13A} und R^{14A}, R^{15A} und R^{16A} R^{16A} und R^{17A}, R^{18A} und R^{19A} sowie R^{25A} und R^{26A} jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di(C₁-C₄)-alkylamino, Hydroxycarbonyl und/oder (C₁-C₄)-Alkoxycarbonyl substituiert sein kann,
m für die Zahl 1 steht,
n für die Zahl 0 oder 1 steht
und
R³ für Wasserstoff steht,
sowie ihre Salze, Solvate und Solvate der Salze.

7. Verbindung nach Anspruch 2 der Formel (I-A) oder (I-B) 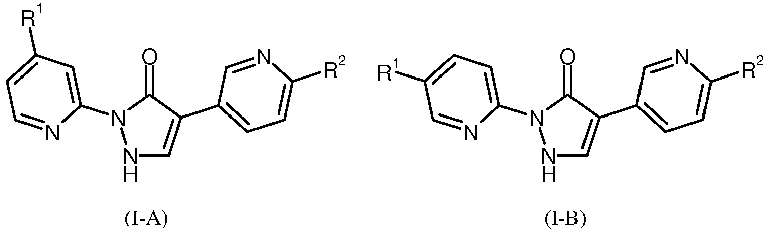 in welchen
R¹ für 4- bis 6-gliedriges Heterocycloalkyl, Phenyl oder 5- oder 6-gliedriges Heteroaryl steht, welche jeweils ein- oder zweifach, gleich oder verschieden, mit einem Rest ausgewählt aus der Reihe Fluor, Chlor, Brom, Cyano, (C₁-C₄)-Alkyl, Hydroxy, Amino, Hydroxycarbonyl, -OR²³ und -NR²⁵R²⁶ substituiert sein können, worin
(C₁-C₄)-Alkyl seinerseits ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl, 4-bis 6-gliedrigem Heterocycloalkyl, Phenyl und/oder 5- oder 6-gliedrigem Heteroaryl substituiert sein kann,
R²³ und R²⁵ unabhängig voneinander bei jedem einzelnen Auftreten (C₁-C₄)-Alkyl bedeuten, welches ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und/ oder 4- bis 6-gliedrigem Heterocycloalkyl substituiert sein kann,
und
R²⁶ bei jedem einzelnen Auftreten Wasserstoff oder (C₁-C₄)-Alkyl bedeutet, welches ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann, und
R² für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

8. Verbindung nach Anspruch 4 der Formel (I-A) oder (I-B) 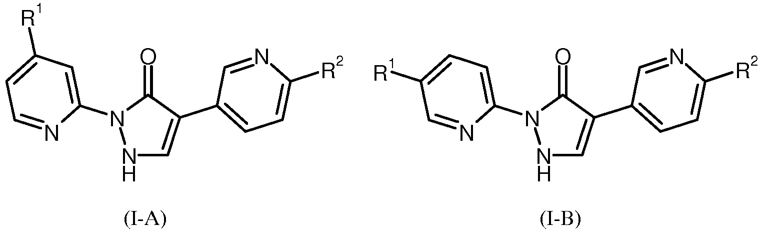 in welchen
R¹ für (C₁-C₆)-Alkyl steht, welches
(*i*) ein- oder zweifach, gleich oder verschieden, mit einem Rest ausgewählt aus der Reihe Fluor, Chlor, Cyano, Trifluormethyl, (C₃-C₆)-Cycloalkyl, 4- bis 6-gliedriges Heterocycloalkyl, Hydroxycarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R-²⁶ substituiert ist
und darüber hinaus mit Hydroxy, (C₁-C₄)-Alkoxy, Amino, Mono-(C₁-C₄)-alkylamino, Di-(C₁-C₄)-alkylamino oder (C₁-C₄)-Acylamino substituiert sein kann,
oder
(*ii*) zweifach, gleich oder verschieden, mit einem Rest ausgewählt aus der Reihe Hydroxy, (C₁-C₄)-Alkoxy, Amino, Mono-(C₁-C₄)-alkylamino, Di-(C₁-C₄)-alkylamino und (C₁-C₄)-Acylamino substituiert ist,
worin
*(a)* die oben genannten Cycloalkyl- und Heterocycloalkyl-Reste ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können,
(*b*) R⁷, R¹⁰, R¹¹, R¹³, R¹⁵ , R¹⁷ , R¹⁸ und R²⁶ unabhängig voneinander bei jedem einzelnen Auftreten für einen Rest ausgewählt aus der Reihe Wasserstoff und (C₁-C₄)-Alkyl stehen,
wobei (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
(c) R⁶, R⁹ und R¹⁴ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
und
(C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und/oder 4- bis 6-gliedrigem Heterocycloalkyl substituiert sein kann,
(*d*) R¹², R¹⁶ und R¹⁹ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
und
(C₁-C₄)-Alkyl ein- bis dreifach, gleich oder verschieden, mit einem Rest ausgewählt aus der Reihe Fluor, Chlor, Cyano, Amino, Mono-(C₁-C₄)alkylamino, Di-(C₁-C₄)-alkylamino, Hydroxycarbonyl, (C₁-C₄)-Alkoxycarbonyl, (C₃-C₆)-Cycloalkyl und 4- bis 6-gliedriges Heterocycloalkyl substituiert ist,
*(e)* R²³ und R²⁵ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
und
(C₁-C₄)-Alkyl ein- bis dreifach, gleich oder verschieden, mit einem Rest ausgewählt aus der Reihe Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und 4- bis 6-gliedriges Heterocycloalkyl substituiert ist,
und/oder worin
(*f*) R⁶ und R⁷, R⁹ und R¹⁰, R¹¹ und R¹², R¹³ und R¹⁴, R¹⁵ und R¹⁶ , R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Fluor, Chlor, Cyano, (C₁-C₄)-Alkyl, Trifluormethyl, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
R² für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

9. Verbindung nach Anspruch 5 der Formel (I-A) oder (I-B) 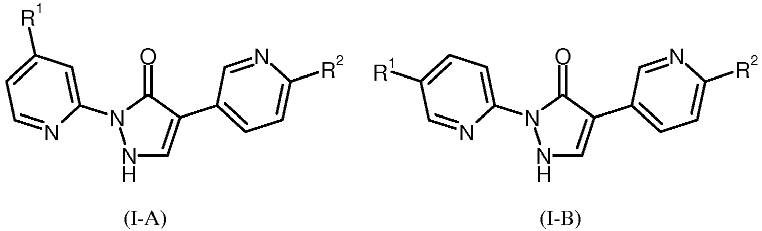 in welchen
R¹ für (C₁-C₆)-Alkoxy steht, welches ein- oder zweifach, gleich oder verschieden, mit einem Rest ausgewählt aus der Reihe Fluor, Chlor, Cyano, Trifluormethyl, (C₃-C₆)-Cycloalkyl, 4- bis 6-gliedriges Heterocycloalkyl, Hydroxy, Amino, Hydroxycarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ und -NR²⁵R²⁶ substituiert ist,
worin
(i) (C₃-C₆)-Cycloalkyl und 4- bis 6-gliedriges Heterocycloalkyl ihrerseits jeweils ein- bis dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können,
(*ii*) R⁶, R⁹, R¹², R¹⁴, R¹⁶ , R¹⁹, R²³ und R²⁵ unabhängig voneinander bei jedem einzelnen Auftreten für einen Rest ausgewählt aus der Reihe (C₁-C₄)-Alkyl, (C₃-C₆)-Cycloalkyl und 4- bis 6-gliedriges Heterocycloalkyl stehen, wobei
(C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können
und (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und/oder 4- bis 6-gliedrigem Heterocycloalkyl substituiert sein kann,
(*iii*) R⁷, R¹⁰, R¹¹, R¹³ , R¹⁵ , R¹⁷, R¹⁸ und R²⁶ unabhängig voneinander bei jedem einzelnen Auftreten für einen Rest ausgewählt aus der Reihe Wasserstoff und (C₁-C₄)-Alkyl stehen,
wobei (C₁-C₄)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
und/oder worin
(*iv*) R⁶ und R⁷, R⁹ und R¹⁰, R¹¹ und R¹², R¹³ und R¹⁴, R¹⁵ und R¹⁶, R¹⁶ und R¹⁷, R¹⁸ und R¹⁹ sowie R²⁵ und R²⁶ jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Fluor, Chlor, Cyano, (C₁-C₄)-Alkyl, Trifluormethyl, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
und
R² für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

10. Verbindung nach Anspruch 6 der Formel (I-A) oder (I-B) 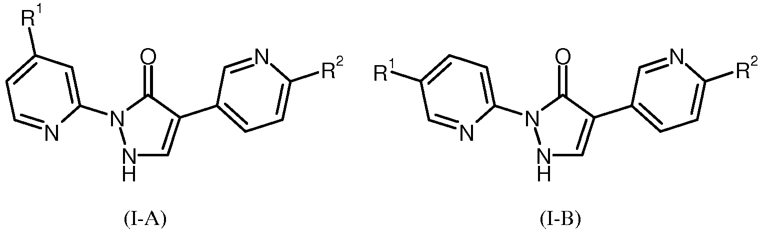 in welchen
R¹ für die Gruppe -C(=O)-NR⁶R⁷ steht,
worin (i)
R⁶ (C₃-C₆)-Cycloalkyl oder 4- bis 6-gliedriges Heterocycloalkyl bedeutet, welche jeweils ein- bis dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können,
oder
(C₁-C₆)-Alkyl bedeutet, welches
(*a*) ein- oder zweifach, gleich oder verschieden, mit einem Rest ausgewählt aus der Reihe Fluor, Chlor, Cyano, Trifluormethyl, Amino, Mono-(C₁-C₄)-alkylamino, Di-(C₁-C₄)-alkylamino, Hydroxycarbonyl, (C₃-C₆)-Cycloalkyl und 4- bis 6-gliedriges Heterocycloalkyl substituiert ist
und darüber hinaus mit Hydroxy oder (C₁-C₄)-Alkoxy substituiert sein kann,
oder
(*b*) zweifach mit Hydroxy und/oder (C₁-C₄)-Alkoxy substituiert ist, und
R⁷ Wasserstoff bedeutet,
oder worin (*ii*)
R⁶ (C₁-C₆)-Alkyl, (C₃-C₆)-Cycloalkyl oder 4- bis 6-gliedriges Heterocycloalkyl bedeutet, wobei (C₃-C₆)-Cycloalkyl und 4- bis 6-gliedriges Heterocycloalkyl ihrerseits jeweils bis zu dreifach, gleich oder verschieden, mit Fluor, Chlor, Cyano, (C₁-C₄)-Alkyl, Trifluormethyl, Hydroxy, (C₁-C₄)-Alkoxy, Oxo, Amino, Mono-(C₁-C₄)-alkylamino, Di-(C₁-C₄)-alkylamino und/oder Hydroxycarbonyl substituiert sein können und
(C₁-C₆)-Alkyl ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino, Hydroxycarbonyl, (C₃-C₆)-Cycloalkyl und/oder 4- bis 6-gliedrigem Heterocycloalkyl substituiert sein kann,
und
R⁷ (C₁-C₄)-Alkyl bedeutet, welches ein- bis dreifach, gleich oder verschieden, mit Fluor, Chlor, Cyano, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann,
oder worin (*iii*)
R⁶ und R⁷ gemeinsam mit dem Stickstoffatom, an das sie gebunden sind, einen 5-oder 6-gliedrigen Heterocycloalkyl-Ring bilden, welcher ein- bis dreifach, gleich oder verschieden, mit Fluor, Chlor, Cyano, (C₁-C₄)-Alkyl, Trifluormethyl, Hydroxy, (C₁-C₄)-Alkoxy, Amino, Mono-(C₁-C₄)-alkylamino, Di-(C₁-C₄)-alkylamino und/oder Hydroxycarbonyl substituiert sein kann, und
R² für Wasserstoff, Fluor, Chlor, Cyano, (C₁-C₄)-Alkyl, Trifluormethyl, Hydroxymethyl, (C₁-C₄)-Alkoxy, Trifluormethoxy, Amino oder Hydroxycarbonyl steht,
sowie ihre Salze, Solvate und Solvate der Salze.

11. Verfahren zur Herstellung einer Verbindung der Formel (I), wie in den Ansprüchen 1 bis 10 definiert, **dadurch gekennzeichnet, dass** man eine Verbindung der Formel (II) 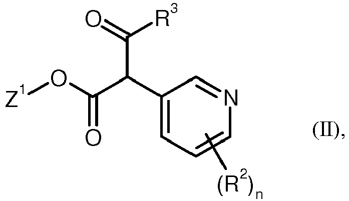 in welcher R², R³ und n die in den Ansprüchen 1 bis 10 angegebenen Bedeutungen aufweisen und
Z¹ für Methyl oder Ethyl steht,
in einem inerten Lösungsmittel gegebenenfalls in Gegenwart einer Säure mit einer Verbindung der Formel (III) 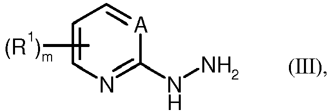 in welcher A, R¹ und m die in den Ansprüchen 1 bis 10 angegebenen Bedeutungen aufweisen,
zu Verbindungen der Formel (IV) 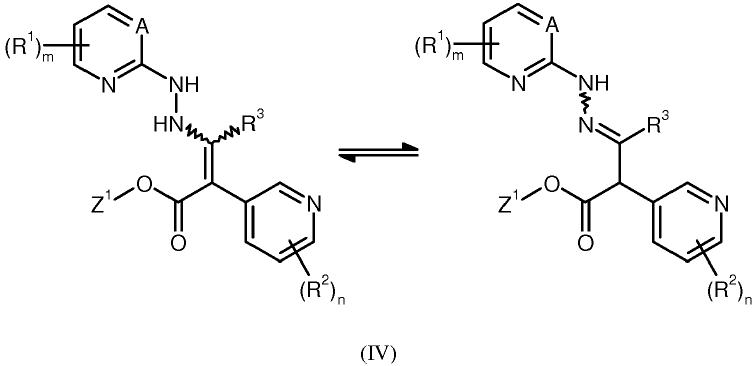 in welcher Z¹, A, R¹, R², R³, m und n die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Solvate, Salze und/oder Solvate der Salze überführt.

12. Verfahren zur Herstellung einer Verbindung der Formel (I), wie in den Ansprüchen 1 bis 10 definiert, in welcher R³ für Wasserstoff steht, **dadurch gekennzeichnet, dass** man zunächst eine Verbindung der Formel (V) 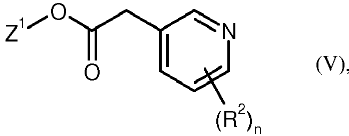 in welcher R² und n die in den Ansprüchen 1 bis 10 angegebenen Bedeutungen aufweisen
und
Z¹ für Methyl oder Ethyl steht,
mit einer Verbindung der Formel (VI) 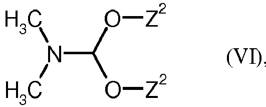 in welcher
Z² für Methyl oder Ethyl steht,
zu Verbindungen der Formel (VII) 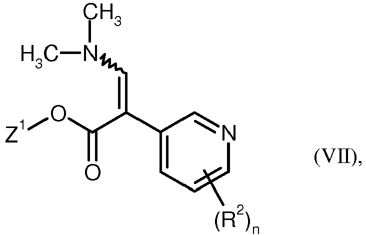 in welcher Z¹, R² und n die oben angegebenen Bedeutungen aufweisen,
kondensiert und anschließend in Gegenwart einer Säure mit einer Verbindung der Formel (III) 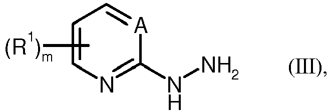 in welcher A, R¹ und m die in den Ansprüchen 1 bis 10 angegebenen Bedeutungen aufweisen,
zu Verbindungen der Formel (IV-A) 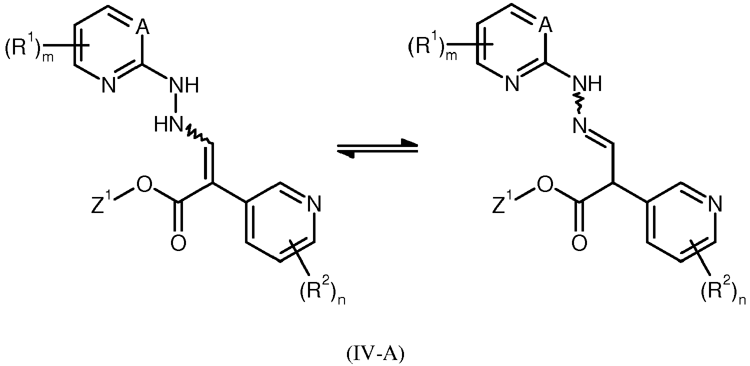 in welcher Z¹, A, R¹, R², m und n die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I), worin R³ für Wasserstoff steht, cyclisieren.

13. Verbindung, wie in einem der Ansprüche 1 bis 10 definiert, zur Behandlung und/oder Prophylaxe von Krankheiten.

14. Verwendung einer Verbindung, wie in einem der Ansprüche 1 bis 10 definiert, zur Herstellung eines Arzneimittels zur Behandlung und/oder Prophylaxe von Herz-Kreislauf-Erkrankungen, Herzinsuffizienz, Anämie, chronischen Nierenerkrankungen und Niereninsuffizienz.

15. Arzneimittel enthaltend eine Verbindung, wie in einem der Ansprüche 1 bis 10 definiert, in Kombination mit einem inerten, nicht-toxischen, pharmazeutisch geeigneten Hilfsstoff.

16. Arzneimittel enthaltend eine Verbindung, wie in einem der Ansprüche 1 bis 10 definiert, in Kombination mit einem oder mehreren weiteren Wirkstoffen ausgewählt aus der Gruppe bestehend aus ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika und Antibiotika.

17. Arzneimittel nach Anspruch 15 oder 16 zur Behandlung und/oder Prophylaxe von Herz-Kreislauf-Erkrankungen, Herzinsuffizienz, Anämie, chronischen Nierenerkrankungen und Niereninsuffizienz.

## Claims

1. Compound of the formula (I) 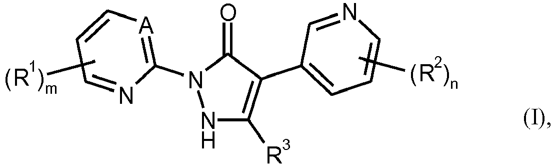 in which
A represents CH,
R¹ represents a substituent selected from the group consisting of (C₃-C₆)-cycloalkyl, 4- to 6-membered heterocycloalkyl, phenyl, 5- or 6-membered heteroaryl, -O-C(=O)-NR⁹R¹⁰, -NR¹¹⁻C(=O)-R¹², -NR¹³-C(=O)-OR¹⁴, -NR¹⁵-C(=O)-NR¹⁶R¹⁷, -NR¹⁸-SO₂-R¹⁹ and -NR²⁵R²⁶ in which
(*i*) (C₃-C₆)-cycloalkyl, 4- to 6-membered heterocycloalkyl, phenyl and 5-or 6-membered heteroaryl for their part may in each case be mono- to trisubstituted by identical or different radicals selected from the group consisting of (C₁-C₆)-alkyl, halogen, cyano, oxo, hydroxyl, amino, hydroxycarbonyl, aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ and -NR²⁵R²⁶,
where the last-mentioned alkyl radical for its part may be substituted up to three times by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl, 4- to 6-membered heterocycloalkyl, phenyl and 5- or 6-membered heteroaryl,
(*ii*) R⁶, R⁹, R¹², R¹⁴, R¹⁶, R¹⁹, R²³ and R²⁵ independently of one another for each individual occurrence represent a radical selected from the group consisting of (C₁-C₆)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl,
(*iii*) R⁷, R¹⁰, R¹¹, R¹³, R¹⁵, R¹⁷, R¹⁸ and R²⁶ independently of one another for each individual occurrence represent a radical selected from the group consisting of hydrogen and (C₁-C₆)-alkyl,
where (C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
and/or in which
(*iv*) R⁶ and R⁷, R⁹ and R¹⁰, R¹¹ and R¹², R¹³ and R¹⁴, R¹⁵ and R¹⁶, R¹⁶ and R¹⁷, R¹⁸ and R¹⁹ and also R²⁵ and R²⁶ in each case as a pair together with the atoms to which they are attached may form a 5- or 6-membered heterocycloalkyl ring which may be mono- to trisubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
R² represents a substituent selected from the group consisting of -O-C(=O)-NR^{9A}R^{10A}, -NR^{11A}-C(=O)-R^{12A} -NR^{13A}-C(=O)-OR^{14A}, -NR^{15A}-C(=O)-NR¹⁶AR^{17A}, -NR^{18A}-SO₂-R^{19A} and -NR ^{25A}R^{26A} in which
(*i*) R^{9A}, R^{12A} , R^{14A}, R^{16A}, R^{19A} and R^{25A} independently of one another represent (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
(*ii*) R^{10A}, R^{11A}, R^{13A}, R^{15A}, R^{17A} , R^{18A} and R^{26A} independently of one another represent hydrogen or (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl**,**
and/or in which
*(iii)* R^{9A} and R^{10A}, R^{11A} and R^{12A}, R^{13A} and R^{14A}, R^{15A} and R^{16A}, R^{16A} and R^{17A} , R^{18A} and R^{19A} and also R^{25A} and R^{26A} in each case as a pair together with the atoms to which they are attached may form a 5- or 6-membered heterocycloalkyl ring which may be mono- or disubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
m represents the number 1,
n represents the number 0 or 1 and
R³ represents hydrogen,
and salts, solvates and solvates of the salts thereof.

2. Compound of the formula (I) in which
A represents CH,
R¹ represents a substituent selected from the group consisting of (C₃-C₆)-cycloalkyl, 4- to 6-membered heterocycloalkyl, phenyl, 5- or 6-membered heteroaryl, -O-C(=O)-NR⁹R¹⁰, -NR¹¹-C(=O)-R¹², -NR¹³-C(=O)-OR¹⁴,-NR¹⁵-C(=O)-NR¹⁶R¹⁷, -NR¹⁸-SO₂-R¹⁹ and -NR²⁵R²⁶ in which
(*i*) (C₃-C₆)-cycloalkyl, 4- to 6-membered heterocycloalkyl, phenyl and 5-or 6-membered heteroaryl for their part may in each case be mono- to trisubstituted by identical or different radicals selected from the group consisting of (C₁-C₆)-alkyl, halogen, cyano, oxo, hydroxyl, amino, hydroxycarbonyl, aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ and -NR²⁵R²⁶,
where the last-mentioned alkyl radical for its part may be substituted up to three times by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl, 4- to 6-membered heterocycloalkyl, phenyl and 5- or 6-membered heteroaryl,
(*ii*) R⁶*,* R⁹, R¹² , R¹⁴, R¹⁶, R¹⁹, R²³ and R²⁵ independently of one another for each individual occurrence represent a radical selected from the group consisting of (C₁-C₆)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl,
*(iii)* R⁷, R¹⁰, R¹¹, R¹³ , R¹⁵, R¹⁷ , R¹⁸ and R²⁶ independently of one another for each individual occurrence represent a radical selected from the group consisting of hydrogen and (C₁-C₆)-alkyl,
where (C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
and/or in which
(*iv*) R⁶ and R⁷, R⁹ and R¹⁰, R¹¹ and R¹², R¹³ and R¹⁴ , R¹⁵ and R¹⁶, R¹⁶ and R¹⁷, R¹⁸ and R¹⁹ and also R²⁵ and R²⁶ in each case as a pair together with the atoms to which they are attached may form a 5- or 6-membered heterocycloalkyl ring which may be mono- to trisubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
R² represents a substituent selected from the group consisting of halogen, cyano, nitro, (C₁-C₆)-alkyl, trifluoromethyl, hydroxyl, (C₁-C₆)-alkoxy, trifluoromethoxy, amino, hydroxycarbonyl and -C(=O)-NH-R^{7A} in which (C₁-C₆)-alkyl and (C₁-C₆)-alkoxy for their part may be substituted by hydroxyl
and
R^{7A} represents hydrogen or (C₁-C₄)-alkyl,
m represents the number 1,
n represents the number 0 or 1
and
R³ represents hydrogen,
and salts, solvates and solvates of the salts thereof.

3. Compound of the formula (I) in which
A represents CH,
R¹ represents a substituent selected from the group consisting of (C₁-C₆)-alkyl, trifluoromethyl, halogen, cyano, nitro, hydroxyl, (C₁-C₆)-alkoxy, amino, hydroxycarbonyl, (C₁-C₆)-alkoxycarbonyl and -C(=O)-NH-R⁷ in which
(C₁-C₆)-alkyl for its part may be substituted by hydroxyl, (C₁-C₄)-alkoxy, amino, mono-(C₁-C₄)-alkylamino, di-(C₁-C₄)-alkylamino or a group of the formula -NH-C(=O)-R¹², -NH-C(=O)-NH-R¹⁶ or -NH-SO₂-R¹⁹ in which
R¹², R¹⁶ and R¹⁹ each represent (C₁-C₆)-alkyl which may be substituted by hydroxyl or (C₁-C₄)-alkoxy,
and
R⁷ represents hydrogen or (C₁-C₆)-alkyl which may be substituted by hydroxyl or (C₁-C₄)-alkoxy,
R² represents a substituent selected from the group consisting of -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} and -NR^{25A}R²⁶A in which
(*i*) R^{9A}, R^{12A} , R^{14A} , R^{16A}, R^{19A} and R^{25A} independently of one another represent (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
(*ii*) R^{10A}*,* R^{11A}, R^{13A} , R^{15A}, R^{17A} , R^{18A} and R^{26A} independently of one another represent hydrogen or (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
and/or in which
*(iii)* R^{9A} and R^{10A}, R^{11A} and R^{12A}, R^{13A} and R^{14A}, R^{15A} and R^{16A}, R^{16A} and R^{17A} , R^{18A} and R^{19A} and also R^{25A} and R^{26A} in each case as a pair together with the atoms to which they are attached may form a 5- or 6-membered heterocycloalkyl ring which may be mono- or disubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
m represents the number 1,
n represents the number 1
and
R³ represents hydrogen,
and salts, solvates and solvates of the salts thereof.

4. Compound of the formula (I) in which
A represents CH,
R¹ represents (C₁-C₆)-alkyl which
(*i*) is mono- or disubstituted by identical or different radicals from the group consisting of halogen, cyano, trifluoromethyl, (C₃-C₆)-cycloalkyl, 4- to 6-membered heterocycloalkyl, hydroxycarbonyl, aminocarbonyl, -C(=O)-NR⁶R⁷, -O-C(=O)-NR⁹R¹⁰, -NR¹¹-C(=O)-R¹², ₋NR¹³-C(=O)-OR¹⁴, -NR¹⁵-C(=O)-NR¹⁶R¹⁷, -NR¹⁸-SO₂-R¹⁹, -OR²³ and -NR²⁵R²⁶
and additionally may be substituted by hydroxyl, (C₁-C₄)-alkoxy**,** amino, mono-(C₁-C₄)-alkylamino, di-(C₁-C₄)-alkylamino or (C₁-C₄)-acylamino,
or
(*ii*) is disubstituted by identical or different radicals from the group consisting of hydroxyl, (C₁-C₄)-alkoxy, amino, mono-(C₁-C₄)-alkylamino, di-(C₁-C₄)-alkylamino and (C₁-C₄)-acylamino
in which
*(a)* the abovementioned cycloalkyl and heterocycloalkyl radicals for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
(*b*) R⁷, R¹⁰, R¹¹ R¹³, R¹⁵, R¹⁷ , R¹⁸ and R²⁶ independently of one another for each individual occurrence represent a radical selected from the group consisting of hydrogen and (C₁-C₆)-alkyl,
where (C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
*(c)* R⁶, R⁹ and R¹⁴ independently of one another for each individual occurrence represent a radical selected from the group consisting of (C₁-C₆)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl
and 4- to 6-membered heterocycloalkyl,
*(d)* R¹², R¹⁶ and R¹⁹ independently of one another for each individual occurrence represent a radical selected from the group consisting of (C₁-C₆)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl is mono- to trisubstituted by identical or different radicals selected from the group consisting of halogen, cyano, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl,
*(e)* R²³ and R²⁵ independently of one another for each individual occurrence represent a radical selected from the group consisting of (C₁-C₆)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁C₄)-alkylamino, di-(C₁-C₄)-alkylamino, hydroxycarbonyl and (C₁-C₄)-alkoxycarbonyl
and
(C₁-C₆)-alkyl is mono- to trisubstituted by identical or different radicals selected from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl,
and/or in which
(*f*) R⁶ and R⁷, R⁹ and R¹⁰, R¹¹ and R¹², R¹³ and R¹⁴, R¹⁵ and R¹⁶, R¹⁶ and R¹⁷, R¹⁸ and R¹⁹ and also R²⁵ and R²⁶ in each case as a pair together with the atoms to which they are attached may form a 5- or 6-membered heterocycloalkyl ring which may be mono- to trisubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl**,**
R² represents a substituent selected from the group consisting of halogen, cyano, (C₁-C₆)-alkyl, trifluoromethyl, hydroxyl, trifluoromethoxy, amino, hydroxycarbonyl, aminocarbonyl, -C(=O)-NR^{6A}R^{7A}, -O-C(=O)-NR^{9A}R^{10A}, -NR^{11A}-C(=O)-R^{12A}, -NR¹³A-C(=O)-OR^{14A}, -NR^{15A}-C(=O)-NR^{16A}R^{17A}, -NR^{18A}-SO₂-R^{19A}, -OR^{23A} and -NR^{25A}R^{26A} in which
(*i*) (C₁-C₆)-alkyl may be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
(*ii*) R^{6A}, R^{9A}, R^{12A}, R^{14A}, R^{16A}, R^{19A}, R^{23A} and R^{25A} independently of one another represent (C₁-C₆)-alkyl which may in each case be substituted by halogen, cyano, trifluoromethyl, hydroxyl, trifluoromethoxy, (C₁-C₄)-alkoxy, amino, mono-(C₁C₄)-alkylamino, di-(C₁-C₄)-alkylamino, hydroxycarbonyl or (C₁-C₄)-alkoxycarbonyl,
(*iii*) R^{7A}, R^{10A}, R^{11A}, R^{13A}, R^{15A}, R^{17A}, R^{18A} and R^{26A} independently of one another represent hydrogen or (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl**,** and/or in which
(*iv*) R^{6A} and R^{7A}, R^{9A} and R^{10A}, R^{11A} and R^{12A}, R^{13A} and R^{14A}, R^{15A} and R^{16A}, R^{16A} and R^{17A} , R^{18A} and R^{19A} and also R^{25A} and R^{26A} in each case as a pair together with the atoms to which they are attached may form a 5- or 6-membered heterocycloalkyl ring which may be mono- or disubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
m represents the number 1,
n represents the number 0 or 1
and
R³ represents hydrogen,
and salts, solvates and solvates of the salts thereof.

5. Compound of the formula (I) in which
A represents CH,
R¹ represents (C₁-C₆)-alkoxy which is mono- or disubstituted by identical or different radicals selected from the group consisting of halogen, cyano, trifluoromethyl, (C₃-C₆)-cycloalkyl, 4- to 6-membered heterocycloalkyl, hydroxyl, amino, hydroxycarbonyl, aminocarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ and -NR²⁵R²⁶ in which
(*i*) (C₃-C₆)-cycloalkyl and 4- to 6-membered heterocycloalkyl for their part may in each case be mono- to trisubstituted by identical or different substituents from the group consisting of halogen, cyano, (C₁-C₄)-alkyl, trifluoromethyl, hydroxyl, (C₁-C₄)-alkoxy**,** trifluoromethoxy, oxo, amino, mono-(C₁-C₄)-alkylamino, di-(C₁-C₄)-alkylamino, hydroxycarbonyl and (C₁-C₄)-alkoxycarbonyl,
(*ii*) R⁶, R⁹, R¹² , R¹⁴, R¹⁶, R¹⁹, R²³ and R²⁵ independently of one another for each individual occurrence represent a radical selected from the group consisting of (C₁-C₆)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl,
(*iii*) R⁷, R¹⁰, R¹¹, R¹³ , R¹⁵, R¹⁷ , R¹⁸ and R²⁶ independently of one another for each individual occurrence represent a radical selected from the group consisting of hydrogen and (C₁-C₆)-alkyl,
where (C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
and/or in which
(*iv*) R⁶ and R⁷, R⁹ and R¹⁰, R¹¹ and R¹², R¹³ and R¹⁴, R¹⁵ and R¹⁶, R¹⁶ and R¹⁷, R¹⁸ and R¹⁹ and also R²⁵ and R²⁶ in each case as a pair together with the atoms to which they are attached may form a 5- or 6-membered heterocycloalkyl ring which may be mono- to trisubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
R² represents a substituent selected from the group consisting of halogen, cyano, (C₁-C₆)-alkyl, trifluoromethyl, hydroxyl, trifluoromethoxy, amino, hydroxycarbonyl, aminocarbonyl, -C(=O)-NR^{6A}R^{7A}, -O-C(=O)-NR^{9A}R^{10A}, -NR^{11A}-C(=O)-R^{12A}, -NR¹³A-C(=O)-OR^{14A}, -NR^{15A}-C(=O)-NR^{16A}R^{17A} -NR^{18A}-SO₂-R^{19A}, -OR^{23A} and -NR^{25A}R^{26A} in which
(*i*) (C₁-C₆)-alkyl may be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
*(ii)* R^{6A}, R^{9A}, R^{12A}, R^{14A}, R^{16A}, R^{19A}, R^{23A} and R^{25A} independently of one another represent (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
*(iii)* R^{7A}*,* R^{10A}, R^{11A}, R^{13A} , R^{15A}, R^{17A}, R^{18A} and R^{26A} independently of one another represent hydrogen or (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
and/or in which
(*iv*) R^{6A} and R^{7A}, R^{9A} and R^{10A}, R^{11A} and R^{12A}, R^{13A} and R^{14A}, R^{15A} and R^{16A}, R^{16A} and R^{17A}, R^{18A} and R^{19A} and also R^{25A} and R^{26A} in each case as a pair together with the atoms to which they are attached may form a 5- or 6-membered heterocycloalkyl ring which may be mono- or disubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
m represents the number 1,
n represents the number 0 or 1
and
R³ represents hydrogen,
and salts, solvates and solvates of the salts thereof.

6. Compound of the formula (I) in which
A represents CH,
R¹ represents the group -C(=O)-NR⁶R⁷
in which (*i*)
R⁶ represents (C₃-C₆)-cycloalkyl or 4- to 6-membered heterocyclo-alkyl which may in each case be mono- to trisubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycar-bonyl,
or
represents (C₁-C₆)-alkyl which
*(a)* is mono- or disubstituted by identical or different radicals selected from the group consisting of halogen, cyano, trifluoromethyl, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl
and may additionally be substituted by hydroxyl or (C₁-C₄)-alkoxy,
or
(*b*) is disubstituted by hydroxyl and/or (C₁-C₄)-alkoxy,
and
R⁷ represents hydrogen,
or in which (*ii*)
R⁶ represents (C₁-C₆)-alkyl, (C₃-C₆)-cycloalkyl or 4- to 6-membered heterocycloalkyl, where
(C₃-C₆)-cycloalkyl and 4- to 6-membered heterocycloalkyl for their part may in each case be substituted up to three times by identical or different substituents selected from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl
and
R⁷ represents (C₁-C₆)-alkyl which may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
or in which (*iii*)
R⁶ and R⁷ together with the nitrogen atom to which they are attached form a 5- or 6-membered heterocycloalkyl ring which may be mono- to trisubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
R² represents a substituent selected from the group consisting of halogen, cyano, (C₁-C₆)-alkyl, trifluoromethyl, hydroxyl, trifluoromethoxy, amino, hydroxycarbonyl, aminocarbonyl, -C(=O)-NR^{6A}R^{7A}, -O-C(=O)-NR^{9A}R^{10A}, -NR^{11A}-C(=O)-R^{12A}, -NR¹³A-C(=O)-OR^{14A}, -NR^{15A}-C(=O)-NR^{16A}R^{17A}, -NR¹⁸A-SO₂-R^{19A}, -OR^{23A} and -NR^{25A}R^{26A} in which
(*i*) (C₁-C₆)-alkyl may be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
(*ii*) R^{6A}, R^{9A}, R^{12A}, R^{14A}, R^{16A}, R^{19A}, R^{23A} and R^{25A} independently of one another represent (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
(*iii*) R^{7A}, R^{10A}, R^{11A}, R^{13A}, R^{15A}, R^{17A}, R^{18A} and R^{26A} independently of one another represent hydrogen or (C₁-C₆)-alkyl which may in each case be substituted by halogen, cyano, trifluoromethyl, hydroxyl, trifluoromethoxy, (C₁-C₄)-alkoxy, amino, mono-(C₁-C₄)-alkylamino, di-(C₁-C₄)-alkylamino, hydroxycarbonyl or (C₁-C₄)-alkoxycarbonyl,
and/or in which
(*iv*) R^{6A} and R^{7A}, R^{9A} and R^{10A}, R^{11A} and R^{12A}, R^{13A} and R^{14A}, R^{15A} and R^{16A}, R^{16A} and R^{17A}, R^{18A} and R^{19A} and also R^{25A} and R^{26A} in each case as a pair together with the atoms to which they are attached may form a 5- or 6-membered heterocycloalkyl ring which may be mono- or disubstituted by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
m represents the number 1,
n represents the number 0 or 1
and
R³ represents hydrogen,
and salts, solvates and solvates of the salts thereof.

7. Compound according to Claim 2 of the formula (I-A) or (I-B) 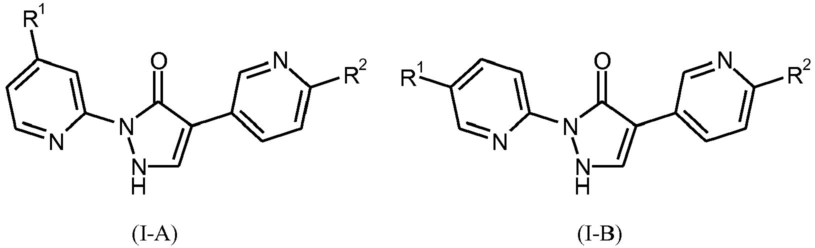 in which
R¹ represents 4- to 6-membered heterocycloalkyl, phenyl or 5- or 6-membered heteroaryl which may in each case be mono- or disubstituted by identical or different radicals selected from the group consisting of fluorine, chlorine, bromine, cyano, (C₁-C₄)-alkyl, hydroxyl, amino, hydroxycarbonyl, -OR²³ and -NR²⁵R²⁶ in which
(C₁-C₄)-alkyl for its part may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino, hydroxycarbonyl, (C₃-C₆)-cycloalkyl, 4- to 6-membered heterocycloalkyl, phenyl and 5- or 6-membered heteroaryl,
R²³ and R²⁵ independently of one another for each individual occurrence represent (C₁-C₄)-alkyl which may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino, hydroxycarbonyl, (C₃-C₆)-cycloalkyl and 4- to 6-membered heterocycloalkyl,
and
R²⁶ for each individual occurrence represents hydrogen or (C₁-C₄)-alkyl which may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino and hydroxycarbonyl,
and
R² represents hydrogen, fluorine, chlorine, cyano, (C₁-C₄)-alkyl, trifluoromethyl, hydroxymethyl, (C₁-C₄)-alkoxy, trifluoromethoxy, amino or hydroxycarbonyl,
and salts, solvates and solvates of the salts thereof.

8. Compound according to Claim 4 of the formula (I-A) or (I-B) 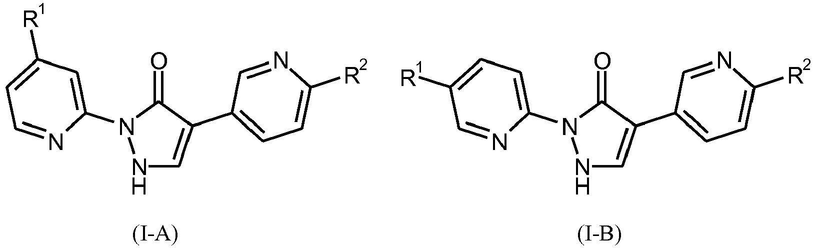 in which
R¹ represents (C₁-C₆)-alkyl which
(*i*) is mono- or disubstituted by identical or different radicals selected from the group consisting of fluorine, chlorine, cyano, trifluoromethyl, (C₃-C₆)-cycloalkyl, 4- to 6-membered heterocyclo-alkyl, hydroxycarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ and -NR²⁵R²⁶
and may additionally be substituted by hydroxyl, (C₁-C₄)-alkoxy, amino, mono-(C₁-C₄)-alkylamino, di-(C₁-C₄)-alkylamino or (C₁-C₄)-acylamino,
or
(*ii*) is disubstituted by identical or different radicals selected from the group consisting of hydroxyl, (C₁-C₄)-alkoxy, amino, mono-(C₁-C₄)-alkylamino, di-(C₁-C₄)-alkylamino and (C₁-C₄)-acylamino
in which
(*a*) the above-mentioned cycloalkyl and heterocycloalkyl radicals for their part may in each case be substituted up to three times by identical or different substituents selected from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl,
(*b*) R⁷, R¹⁰, R¹¹, R¹³, R¹⁵, R¹⁷, R¹⁸ and R²⁶ independently of one another for each individual occurrence represent a radical selected from the group consisting of hydrogen and (C₁-C₄)-alkyl,
where (C₁-C₄)-alkyl may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino and hydroxycarbonyl,
(*c*) R⁶, R⁹ and R¹⁴ independently of one another for each individual occurrence represent a radical selected from the group consisting of (C₁-C₄)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl
and
(C₁-C₄)-alkyl may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino, hydroxycarbonyl, (C₃-C₆)-cycloalkyl and 4- to 6-membered heterocycloalkyl,
*(d)* R¹², R¹⁶ and R¹⁹ independently of one another for each individual occurrence represent a radical selected from the group consisting of (C₁-C₄)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl
and
(C₁-C₄)-alkyl is mono- to trisubstituted by identical or different radicals selected from the group consisting of fluorine, chlorine, cyano, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl and 4- to 6-membered heterocycloalkyl,
*(e)* R²³ and R²⁵ independently of one another for each individual occurrence represent a radical selected from the group consisting of (C₁-C₄)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl
and
(C₁-C₄)-alkyl is mono- to trisubstituted by identical or different radicals selected from the group consisting of fluorine, chlorine, cyano, hydroxyl, (C₁-C₄)-alkoxy, amino, mono-(C₁-C₄)-alkyl-amino, di-(C₁-C₄)-alkylamino, hydroxycarbonyl, (C₃-C₆)-cyclo-alkyl and 4- to 6-membered heterocycloalkyl,
and/or in which
(*f*) R⁶ and R⁷, R⁹ and R¹⁰, R¹¹ and R¹², R¹³ and R¹⁴, R¹⁵ and R¹⁶, R¹⁶ and R¹⁷, R¹⁸ and R¹⁹ and also R²⁵ and R²⁶ in each case as a pair together with the atoms to which they are attached may form a 5- or 6-membered heterocycloalkyl ring which may be mono- to trisubstituted by identical or different substituents from the group consisting of fluorine, chlorine, cyano, (C₁-C₄)-alkyl, trifluoromethyl, hydroxyl, (C₁-C₄)-alkoxy, amino, mono-(C₁-C₄)-alkylamino, di-(C₁-C₄)-alkylamino and hydroxycarbonyl, and
R² represents hydrogen, fluorine, chlorine, cyano, (C₁-C₄)-alkyl, trifluoromethyl, hydroxymethyl, (C₁-C₄)-alkoxy, trifluoromethoxy, amino or hydroxycarbonyl,
and salts, solvates and solvates of the salts thereof.

9. Compound according to Claim 5 of the formula (I-A) or (I-B) 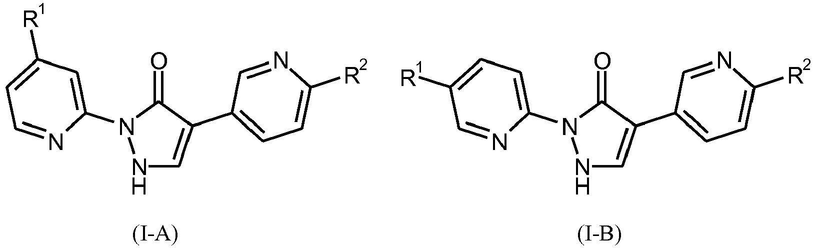 in which
R¹ represents (C₁-C₆)-alkoxy which is mono- or disubstituted by identical or different radicals selected from the group consisting of fluorine, chlorine, cyano, trifluoromethyl, (C₃-C₆)-cycloalkyl, 4- to 6-membered heterocyclo-alkyl, hydroxyl, amino, hydroxycarbonyl, -C(=O)-NR⁶R⁷, -O-C(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ and -NR²⁵R²⁶ in which
(*i*) (C₃-C₆)-cycloalkyl and 4- to 6-membered heterocycloalkyl for their part may in each case be mono- to trisubstituted by identical or different substituents from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl,
(*ii*) R⁶, R⁹, R¹², R¹⁴, R¹⁶, R¹⁹, R²³ and R²⁵ independently of one another for each individual occurrence represent a radical selected from the group consisting of (C₁-C₄)-alkyl, (C₃-C₆)-cycloalkyl and 4- to 6-membered heterocycloalkyl, where
(C₃-C₆)-cycloalkyl and 4- to 6-membered heterocycloalkyl for their part may in each case be substituted up to three times by identical or different substituents selected from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl
and
(C₁-C₄)-alkyl may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino, hydroxycarbonyl, (C₃-C₆)-cycloalkyl and 4- to 6-membered heterocycloalkyl,
(*iii*) R⁷, R¹⁰, R¹¹, R¹³, R¹⁵, R¹⁷, R¹⁸ and R²⁶ independently of one another for each individual occurrence represent a radical selected from the group consisting of hydrogen and (C₁-C₄)-alkyl,
where (C₁-C₄)-alkyl may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino and hydroxycarbonyl,
and/or in which
(*iv*) R⁶ and R⁷, R⁹ and R¹⁰, R¹¹ and R¹², R¹³ and R¹⁴, R¹⁵ and R¹⁶, R¹⁶ and R¹⁷, R¹⁸ and R¹⁹ and also R²⁵ and R²⁶ in each case as a pair together with the atoms to which they are attached may form a 5- or 6-membered heterocycloalkyl ring which may be mono- to trisubstituted by identical or different substituents from the group consisting of fluorine, chlorine, cyano, (C₁-C₄)-alkyl, trifluoromethyl, hydroxyl, (C₁-C₄)-alkoxy, amino, mono-(C₁-C₄)-alkylamino, di-(C₁-C₄)-alkylamino and hydroxycarbonyl,
and
R² represents hydrogen, fluorine, chlorine, cyano, (C₁-C₄)-alkyl, trifluoromethyl, hydroxymethyl, (C₁-C₄)-alkoxy, trifluoromethoxy, amino or hydroxycarbonyl,
and salts, solvates and solvates of the salts thereof.

10. Compound according to Claim 6 of the formula (I-A) or (I-B) 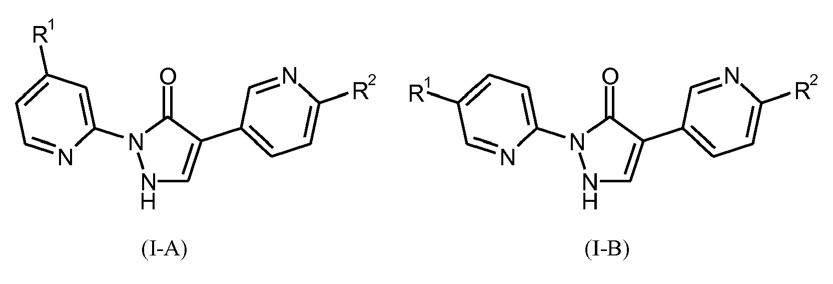 in which
R¹ represents the group -C(=O)-NR⁶R⁷
in which (i)
R⁶ represents (C₃-C₆)-cycloalkyl or 4- to 6-membered heterocycloalkyl which may in each case be mono- to trisubstituted by identical or different substituents from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl,
or
represents (C₁-C₆)-alkyl which
(*a*) is mono- or disubstituted by identical or different radicals selected from the group consisting of fluorine, chlorine, cyano, trifluoromethyl, amino, mono-(C₁-C₄)-alkylamino, di-(C₁-C₄)-alkylamino, hydroxycarbonyl, (C₃-C₆)-cycloalkyl and 4- to 6-membered heterocycloalkyl
and may additionally be substituted by hydroxyl or (C₁-C₄)-alkoxy,
or
(*b*) is disubstituted by hydroxyl and/or (C₁-C₄)-alkoxy, and
R⁷ represents hydrogen,
or in which (*ii*)
R⁶ represents (C₁-C₆)-alkyl, (C₃-C₆)-cycloalkyl or 4- to 6-membered heterocycloalkyl, where
(C₃-C₆)-cycloalkyl and 4- to 6-membered heterocycloalkyl for their part may in each case be substituted up to three times by identical or different substituents selected from the group consisting of fluorine, chlorine, cyano, (C₁-C₄)-alkyl, trifluoromethyl, hydroxyl, (C₁-C₄)-alkoxy, oxo, amino, mono-(C₁-C₄)-alkylamino, di-(C₁-C₄)-alkylamino and hydroxycarbonyl
and
(C₁-C₆)-alkyl may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino, hydroxycarbonyl, (C₃-C₆)-cycloalkyl and 4- to 6-membered heterocycloalkyl,
and
R⁷ represents (C₁-C₄)-alkyl which may be mono- to trisubstituted by identical or different substituents from the group consisting of fluorine, chlorine, cyano, hydroxyl, (C₁-C₄)-alkoxy, amino, mono-(C₁-C₄)-alkylamino, di-(C₁-C₄)-alkylamino and hydroxycarbonyl,
or in which (*iii*)
R⁶ and R⁷ together with the nitrogen atom to which they are attached form a 5- or 6-membered heterocycloalkyl ring which may be mono- to trisubstituted by identical or different substituents from the group consisting of fluorine, chlorine, cyano, (C₁-C₄)-alkyl, trifluoromethyl, hydroxyl, (C₁-C₄)-alkoxy, amino, mono-(C₁-C₄)-alkylamino, di-(C₁-C₄)-alkylamino and hydroxycarbonyl, and
R² represents hydrogen, fluorine, chlorine, cyano, (C₁-C₄)-alkyl, trifluoromethyl, hydroxymethyl, (C₁-C₄)-alkoxy, trifluoromethoxy, amino or hydroxycarbonyl,
and salts, solvates and solvates of the salts thereof.

11. Process for the preparation of a compound of the formula (I) as defined in any of Claims 1 to 10, **characterized in that** a compound of the formula (II) 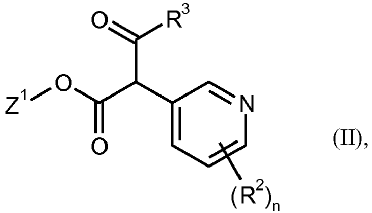 in which R², R³ and n have the meanings given in any of Claims 1 to 10 and
Z¹ represents methyl or ethyl,
is reacted in an inert solvent, if appropriate in the presence of an acid, with a compound of the formula (III) 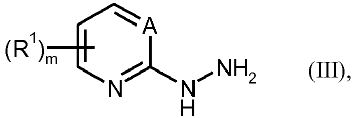 in which A, R¹ and m have the meanings given in any of Claims 1 to 10,
to give compounds of the formula (IV) 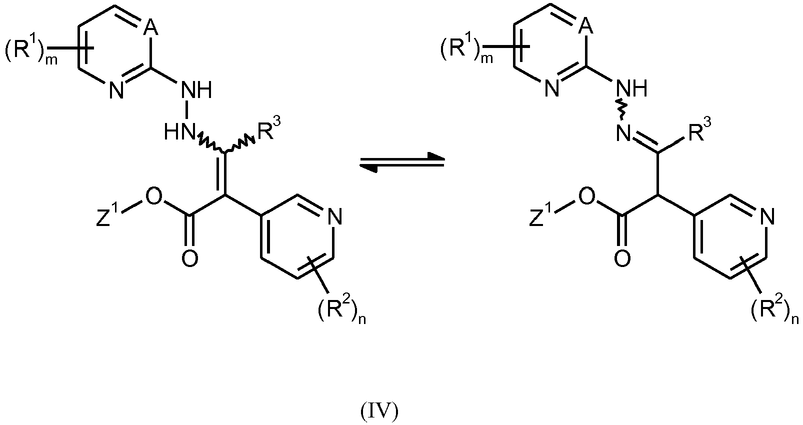 in which Z¹, A, R¹, R², R³, m and n have the meanings given above,
which, already under these reaction conditions or in a subsequent reaction step under the influence of a base, cyclize to give the compounds of the formula (I),
and the compounds of the formula (I) are, if appropriate with the appropriate (i) solvents and/or (ii) bases or acids, converted into their solvates, salts and/or solvates of the salts.

12. Process for the preparation of a compound of the formula (I) as defined in any of Claims 1 to 10 in which R³ represents hydrogen, **characterized in that** a compound of the formula (V) 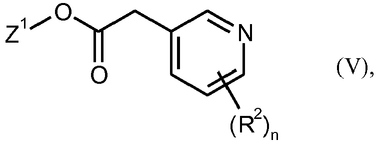 in which R² and n have the meanings given in any of Claims 1 to 10 and
Z¹ represents methyl or ethyl,
is initially condensed with a compound of the formula (VI) 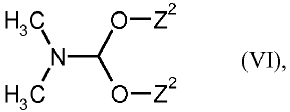 in which
Z² represents methyl or ethyl,
to give compounds of the formula (VII) 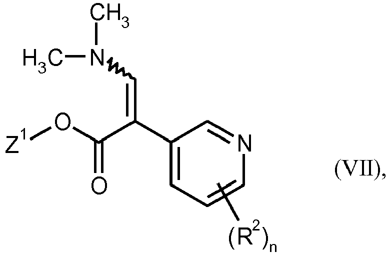 in which Z¹, R² and n have the meanings given above,
and then reacted in the presence of an acid with a compound of the formula (III) 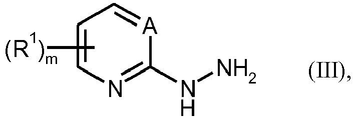 in which A, R¹ and m have the meanings given in any of Claims 1 to 10,
to give compounds of the formula (IV-A) 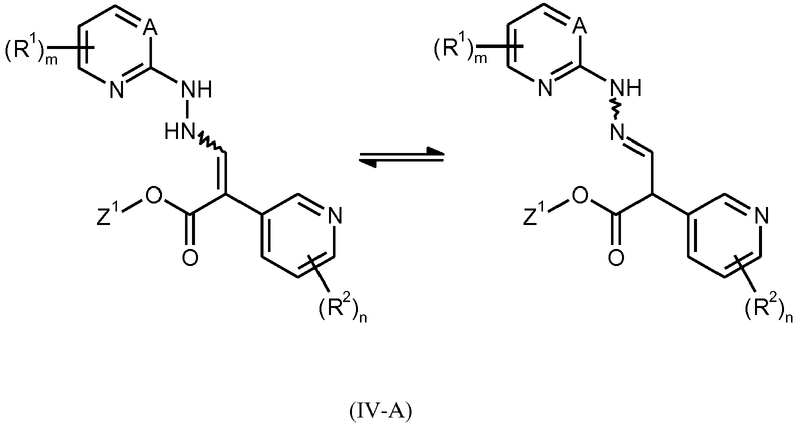 in which Z¹, A, R¹, R², m and n have the meanings given above,
which, already under these reaction conditions or in a subsequent reaction step under the influence of a base, cyclize to give the compounds of the formula (I) in which R³ represents hydrogen.

13. Compound as defined in any of Claims 1 to 10 for treatment and/or prophylaxis of diseases.

14. Use of a compound as defined in any of Claims 1 to 10 for the preparation of a medicament for treatment and/or prophylaxis of cardiovascular diseases, cardiac insufficiency, anemia, chronic kidney diseases and renal insufficiency.

15. Medicament comprising a compound as defined in any of Claims 1 to 10 in combination with an inert, non-toxic, pharmaceutically suitable auxiliary.

16. Medicament comprising a compound as defined in any of Claims 1 to 10 in combination with one or more further active compounds chosen from the group consisting of ACE inhibitors, angiotensin II receptor antagonists, beta receptor blockers, calcium antagonists, PDE inhibitors, mineralocorticoid receptor antagonists, diuretics, aspirin, iron supplements, vitamin B12 and folic acid supplements, statins, digitalis (digoxin) derivatives, tumor chemotherapeutics and antibiotics.

17. Medicament according to Claim 15 or 16 for treatment and/or prophylaxis of cardiovascular diseases, cardiac insufficiency, anemia, chronic kidney diseases and renal insufficiency.

## Revendications

1. Composé de formule (I) 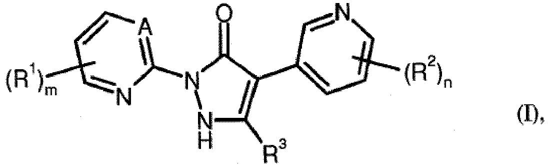 dans lequel
A représente CH,
R¹ représente un substituant choisi dans la série constituée par cycloalkyle en (C₃-C₆), hétérocycloalkyle de 4 à 6 éléments, phényle, hétéroaryle à 5 ou 6 éléments, -O-C(=O)-NR⁹R¹⁰, -NR¹¹-C(=O)-R¹², -NR¹³-(C=O)-OR¹⁴, -NR¹⁵-C(=O)-NR¹⁶R¹⁷, -NR¹⁸-SO₂-R¹⁹ et -NR²⁵R²⁶,
(i) le cycloalkyle en (C₃-C₆), l'hétérocycloalkyle de 4 à 6 éléments, le phényle et l'hétéroaryle à 5 ou 6 éléments pouvant chacun de leur côté être substitués une à trois fois, de manière identique ou différente, avec des radicaux choisis dans la série constituée par alkyle en (C₁-C₆), halogène, cyano, oxo, hydroxy, amino, hydroxycarbonyle, aminocarbonyle, -C(=O)-NR⁶R⁷, -OC(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ et -NR²⁵R²⁶,
le radical alkyle mentionné en dernier pouvant de son côté être substitué jusqu'à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆), hétérocycloalkyle de 4 à 6 éléments, phényle et/ou hétéroaryle à 5 ou 6 éléments,
(ii) R⁶, R⁹, R¹², R¹⁴, R¹⁶, R¹⁹, R²³ et R²⁵ représentant indépendamment les uns des autres à chaque occurrence individuelle un radical choisi dans la série constituée par alkyle en (C₁-C₆), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆) et/ou hétérocycloalkyle de 4 à 6 éléments,
(iii) R⁷, R¹⁰, R¹¹, R¹³, R¹⁵, R¹⁷, R¹⁸ et R²⁶ représentant indépendamment les uns des autres à chaque occurrence individuelle un radical choisi dans la série constituée par hydrogène et alkyle en (C₁-C₆),
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
et/ou
(iv) R⁶ et R⁷, R⁹ et R¹⁰, R¹¹ et R12, R¹³ et R¹⁴, R¹⁵ et R¹⁶, R¹⁶ et R¹⁷, R¹⁸ et R¹⁹, ainsi que R²⁵ et R²⁶ pouvant chacun former en paires conjointement avec les atomes auxquels ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
R² représente un substituant choisi dans la série constituée par -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} et-NR^{25A}R^{26A},
(i) R^{9A}, R^{12A}, R^{14A}, R^{16A}, R^{19A} et R^{25A} représentant indépendamment les uns des autres alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(ii) R^{10A}, R^{11A}, R^{13A}, R^{15A}, R^{17A}, R^{18A} et R^{26A} représentant indépendamment les uns des autres hydrogène ou alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
et/ou
(iii) R^{9A} et R^{10A}, R^{11A} et R^{12A}, R^{13A} et R^{14A}, R^{15A} et R^{16A}, R^{16A} et R^{17A}, R^{18A} et R^{19A}, ainsi que R^{25A} et R^{26A} pouvant chacun former en paires conjointement avec les atomes auxquels ils sont reliés un cycle hétérocycloalkyle à 5 ou 6 éléments, qui peut être substitué une ou deux fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
m représente le nombre 1,
n représente le nombre 0 ou 1,
et
R³ représente hydrogène,
ainsi que leurs sels, solvates et solvates des sels.

2. Composé de formule (I), dans lequel
A représente CH,
R¹ représente un substituant choisi dans la série constituée par cycloalkyle en (C₃-C₆), hétérocycloalkyle de 4 à 6 éléments, phényle, hétéroaryle à 5 ou 6 éléments, -O-C(=O)-NR⁹R¹⁰, -NR¹¹-C(=O)-R¹², -NR¹³-(C=O)-OR¹⁴, -NR¹⁵-C(=O)-NR¹⁶R¹⁷, -NR¹⁸-SO₂-R¹⁹ et -NR²⁵R²⁶,
(i) le cycloalkyle en (C₃-C₆), l'hétérocycloalkyle de 4 à 6 éléments, le phényle et l'hétéroaryle à 5 ou 6 éléments pouvant chacun de leur côté être substitués une à trois fois, de manière identique ou différente, avec des radicaux choisis dans la série constituée par alkyle en (C₁-C₆), halogène, cyano, oxo, hydroxy, amino, hydroxycarbonyle, aminocarbonyle, -C(=O)-NR⁶R⁷, -OC(=O)-NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ et -NR²⁵R²⁶,
le radical alkyle mentionné en dernier pouvant de son côté être substitué jusqu'à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₁-C₆), hétérocycloalkyle de 4 à 6 éléments, phényle et/ou hétéroaryle à 5 ou 6 éléments,
(ii) R⁶, R⁹, R¹², R¹⁴, R¹⁶, R¹⁹, R²³ et R²⁵ représentant indépendamment les uns des autres à chaque occurrence individuelle un radical choisi dans la série constituée par alkyle en (C₁-C₆), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆) et/ou hétérocycloalkyle de 4 à 6 éléments,
(iii) R⁷, R¹⁰, R¹¹, R¹³, R¹⁵, R¹⁷, R¹⁸ et R²⁶ représentant indépendamment les uns des autres à chaque occurrence individuelle un radical choisi dans la série constituée par hydrogène et alkyle en (C₁-C₆),
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
et/ou
(iv) R⁶ et R⁷, R⁹ et R¹⁰, R¹¹ et R¹², R¹³ et R¹⁴, R¹⁵ et R¹⁶, R¹⁶ et R¹⁷, R¹⁸ et R¹⁹, ainsi que R²⁵ et R²⁶ pouvant chacun former en paires conjointement avec les atomes auxquels ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
R² représente un substituant choisi dans la série constituée par halogène, cyano, nitro, alkyle en (C₁-C₆), trifluorométhyle, hydroxy, alcoxy en (C₁-C₆), trifluorométhoxy, amino, hydroxycarbonyle et -C(=O)-NH-R^{7A}, l'alkyle en (C₁-C₆) et l'alcoxy en (C₁-C₆) pouvant de leur côté être substitués avec hydroxy,
et
R^{7A} signifiant hydrogène ou alkyle en (C₁-C₄),
m représente le nombre 1,
n représente le nombre 0 ou 1,
et
R³ représente hydrogène,
ainsi que leurs sels, solvates et solvates des sels.

3. Composé de formule (I), dans lequel
A représente CH,
R¹ représente un substituant choisi dans la série constituée par alkyle en (C₁-C₆), trifluorométhyle, halogène, cyano, nitro, hydroxy, alcoxy en (C₁-C₆), amino, hydroxycarbonyle, alcoxycarbonyle en (C₁-C₆) et - C(=O)-NH-R⁷,
l'alkyle en (C₁-C₆) pouvant de son côté être substitué avec hydroxy, alcoxy en (C₁-C₄), amino, mono-alkylamino en (C₁-C₄), di-alkylamino en (C₁-C₄) ou un groupe de formule -NH-C(=O)-R¹², -NH-C(=O)-NH-R¹⁶ ou -NH-SO₂-R¹⁹, R¹², R¹⁶ et R¹⁹ signifiant chacun alkyle en (C₁-C₆), qui peut être substitué avec hydroxy ou alcoxy en (C₁-C₄), et
R⁷ signifiant hydrogène ou alkyle en (C₁-C₆), qui peut être substitué avec hydroxy ou alcoxy en (C₁-C₄),
R² représente un substituant choisi dans la série constituée par -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A} et-NR^{25A}R^{26A},
(i) R^{9A}, R^{12A}, R^{14A}, R^{16A}, R^{19A} et R^{25A} représentant indépendamment les uns des autres alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(ii) R^{10A}, R^{11A}, R^{13A}, R^{15A}, R^{17A}, R^{18A} et R^{26A} représentant indépendamment les uns des autres hydrogène ou alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
et/ou
(iii) R^{9A} et R^{10A}, R^{11A} et R^{12A}, R^{13A} et R^{14A}, R^{15A} et R^{16A}, R^{16A} et R^{17A}, R^{18A} et R^{19A}, ainsi que R^{25A} et R^{26A} pouvant chacun former en paires conjointement avec les atomes auxquels ils sont reliés un cycle hétérocycloalkyle à 5 ou 6 éléments, qui peut être substitué une ou deux fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
m représente le nombre 1,
n représente le nombre 1,
et
R³ représente hydrogène,
ainsi que leurs sels, solvates et solvates des sels.

4. Composé de formule (I), dans lequel
A représente CH,
R¹ représente alkyle en (C₁-C₆), qui
(i) est substitué une ou deux fois, de manière identique ou différente, avec un radical choisi dans la série constituée par halogène, cyano, trifluorométhyle, cycloalkyle en (C₃-C₆), hétérocycloalkyle de 4 à 6 éléments, hydroxycarbonyle, aminocarbonyle, -C(=O)-NR⁶R⁷, -O-C(=O)-NR⁹R¹⁰, -NR¹¹-C(=O)-R¹², -NR¹³-C(=O)-OR¹⁴, - NR¹⁵-C(=O)-NR¹⁶R¹⁷, -NR¹⁸-SO₂-R¹⁹, -OR²³ et -NR²⁵R²⁶,
et peut également être substitué avec hydroxy, alcoxy en (C₁-C₄), amino, mono-alkylamino en (C₁-C₄), di-alkylamino en (C₁-C₄) ou acylamino en (C₁-C₄),
ou
(ii) est substitué deux fois, de manière identique ou différente, avec un radical choisi dans la série constituée par hydroxy, alcoxy en (C₁-C₄), amino, mono-alkylamino en (C₁-C₄), di-alkylamino en (C₁-C₄) et acylamino en (C₁-C₄),
(a) les radicaux cycloalkyle et hétérocycloalkyle susmentionnés pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
(b) R⁷, R¹⁰, R¹¹, R¹³, R¹⁵, R¹⁷, R¹⁸ et R²⁶ représentant indépendamment les uns des autres à chaque occurrence individuelle un radical choisi dans la série constituée par hydrogène et alkyle en (C₁-C₆),
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
(c) R⁶, R⁹ et R¹⁴ représentant indépendamment les uns des autres à chaque occurrence individuelle un radical choisi dans la série constituée par alkyle en (C₁-C₆), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆) et/ou hétérocycloalkyle de 4 à 6 éléments,
(d) R¹², R¹⁶ et R¹⁹ représentant indépendamment les uns des autres à chaque occurrence individuelle un radical choisi dans la série constituée par alkyle en (C₁-C₆), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec un radical choisi dans la série constituée par halogène, cyano, amino, mono-alkylamino en (C₁-C₄), di-alkylamino en (C₁-C₄), hydroxycarbonyle, alcoxycarbonyle en (C₁-C₄), cycloalkyle en (C₃-C₆) et hétérocycloalkyle de 4 à 6 éléments,
(e) R²³ et R²⁵ représentant indépendamment l'un de l'autre à chaque occurrence individuelle un radical choisi dans la série constituée par alkyle en (C₁-C₆), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec un radical choisi dans la série constituée par halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆) et hétérocycloalkyle de 4 à 6 éléments,
et/ou
(f) R⁶ et R⁷, R⁹ et R¹⁰, R¹¹ et R¹², R¹³ et R¹⁴, R¹⁵ et R¹⁶, R¹⁶ et R¹⁷, R¹⁸ et R¹⁹, ainsi que R²⁵ et R²⁶ pouvant chacun former en paires conjointement avec les atomes auxquels ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
R² représente un substituant choisi dans la série constituée par halogène, cyano, alkyle en (C₁-C₆), trifluorométhyle, hydroxy, trifluorométhoxy, amino, hydroxycarbonyle, aminocarbonyle, -C(=O)-NR^{6A}R^{7A}, -O-C(=O)-NR^{9A}R^{10A}, -NR^{11A}-C(=O)-R^{12A}, -NR^{13A}-C(=O)-OR^{14A}, -NR^{15A}-C(=O)-NR^{16A}R^{17A}, -NR^{18A}-SO₂-R^{19A}, -OR^{23A} et -NR^{25A}R^{26A},
(i) l'alkyle en (C₁-C₆) pouvant être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(ii) R^{6A}, R^{9A}, R^{12A}, R^{14A}, R^{16A}, R^{19A}, R^{23A} et R^{25A} représentant indépendamment les uns des autres alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(iii) R^{7A}, R^{10A}, R^{11A}, R^{13A}, R^{15A}, R^{17A}, R^{18A} et R^{26A} représentant indépendamment les uns des autres hydrogène ou alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄) , hydroxycarbonyle ou alcoxycarbonyle en (C₁-C₄),
et/ou
(iv) R^{6A} et R^{7A}, R^{9A} et R^{10A}, R^{11A} et R^{12A}, R^{13A} et R^{14A}, R^{15A} et R^{16A}, R^{16A} et R^{17A} , R^{18A} et R^{19A}, ainsi que R^{25A} et R^{26A} pouvant chacun former en paires conjointement avec les atomes auxquels ils sont reliés un cycle hétérocycloalkyle à 5 ou 6 éléments, qui peut être substitué une ou deux fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
m représente le nombre 1,
n représente le nombre 0 ou 1,
et
R³ représente hydrogène,
ainsi que leurs sels, solvates et solvates des sels.

5. Composé de formule (I), dans lequel
A représente CH,
R¹ représente alcoxy en (C₁-C₆), qui est substitué une ou deux fois, de manière identique ou différente, avec un radical choisi dans la série constituée par halogène, cyano, trifluorométhyle, cycloalkyle en (C₃-C₆), hétérocycloalkyle de 4 à 6 éléments, hydroxy, amino, hydroxycarbonyle, aminocarbonyle, -C(=O)-NR⁶R⁷,-O-C (=O) -NR⁹R¹⁰, -NR¹¹-C (=O)-R¹², -NR¹³-C (=O)-OR¹⁴, -NR¹⁵-C (=O) -NR¹⁶R¹⁷, -NR¹⁸-SO₂-R¹⁹, -OR²³ et -NR²⁵R²⁶,
(i) le cycloalkyle en (C₃-C₆) et l'hétérocycloalkyle de 4 à 6 éléments pouvant chacun de leur côté être substitués une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
(ii) R⁶, R⁹, R¹², R¹⁴, R¹⁶, R¹⁹, R²³ et R²⁵ représentant indépendamment les uns des autres à chaque occurrence individuelle un radical choisi dans la série constituée par alkyle en (C₁-C₆), cycloalkyl en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄) , amino, mono-alkylamino en (C₁-C₄), di-alkylamino en (C₁-C₄), hydroxycarbonyle, alcoxycarbonyle en (C₁-C₄), cycloalkyle en (C₃-C₆) et/ou hétérocycloalkyle de 4 à 6 éléments,
(iii) R⁷, R¹⁰, R¹¹, R¹³, R¹⁵, R¹⁷, R¹⁸ et R²⁶ représentant indépendamment les uns des autres à chaque occurrence individuelle un radical choisi dans la série constituée par hydrogène et alkyle en (C₁-C₆),
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
et/ou
(iv) R⁶ et R⁷, R⁹ et R¹⁰, R¹¹ et R¹², R¹³ et R¹⁴, R¹⁵ et R¹⁶, R¹⁶ et R¹⁷, R¹⁸ et R¹⁹, ainsi que R²⁵ et R²⁶ pouvant chacun former en paires conjointement avec les atomes auxquels ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
R² représente un substituant choisi dans la série constituée par halogène, cyano, alkyle en (C₁-C₆), trifluorométhyle, hydroxy, trifluorométhoxy, amino, hydroxycarbonyle, aminocarbonyle, -C (=O) -NR^{6A}R^{7A}, -O-C (=O) -NR^{9A}R^{10A}, -NR^{11A}-C (=O)-R^{12A}, -NR^{13A}-C (=O) -OR^{14A}, -NR^{15A}-C (=O) -NR^{16A}R^{17A}, -NR^{18A}-SO₂-R^{19A}, -OR^{23A} et -NR^{25A}R^{26A},
(i) l'alkyle en (C₁-C₆) pouvant être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(ii) R^{6A}, R^{9A}, R^{12A}, R^{14A}, R^{16A}, R^{19A}, R^{23A} et R^{25A} représentant indépendamment les uns des autres alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(iii) R^{7A}, R^{10A}, R^{11A}, R^{13A}, R^{15A}, R^{17A}, R^{18A} et R^{26A} représentant indépendamment les uns des autres hydrogène ou alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
et/ou
(iv) R^{6A} et R^{7A}, R^{9A} et R^{10A}, R^{11A} et R^{12A}, R^{13A} et R^{14A}, R^{15A} et R^{16A}, R^{16A} et R^{17A}, R^{18A} et R^{19A}, ainsi que R^{25A} et R^{26A} pouvant chacun former en paires conjointement avec les atomes auxquels ils sont reliés un cycle hétérocycloalkyle à 5 ou 6 éléments, qui peut être substitué une ou deux fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
m représente le nombre 1,
n représente le nombre 0 ou 1,
et
R³ représente hydrogène,
ainsi que leurs sels, solvates et solvates des sels.

6. Composé de formule (I), dans lequel
A représente CH,
R¹ représente le groupe -C(=O)-NR⁶R⁷,
dans lequel (i)
R⁶ signifie cycloalkyle en (C₃-C₆) ou hétérocycloalkyle de 4 à 6 éléments, qui peuvent chacun être substitués une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
ou
signifie alkyle en (C₁-C₆), qui
(a) est substitué une ou deux fois, de manière identique ou différente, avec un radical choisi dans la série constituée par halogène, cyano, trifluorométhyle, amino, mono-alkylamino en (C₁-C₄), di-alkylamino en (C₁-C₄), hydroxycarbonyle, alcoxycarbonyle en (C₁-C₄), cycloalkyle en (C₃-C₆) et hétérocycloalkyle de 4 à 6 éléments,
et peut par ailleurs être substitué avec hydroxy ou alcoxy en (C₁-C₄),
ou
(b) est substitué deux fois avec hydroxy et/ou alcoxy en (C₁-C₄),
et
R⁷ signifie hydrogène,
ou (ii)
R⁶ signifie alkyle en (C₁-C₆), cycloalkyle en (C₃-C₆) ou hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆) et/ou hétérocycloalkyle de 4 à 6 éléments,
et
R⁷ signifie alkyle en (C₁-C₆), qui peut être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
ou (iii)
R⁶ et R⁷ forment ensemble avec l'atome d'azote auquel ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
R² représente un substituant choisi dans la série constituée par halogène, cyano, alkyle en (C₁-C₆), trifluorométhyle, hydroxy, trifluorométhoxy, amino, hydroxycarbonyle, aminocarbonyle, -C (=O) -NR^{6A}R^{7A}, -O-C (=O) -NR^{9A}R^{10A}, -NR^{11A}-C (=O)-R^{12A}, -NR^{13A}-C (=O) -OR^{14A}, -NR^{15A}-C (=O) -NR^{16A}R^{17A}, -NR^{18A}-SO₂-R^{19A}, -OR^{23A} et -NR^{25A}R^{26A},
(i) l'alkyle en (C₁-C₆) pouvant être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(ii) R^{6A}, R^{9A}, R^{12A}, R^{14A}, R^{16A}, R^{19A}, R^{23A} et R^{25A} représentant indépendamment les uns des autres alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄),
(iii) R^{7A}, R^{10A}, R^{11A}, R^{13A}, R^{15A}, R^{17A}, R^{18A} et R^{26A} représentant indépendamment les uns des autres hydrogène ou alkyle en (C₁-C₆), qui peut à chaque fois être substitué avec halogène, cyano, trifluorométhyle, hydroxy, trifluorométhoxy, alcoxy en (C₁-C₄), amino, mono-alkylamino en (C₁-C₄), di-alkylamino en (C₁-C₄), hydroxycarbonyle ou alcoxycarbonyle en (C₁-C₄), et/ou
(iv) R^{6A} et R^{7A}, R^{9A} et R^{10A}, R^{11A} et R^{12A}, R^{13A} et R^{14A}, R^{15A} et R^{16A}, R^{16A} et R^{17A}, R^{18A} et R^{19A}, ainsi que R^{25A} et R^{26A} pouvant chacun former en paires conjointement avec les atomes auxquels ils sont reliés un cycle hétérocycloalkyle à 5 ou 6 éléments, qui peut être substitué une ou deux fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
m représente le nombre 1,
n représente le nombre 0 ou 1,
et
R³ représente hydrogène,
ainsi que leurs sels, solvates et solvates des sels.

7. Composé selon la revendication 2, de formule (I-A) ou (I-B) 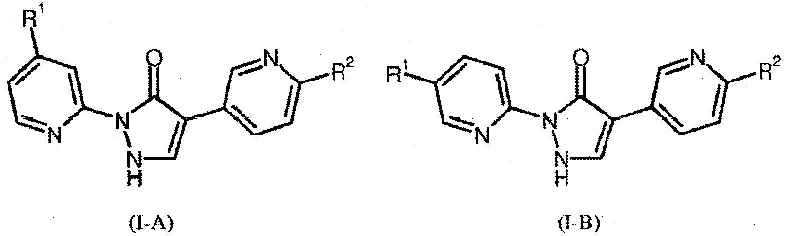 dans lesquelles
R¹ représente hétérocycloalkyle de 4 à 6 éléments, phényle ou hétéroaryle à 5 ou 6 éléments, qui peuvent chacun être substitués une ou deux fois, de manière identique ou différente, avec un radical choisi dans la série constituée par fluor, chlore, brome, cyano, alkyle en (C₁-C₄), hydroxy, amino, hydroxycarbonyle,-OR²³ et -NR²⁵R²⁶,
l'alkyle en (C₁-C₄) pouvant être substitué de son côté une à trois fois, de manière identique ou différente, avec fluor, chlore, cyano, hydroxy, alcoxy en (C₁-C₄), amino, mono-alkylamino en (C₁-C₄), di-alkylamino en (C₁-C₄), hydroxycarbonyle, cycloalkyle en (C₃-C₆), hétérocycloalkyle de 4 à 6 éléments, phényle et/ou hétéroaryle à 5 ou 6 éléments,
R²³ et R²⁵ représentant indépendamment l'un de l'autre à chaque occurrence individuelle alkyle en (C₁-C₄), qui peut être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄), hydroxycarbonyle, cycloalkyle en (C₃-C₆) et/ou hétérocycloalkyle de 4 à 6 éléments,
et
R²⁶ représentant à chaque occurrence individuelle hydrogène ou alkyle en (C₁-C₄), qui peut être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄) et/ou hydroxycarbonyle,
et
R² représente hydrogène, fluor, chlore, cyano, alkyle en (C₁-C₄), trifluorométhyle, hydroxyméthyle, alcoxy en (C₁-C₄), trifluorométhoxy, amino ou hydroxycarbonyle, ainsi que leurs sels, solvates et solvates des sels.

8. Composé selon la revendication 4, de formule (I-a) ou (I-B) 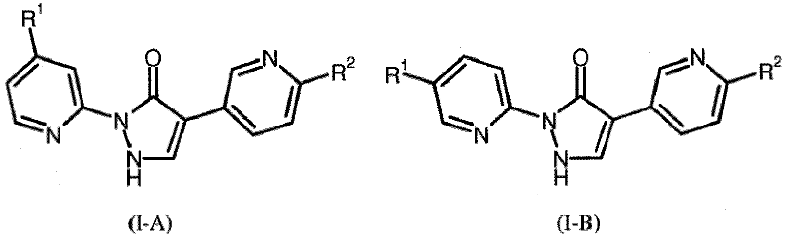 dans lesquelles
R¹ représente alkyle en (C₁-C₆), qui
(i) est substitué une ou deux fois, de manière identique ou différente, avec un radical choisi dans la série constituée par fluor, chlore, cyano, trifluorométhyle, cycloalkyle en (C₃-C₆), hétérocycloalkyle de 4 à 6 éléments, hydroxycarbonyle, -C (=O) -NR⁶R⁷, -O-C (=O) -NR⁹R¹⁰, -NR¹¹-C (=O) -R¹², -NR¹³-C(=O)-OR¹⁴, -NR¹⁵-C (=O) -NR¹⁶R¹⁷, -NR¹⁸-SO₂-R¹⁹, -OR²³ et-NR²⁵R²⁶,
et peut également être substitué avec hydroxy, alcoxy en (C₁-C₄), amino, mono-alkylamino en (C₁-C₄), di-alkylamino en (C₁-C₄) ou acylamino en (C₁-C₄),
ou
(ii) est substitué deux fois, de manière identique ou différente, avec un radical choisi dans la série constituée par hydroxy, alcoxy en (C₁-C₄), amino, mono-alkylamino en (C₁-C₄), di-alkylamino en (C₁-C₄) et acylamino en (C₁-C₄),
(a) les radicaux cycloalkyle et hétérocycloalkyle susmentionnés pouvant chacun de leur côté être substitués jusqu'à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
(b) R⁷, R¹⁰, R¹¹, R¹³, R¹⁵, R¹⁷, R¹⁸ et R²⁶ représentant indépendamment les uns des autres à chaque occurrence individuelle un radical choisi dans la série constituée par hydrogène et alkyle en (C₁-C₄), l'alkyle en (C₁-C₄) pouvant être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄) et/ou hydroxycarbonyle,
(c) R⁶, R⁹ et R¹⁴ représentant indépendamment les uns des autres à chaque occurrence individuelle un radical choisi dans la série constituée par alkyle en (C₁-C₄), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
et
l'alkyle en (C₁-C₄) pouvant être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄), hydroxycarbonyle, cycloalkyle en (C₃-C₆) et/ou hétérocycloalkyle de 4 à 6 éléments,
(d) R¹², R¹⁶ et R¹⁹ représentant indépendamment les uns des autres à chaque occurrence individuelle un radical choisi dans la série constituée par alkyle en (C₁-C₄), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
et
l'alkyle en (C₁-C₄) pouvant être substitué une à trois fois, de manière identique ou différente, avec un radical choisi dans la série constituée par fluor, chlore, cyano, amino, mono-alkylamino en (C₁-C₄), di-alkylamino en (C₁-C₄), hydroxycarbonyle, alcoxycarbonyle en (C₁-C₄), cycloalkyle en (C₃-C₆) et hétérocycloalkyle de 4 à 6 éléments,
(e) R²³ et R²⁵ représentant indépendamment l'un de l'autre à chaque occurrence individuelle un radical choisi dans la série constituée par alkyle en (C₁-C₄), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
et
l'alkyle en (C₁-C₄) pouvant être substitué une à trois fois, de manière identique ou différente, avec un radical choisi dans la série constituée par fluor, chlore, cyano, hydroxy, alcoxy en (C₁-C₄), amino, mono-alkylamino en (C₁-C₄), di-alkylamino en (C₁-C₄), hydroxycarbonyle, cycloalkyle en (C₃-C₆) et hétérocycloalkyle de 4 à 6 éléments,
et/ou
(f) R⁶ et R⁷, R⁹ et R¹⁰, R¹¹ et R¹², R¹³ et R¹⁴, R¹⁵ et R¹⁶, R¹⁶ et R¹⁷, R¹⁸ et R¹⁹, ainsi que R²⁵ et R²⁶ pouvant chacun former en paires conjointement avec les atomes auxquels ils sont reliés un cycle hétérocycloalkyle à 5 ou 6 éléments, qui peut être substitué une à trois fois, de manière identique ou différente, avec fluor, chlore, cyano, alkyle en (C₁-C₄), trifluorométhyle, hydroxy, alcoxy en (C₁-C₄), amino, mono-alkylamino en (C₁-C₄), di-alkylamino en (C₁-C₄) et/ou hydroxycarbonyle,
et
R² représente hydrogène, fluor, chlore, cyano, alkyle en (C₁-C₄), trifluorométhyle, hydroxyméthyle, alcoxy en (C₁-C₄), trifluorométhoxy, amino ou hydroxycarbonyle, ainsi que leurs sels, solvates et solvates des sels.

9. Composé selon la revendication 5, de formule (I-A) ou (I-B) 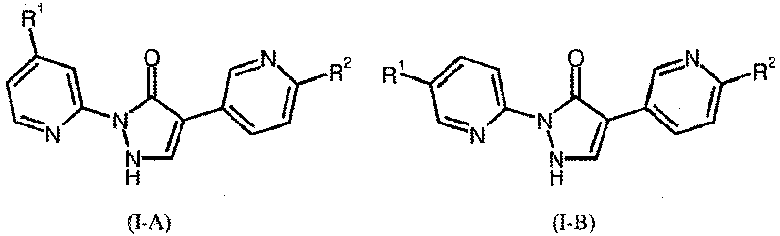 dans lesquelles
R¹ représente alcoxy en (C₁-C₆), qui est substitué une ou deux fois, de manière identique ou différente, avec un radical choisi dans la série constituée par fluor, chlore, cyano, trifluorométhyle, cycloalkyle en (C₃-C₆), hétérocycloalkyle de 4 à 6 éléments, hydroxy, amino, hydroxycarbonyle, -C (=O) -NR⁶R⁷, -O-C (=O) -NR⁹R¹⁰, -NR¹¹-C(=O)-R¹², -NR¹³-C(=O)-OR¹⁴, -NR¹⁵-C(=O)-NR¹⁶R¹⁷, -NR¹⁸-SO₂-R¹⁹, -OR²³ et -NR²⁵R²⁶,
(i) le cycloalkyle en (C₃-C₆) et l'hétérocycloalkyle de 4 à 6 éléments pouvant chacun de leur côté être substitués une à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
(ii) R⁶, R⁹, R¹², R¹⁴, R¹⁶, R¹⁹, R²³ et R²⁵ représentant indépendamment les uns des autres à chaque occurrence individuelle un radical choisi dans la série constituée par alkyle en (C₁-C₄), cycloalkyle en (C₃-C₆) et hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
et
l'alkyle en (C₁-C₄) pouvant être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄), hydroxycarbonyle, cycloalkyle en (C₃-C₆) et/ou hétérocycloalkyle de 4 à 6 éléments,
(iii) R⁷, R¹⁰, R¹¹, R¹³, R¹⁵, R¹⁷, R¹⁸ et R²⁶ représentant indépendamment les uns des autres à chaque occurrence individuelle un radical choisi dans la série constituée par hydrogène et alkyle en (C₁-C₄),
l'alkyle en (C₁-C₄) pouvant être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄) et/ou hydroxycarbonyle,
et/ou
(iv) R⁶ et R⁷, R⁹ et R¹⁰, R¹¹ et R¹², R¹³ et R¹⁴, R¹⁵ et R¹⁶, R¹⁶ et R¹⁷, R¹⁸ et R¹⁹, ainsi que R²⁵ et R²⁶ pouvant chacun former en paires conjointement avec les atomes auxquels ils sont reliés un cycle hétérocycloalkyle à 5 ou 6 éléments, qui peut être substitué une à trois fois, de manière identique ou différente, avec fluor, chlore, cyano, alkyle en (C₁-C₄), trifluorométhyle, hydroxy, alcoxy en (C₁-C₄), amino, mono-alkylamino en (C₁-C₄), di-alkylamino en (C₁-C₄) et/ou hydroxycarbonyle,
et
R² représente hydrogène, fluor, chlore, cyano, alkyle en (C₁-C₄), trifluorométhyle, hydroxyméthyle, alcoxy en (C₁-C₄), trifluorométhoxy, amino ou hydroxycarbonyle, ainsi que leurs sels, solvates et solvates des sels.

10. Composé selon la revendication 6, de formule (I-A) ou (I-B) 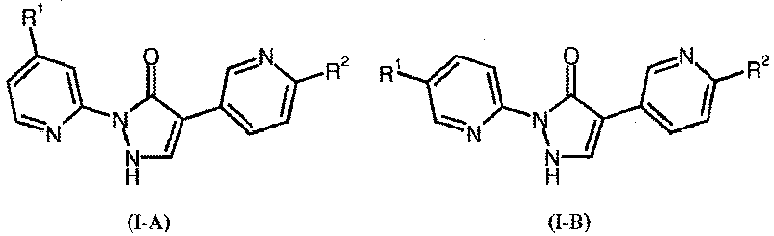 dans lesquelles
R¹ représente le groupe -C(=O)-NR⁶R⁷,
dans lequel (i)
R⁶ signifie cycloalkyle en (C₃-C₆) ou hétérocycloalkyle de 4 à 6 éléments, qui peuvent chacun être substitués une à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
ou
signifie alkyle en (C₁-C₆), qui
(a) est substitué une ou deux fois, de manière identique ou différente, avec un radical choisi dans la série constituée par fluor, chlore, cyano, trifluorométhyle, amino, mono-alkylamino en (C₁-C₄), di-alkylamino en (C₁-C₄), hydroxycarbonyle, cycloalkyle en (C₃-C₆) et hétérocycloalkyle de 4 à 6 éléments, et peut par ailleurs être substitué avec hydroxy ou alcoxy en (C₁-C₄),
ou
(b) est substitué deux fois avec hydroxy et/ou alcoxy en (C₁-C₄),
et
R⁷ signifie hydrogène,
ou (ii)
R⁶ signifie alkyle en (C₁-C₆), cycloalkyle en (C₃-C₆) ou hétérocycloalkyle de 4 à 6 éléments,
le cycloalkyle en (C₃-C₆) et l'hétérocycloalkyle de 4 à 6 éléments pouvant chacun de leur côté être substitués jusqu'à trois fois, de manière identique ou différente, avec fluor, chlore, cyano, alkyle en (C₁-C₄), trifluorométhyle, hydroxy, alcoxy en (C₁-C₄), oxo, amino, mono-alkylamino en (C₁-C₄), di-alkylamino en (C₁-C₄) et/ou hydroxycarbonyle,
et
l'alkyle en (C₁-C₆) pouvant être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄), hydroxycarbonyle, cycloalkyle en (C₃-C₆) et/ou hétérocycloalkyle de 4 à 6 éléments,
et
R⁷ signifie alkyle en (C₁-C₄), qui peut être substitué une à trois fois, de manière identique ou différente, avec fluor, chlore, cyano, hydroxy, alcoxy en (C₁-C₄), amino, mono-alkylamino en (C₁-C₄), di-alkylamino en (C₁-C₄) et/ou hydroxycarbonyle,
ou (iii)
R⁶ et R⁷ forment ensemble avec l'atome d'azote auquel ils sont reliés un cycle hétérocycloalkyle à 5 ou 6 éléments, qui peut être substitué une à trois fois, de manière identique ou différente, avec fluor, chlore, cyano, alkyle en (C₁-C₄), trifluorométhyle, hydroxy, alcoxy en (C₁-C₄), amino, mono-alkylamino en (C₁-C₄), di-alkylamino en (C₁-C₄) et/ou hydroxycarbonyle,
et
R² représente hydrogène, fluor, chlore, cyano, alkyle en (C₁-C₄), trifluorométhyle, hydroxyméthyle, alcoxy en (C₁-C₄), trifluorométhoxy, amino ou hydroxycarbonyle, ainsi que leurs sels, solvates et solvates des sels.

11. Procédé de fabrication d'un composé de formule (I), tel que défini dans les revendications 1 à 10, **caractérisé en ce qu'**un composé de formule (II) 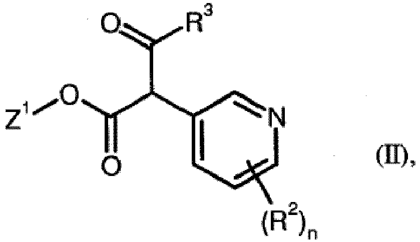
dans laquelle R², R³ et n ont les significations indiquées dans les revendications 1 à 10, et
Z¹ représente méthyle ou éthyle,
est mis en réaction dans un solvant inerte, éventuellement en présence d'un acide, avec un composé de formule (III) 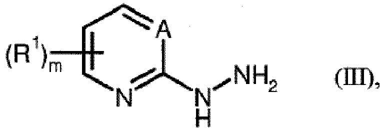 dans laquelle A, R¹ et m ont les significations indiquées dans les revendications 1 à 10,
pour former des composés de formule (IV) 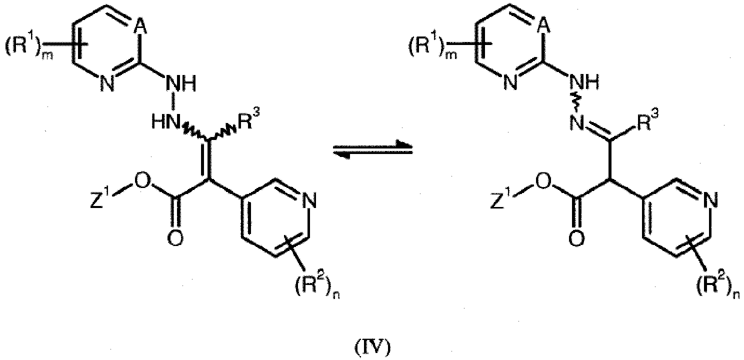 dans laquelle Z¹, A, R¹, R², R³, m et n ont les significations indiquées précédemment,
qui sont cyclisés déjà dans ces conditions de réaction ou lors d'une étape de réaction ultérieure sous l'effet d'une base en les composés de formule (I),
et les composés de formule (I) sont éventuellement transformés avec (i) les solvants et/ou (ii) les bases ou les acides appropriés en leurs solvates, sels et/ou solvates des sels.

12. Procédé de fabrication d'un composé de formule (I), tel que défini dans les revendications 1 à 10, dans lequel R³ représente l'hydrogène, **caractérisé en ce qu'**un composé de formule (V) 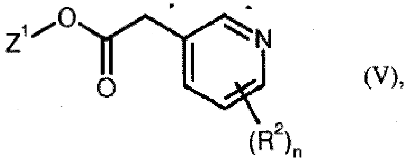
dans laquelle R² et n ont les significations indiquées dans les revendications 1 à 10, et
Z¹ représente méthyle ou éthyle,
est tout d'abord condensé avec un composé de formule (VI) 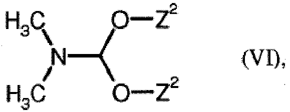 dans laquelle
Z² représente méthyle ou éthyle,
pour former des composés de formule (VII) 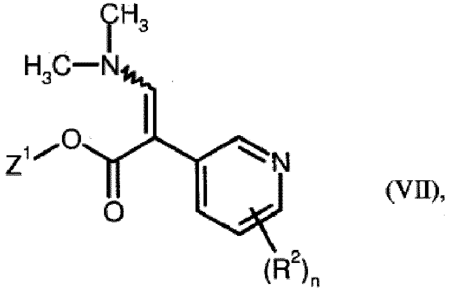 dans laquelle Z¹, R² et n ont les significations indiquées précédemment,
puis mis en réaction en présence d'un acide avec un composé de formule (III) 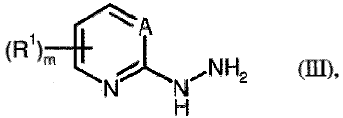 dans laquelle A, R¹ et m ont les significations indiquées dans les revendications 1 à 10,
pour former des composés de formule (IV-A) 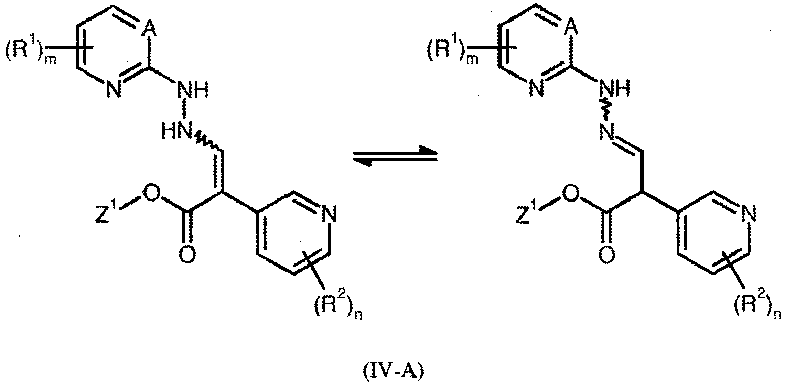 dans laquelle Z¹, A, R¹, R², m et n ont les significations indiquées précédemment,
qui sont cyclisés déjà dans ces conditions de réaction ou lors d'une étape de réaction ultérieure sous l'effet d'une base en les composés de formule (I) dans lesquels R³ représente l'hydrogène.

13. Composé, tel que défini dans l'une quelconque des revendications 1 à 10, pour le traitement et/ou la prophylaxie de maladies.

14. Utilisation d'un composé, tel que défini dans l'une quelconque des revendications 1 à 10, pour la fabrication d'un médicament pour le traitement et/ou la prophylaxie de maladies cardiovasculaires, de l'insuffisance cardiaque, de l'anémie, de l'insuffisance rénale chronique et de l'insuffisance rénale.

15. Médicament contenant un composé, tel que défini dans l'une quelconque des revendications 1 à 10, en combinaison avec un adjuvant inerte, non toxique, pharmaceutiquement approprié.

16. Médicament contenant un composé, tel que défini dans l'une quelconque des revendications 1 à 10, en combinaison avec un ou plusieurs agents actifs supplémentaires choisis dans le groupe constitué par les inhibiteurs d'ACE, les antagonistes des récepteurs d'angiotensine II, les bloquants des récepteurs bêta, les antagonistes de calcium, les inhibiteurs de PDE, les antagonistes des récepteurs minéralocorticoïdes, les diurétiques, l'aspirine, les suppléments de fer, les suppléments de vitamine B12 et d'acide folique, les statines, les dérivés de digitalis (digoxine), les agents chimiothérapeutiques contre les tumeurs et les antibiotiques.

17. Médicament selon la revendication 15 ou 16 pour le traitement et/ou la prophylaxie de maladies cardiovasculaires, de l'insuffisance cardiaque, de l'anémie, de l'insuffisance rénale chronique et de l'insuffisance rénale.
